# EUROPEAN PATENT SPECIFICATION

(11) **EP 3 156 402 B1**
(45) Date of publication and mention of the grant of the patent: **10.11.2021**
(21) Application number: 15807524.2
(22) Date of filing: 04.06.2015
(51) Int. Cl.: C07D 401/10, C09K 11/06, H01L 51/50

(54) **PYRIMIDINE DERIVATIVE AND ORGANIC ELECTROLUMINESCENT ELEMENT**
PYRIMIDINDERIVAT UND ORGANISCHES ELEKTROLUMINESZENTES ELEMENT
DÉRIVÉ DE PYRIMIDINE ET ÉLÉMENT ÉLECTROLUMINESCENT ORGANIQUE

(30) Priority: 11.06.2014 JP 2014120362
(43) Date of publication of application: 19.04.2017
(60) Divisional application: 20153606.7
(73) Proprietor: Hodogaya Chemical Co., Ltd., Chuo-ku Tokyo 104-0028 (JP)
(72) Inventor: HAYASHI, Shuichi, Tokyo 104-0028 (JP); KITAHARA, Hideyoshi, Tokyo 104-0028 (JP); KABASAWA, Naoaki, Tokyo 104-0028 (JP); CHOI, Sung Keum, Tokyo 104-0028 (JP); KIM, Si In, Tokyo 104-0028 (JP); SHIN, Yoo Na, Tokyo 104-0028 (JP); KIM, Ji Yung, Tokyo 104-0028 (JP)
(74) Representative: Müller-Boré & Partner Patentanwälte PartG mbB
(86) International application number: PCT/JP2015/066251
(87) International publication number: WO 2015/190400

(56) References cited:
- WO-A1-2011/021689
- WO-A1-2013/077352
- WO-A1-2013/077362
- WO-A1-2014/129201
- WO-A1-2016/175211
- WO-A2-2011/108902
- CN-A- 103 396 404
- DE-A1-102010 054 316
- JP-A- 2006 199 679
- JP-A- 2009 126 793
- JP-A- 2010 045 034
- JP-A- 2012 097 006
- JP-A- 2012 501 091
- JP-A- 2012 514 324
- JP-A- 2013 514 348
- JP-A- 2013 516 405
- JP-A- 2013 520 410
- JP-A- 2014 075 556
- KR-A- 20130 060 157
- KR-A- 20130 110 051
- KR-A- 20140 004 005
- KR-A- 20140 057 687
- LIU MING ET AL.: 'Hybrid Heterocycle-Containing Electron-Transport Materials Synthesized by Regioselective Suzuki Cross-Coupling Reactions for Highly Efficient Phosphorescent OLEDs with Unprecedented Low Operating Voltage' CHEMISTRY OF MATERIALS vol. 24, no. 20, 2012, pages 3817 - 3827, XP055242410
- SU SHI-JIAN ET AL.: 'Three-carbazole-armed host materials with various cores for RGB phosphorescent organic light-emitting diodes' JOURNAL OF MATERIALS CHEMISTRY vol. 22, no. 8, 2012, pages 3447 - 3456, XP055242421
- SASABE HISAHIRO ET AL.: '2-Phenylpyrimidine skeleton-based electron-transport materials for extremely efficient green organic light- emitting devices' CHEMICAL COMMUNICATIONS vol. 44, 2008, pages 5821 - 5823, XP055006715

## Description

This invention relates to a compound suitable for an organic electroluminescent device, and the device. More specifically, the invention relates to a pyrimidine derivative, and an organic electroluminescent device (hereinafter will be abbreviated as organic EL device) using the derivative.

An organic EL device is a self light-emitting device, and is thus brighter, better in visibility, and capable of clearer display, than a liquid crystal device. Hence, active researches have been conducted on organic EL devices.

In 1987, C.W. Tang et al. of Eastman Kodak developed a laminated structure device sharing various roles among different materials, thereby imparting practical applicability to organic EL devices using organic materials. They laminated a layer of a fluorescent body capable of transporting electrons, and a layer of an organic substance capable of transporting holes. Upon injecting the charges of electrons and holes into the layer of the fluorescent body to perform light emission, the device was capable of attaining a high luminance of 1,000 cd/m² or more at a voltage of 10V or less (see Patent Document 1 and Patent Document 2).

Many improvements have been made to put the organic EL devices to practical use. For example, high efficiency and durability are achieved by an electroluminescent device sharing the various roles among more types of materials, and having an anode, a hole injection layer, a hole transport layer, a luminous layer, an electron transport layer, an electron injection layer, and a cathode provided in sequence on a substrate.

For a further increase in the luminous efficiency, it has been attempted to utilize triplet excitons, and the utilization of phosphorescent luminous compounds has been considered. Furthermore, devices utilizing light emission by thermally activated delayed fluorescence (TADF) have been developed. An external quantum efficiency of 5.3% has been realized by an device using a thermally activated delayed fluorescence material.

The luminous layer can also be prepared by doping a charge transporting compound, generally called a host material, with a fluorescent compound, a phosphorescent luminous compound, or a material radiating delayed fluorescence. The selection of the organic material in the organic EL device greatly affects the characteristics of the device, such as efficiency and durability.

With the organic EL device, the charges injected from both electrodes recombine in the luminous layer to obtain light emission, and how efficiently the charges of the holes and the electrons are passed on to the luminous layer is of importance. Electron injection properties are enhanced, and electron mobility is increased to increase the probability of holes and electrons recombining. Moreover, excitons generated within the luminous layer are confined. By so doing, a high luminous efficiency can be obtained. Thus, the role of the electron transport material is so important that there has been a desire for an electron transport material having high electron injection properties, allowing marked electron mobility, possessing high hole blocking properties, and having high durability to holes.

From the viewpoint of device lifetime, heat resistance and amorphousness of the material are also important. A material with low heat resistance is thermally decomposed even at a low temperature by heat produced during device driving, and the material deteriorates. In a material with low amorphousness, crystallization of a thin film occurs even in a short time, and the device deteriorates. Thus, high resistance to heat and good amorphousness are required of the material to be used.

A representative luminescent material, tris(8-hydroxyquinoline)aluminum (hereinafter will be abbreviated as Alq), is generally used as an electron transport material as well. However, its hole blocking performance is insufficient.

Among measures to prevent some holes from passing through the luminous layer and increase the probability of charge recombination in the luminous layer is the insertion of a hole blocking layer. As hole blocking materials, proposals have been made, for example, for triazole derivatives (see Patent Document 3), bathocuproine (hereinafter abbreviated as BCP), and an aluminum-mixed ligand complex [aluminum (III) bis(2-methyl-8-quinolinato)-4-phenylphenolate (hereinafter abbreviated as BAlq)].

As an electron transport material with excellent hole blocking properties, a proposal has been made for 3-(4-biphenylyl)-4-phenyl-5-(4-t-butylphenyl)-1,2,4-triazol e (hereinafter abbreviated as TAZ) (see Patent Document 4).

TAZ has a great work function of 6.6 eV and possesses high hole blocking capability. Thus, TAZ is laminated on the cathode side of a fluorescent luminous layer or a phosphorescent luminous layer prepared by vacuum deposition, coating or the like, and is used as a hole blocking layer having electron transporting properties. TAZ thus contributes to increasing the efficiency of an organic EL device.

TAZ, however, has low properties of transporting electrons. It has been necessary, therefore, to combine TAZ with an electron transport material having higher electron transporting properties in preparing an organic EL device.

BCP also has a great work function of 6.7 eV and possesses high hole blocking capability. However, its glass transition point (Tg) is as low as 83°C, and thus its stability when as a thin film is poor.

That is, the materials cited above are all insufficient in device lifetime, or insufficient in the function of blocking holes. In order to improve the device characteristics of an organic EL device, therefore, there has been a desire for an organic compound excellent in electron injection/transport performance and hole blocking capability and having a long device lifetime.

Patent Document 1: JP-A-Hei 8-48656
Patent Document 2: Japanese Patent No. 3194657
Patent Document 3: Japanese Patent No. 2734341
Patent Document 4: WO2003/060956
Patent Document 5: WO2014/009310

It is an object of the present invention to provide an organic compound, which is excellent in electron injection/transport performance, has hole blocking capability, and excels in characteristics, as a material for a high efficiency, high durability organic EL device.

It is another object of the present invention to provide an organic EL device having high efficiency, high durability, and long lifetime with the use of this compound.

To attain the above objects, the present inventors noted that the nitrogen atom of a pyrimidine ring having electron affinity had the ability to be coordinated to a metal, and also led to excellent heat resistance. Based on these facts, they designed and chemically synthesized a compound having a pyrimidine ring structure. Using this compound, moreover, they experimentally produced various organic EL devices, and extensively evaluated the characteristics of the devices. As a result, they have accomplished the present invention.

There is generally described herein a pyrimidine derivative represented by the following general formula (1) 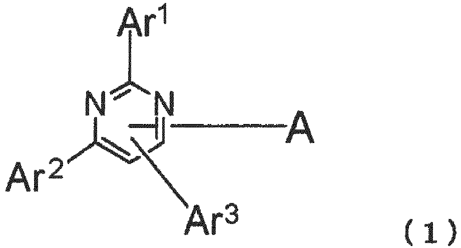 wherein,
Ar¹ represents an aromatic hydrocarbon group, a condensed polycyclic aromatic group, or an aromatic heterocyclic group,
Ar² and Ar³ may be the same or different, and each represent a hydrogen atom, an aromatic hydrocarbon group, a condensed polycyclic aromatic group, or an aromatic heterocyclic group,
Ar² and Ar³ are not simultaneously hydrogen atoms, and
A represents a monovalent group represented by the following structural formula (2), 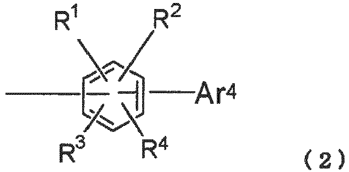 wherein,
   Ar⁴ represents an aromatic heterocyclic group,
   R¹ to R⁴ may be the same or different, and each represent a hydrogen atom, a deuterium atom, a fluorine atom, a chlorine atom, a cyano group, a trifluoromethyl group, an alkyl group having 1 to 6 carbon atoms, an aromatic hydrocarbon group, a condensed polycyclic aromatic group, or an aromatic heterocyclic group, and
   R¹ to R⁴ and Ar⁴ may be bonded to each other via a single bond, a methylene group, an oxygen atom, or a sulfur atom to form a ring.

1) According to the present invention, there is provided
   a pyrimidine derivative represented by the following general formula (1-1): 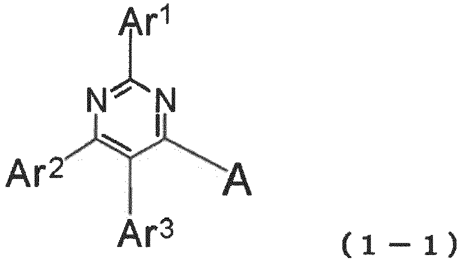 wherein,
   Ar¹ is a phenyl group having, as a substituent, an unsubstituted condensed polycyclic aromatic group selected from the group consisting of a naphthyl group, a phenanthrenyl group, a pyrenyl group, a fluoranthenyl group, a triphenylenyl group, and a spirobifluorenyl group,
   Ar² is a phenyl group having, as a substituent, an unsubstituted condensed polycyclic aromatic group selected from the group consisting of a naphthyl group, a phenanthrenyl group, a pyrenyl group, a fluoranthenyl group, a triphenylenyl group, and a spirobifluorenyl group,
   Ar³ is a hydrogen atom, and
   A is a monovalent group represented by the following structural formula (2-2): 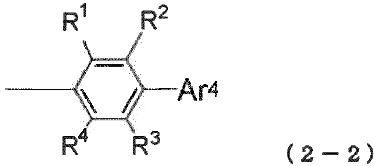 wherein,
      Ar⁴ is an unsubstituted pyridyl group, and
      R¹ to R⁴ are hydrogen atoms or deuterium atoms.

According to the present invention, moreover, there is provided an organic EL device comprising a pair of electrodes and at least one organic layer sandwiched therebetween, wherein the pyrimidine derivative is used as a constituent material for the at least one organic layer.

In the organic EL device of the present invention, it is preferred that the organic layer for which the pyrimidine derivative is used be an electron transport layer, a hole blocking layer, a luminous layer, or an electron injection layer.

The pyrimidine derivative of the present invention is a novel compound, and has the following properties:
(1) Electron injection characteristics are satisfactory.
(2) Electron mobility is high.
(3) Hole blocking capability is excellent.
(4) Thin film state is stable.
(5) Heat resistance is excellent.

Moreover, the organic EL device of the present invention has the following properties:
(6) Luminous efficiency is high.
(7) Light emission starting voltage is low.
(8) Practical driving voltage is low.
(9) Lifetime is long.

The pyrimidine derivative of the present invention has high electron injection and moving rates. With an organic EL device having an electron injection layer and/or an electron transport layer prepared using the pyrimidine derivative of the present invention, therefore, the efficiency of electron transport from the electron transport layer to the luminous layer is raised to increase the luminous efficiency. Also, the driving voltage is lowered to enhance the durability of the resulting organic EL device.

The pyrimidine derivative of the present invention has excellent ability to block holes, is excellent in electron transporting properties, and is stable in a thin film state. Thus, an organic EL device having a hole blocking layer prepared using the pyrimidine derivative of the present invention has a high luminous efficiency, is lowered in driving voltage, and is improved in current resistance, so that the maximum light emitting brightness of the organic EL device is increased.

The pyrimidine derivative of the present invention has excellent electron transporting properties, and has a wide bandgap. Therefore, the pyrimidine derivative of the present invention is used as a host material to carry a fluorescence emitting substance, a phosphorescence emitting substance or a delayed fluorescence emitting substance, called a dopant, thereon so as to form a luminous layer. This makes it possible to realize an organic EL device that drives on lowered voltage and features an improved luminous efficiency.

As described above, the pyrimidine derivative of the present invention is useful as a constituent material for an electron injection layer, an electron transport layer, a hole blocking layer, or a luminous layer of an organic EL device. With the organic EL device of the present invention, excitons generated within the luminous layer can be confined, and the probability of recombination of holes and electrons can be further increased to obtain a high luminous efficiency. In addition, the driving voltage is so low that high durability can be achieved.

[Fig. 1] is a ¹H-NMR chart diagram of the compound of Example 1 (Compound 74) (Reference Example).
[Fig. 2] is a ¹H-NMR chart diagram of the compound of Example 2 (Compound 84) (Reference Example).
[Fig. 3] is a ¹H-NMR chart diagram of the compound of Example 3 (Compound 89) (Reference Example).
[Fig. 4] is a ¹H-NMR chart diagram of the compound of Example 4 (Compound 130).
[Fig. 5] is a ¹H-NMR chart diagram of the compound of Example 5 (Compound 131) .
[Fig. 6] is a ¹H-NMR chart diagram of the compound of Example 6 (Compound 92).
[Fig. 7] is a ¹H-NMR chart diagram of the compound of Example 7 (Compound 136).
[Fig. 8] is a ¹H-NMR chart diagram of the compound of Example 8 (Compound 125).
[Fig. 9] is a ¹H-NMR chart diagram of the compound of Example 9 (Compound 138).
[Fig. 10] is a ¹H-NMR chart diagram of the compound of Example 10 (Compound 78).
[Fig. 11] is a ¹H-NMR chart diagram of the compound of Example 11 (Compound 76).
[Fig. 12] is a ¹H-NMR chart diagram of the compound of Example 12 (Compound 126) (Reference Example).
[Fig. 13] is a ¹H-NMR chart diagram of the compound of Example 13 (Compound 124) (Reference Example).
[Fig. 14] is a ¹H-NMR chart diagram of the compound of Example 14 (Compound 123) (Reference Example).
[Fig. 15] is a ¹H-NMR chart diagram of the compound of Example 15 (Compound 146) (Reference Example).
[Fig. 16] is a ¹H-NMR chart diagram of the compound of Example 16 (Compound 98).
[Fig. 17] is a ¹H-NMR chart diagram of the compound of Example 17 (Compound 153).
[Fig. 18] is a ¹H-NMR chart diagram of the compound of Example 18 (Compound 155).
[Fig. 19] is a ¹H-NMR chart diagram of the compound of Example 19 (Compound 82).
[Fig. 20] is a ¹H-NMR chart diagram of the compound of Example 20 (Compound 182) (Reference Example).
[Fig. 21] is a ¹H-NMR chart diagram of the compound of Example 21 (Compound 227) (Reference Example).
[Fig. 22] is a ¹H-NMR chart diagram of the compound of Example 22 (Compound 234) (Reference Example).
[Fig. 23] is a ¹H-NMR chart diagram of the compound of Example 23 (Compound 235) (Reference Example).
[Fig. 24] is a view showing the EL device configuration of Organic EL Device Examples 1 to 20 (Examples 1 to 3, 12 to 15 and 20 to 23 are Reference Examples) and Organic EL Device Comparative Example 1.

The pyrimidine derivative of the present invention, concretely, has the structure of the following general formula (1-1) :

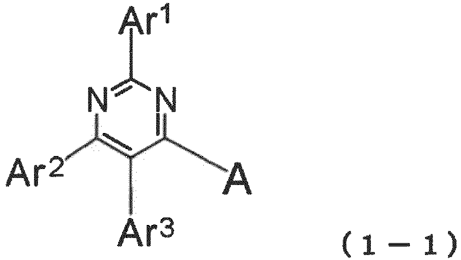

In the above general formula (1-1),
Ar¹ is a phenyl group having, as a substituent, an unsubstituted condensed polycyclic aromatic group selected from the group consisting of a naphthyl group, a phenanthrenyl group, a pyrenyl group, a fluoranthenyl group, a triphenylenyl group, and a spirobifluorenyl group,
Ar² is a phenyl group having, as a substituent, an unsubstituted condensed polycyclic aromatic group selected from the group consisting of a naphthyl group, a phenanthrenyl group, a pyrenyl group, a fluoranthenyl group, a triphenylenyl group, and a spirobifluorenyl group,
Ar³ is a hydrogen atom, and
A is a monovalent group represented by the following structural formula (2-2): 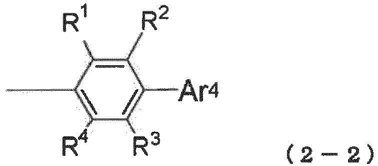 wherein,
   Ar⁴ is an unsubstituted pyridyl group, and
   R¹ to R⁴ are hydrogen atoms or deuterium atoms.

<Ar¹ to Ar³>

The aromatic hydrocarbon group, represented by Ar¹ and Ar², is a phenyl group.

The aromatic hydrocarbon group, represented by Ar¹ and Ar², has a substituent. The substituent is an unsubstituted condensed polycyclic aromatic group selected from the group, consisting of a naphthyl group, a phenanthrenyl group, a pyrenyl group, a fluoranthenyl group, a triphenylenyl group, and a spirobifluorenyl group.

The substituent and Ar¹ or Ar², to which the substituent is bound, may be bonded to each other via an oxygen atom or a sulfur atom to form a ring, but may be present independently of each other without forming a ring.

<A> A represents a monovalent group represented by the following structural formula (2-2): 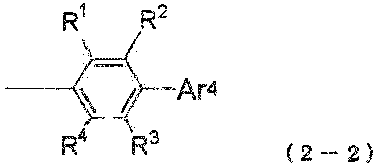 wherein,
   Ar⁴ is an unsubstituted pyridyl group, and
   R¹ to R⁴ are hydrogen atoms or deuterium atoms.

### (Ar⁴)

The aromatic heterocyclic group, represented by Ar⁴, is an unsubstituted pyridyl group.

### (R¹ to R⁴)

R¹ to R⁴ are hydrogen atoms or deuterium atoms.

### <Preferred embodiments>

Ar¹ and Ar² may be the same, but from the viewpoint of thin film stability, are preferably different. If Ar¹ and Ar² represent the same group, Ar¹ and Ar² may have different substituents, or may have the same substituent at different substitution positions.

Ar² and Ar³ may generally be the same group, but increased symmetry of the entire molecule results in a higher possibility for crystallization. From the viewpoint of stability in a thin film state, therefore, Ar² and Ar³ are different groups according to the present invention.

### (A)

According to a general embodiment not within the scope of the present invention, the group A is represented by the structural formula (2-1) below, in which group Ar⁴ is bound, at the meta-position in the benzene ring, to the pyrimidine ring represented by the general formula (1).

According to the present invention, the group A is represented by the structural formula (2-2) below, from the viewpoint of synthesis. That is, the group Ar⁴ is bound, at the para-position in the benzene ring, to the pyrimidine ring represented by the general formula (1).

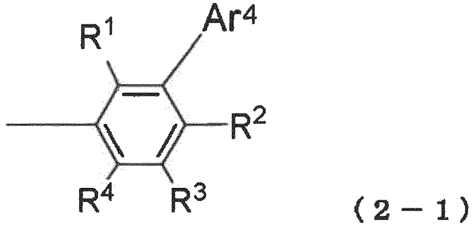

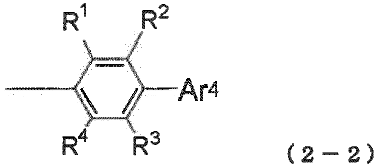

Ar⁴ is an unsubstituted pyridyl group.

As the pyridyl group, a pyridin-3-yl group is preferred.

R¹ to R⁴ are a hydrogen atom or a deuterium atom.

### <Manufacturing method>

The pyrimidine derivative of the present invention can be produced, for example, by the following method: A Suzuki coupling reaction between 2,4,6-trichloropyrimidine and an arylboronic acid or an arylboronic ester having a group corresponding to the 4-position is performed. Then, phenylboronic acid or phenylboronic ester having a corresponding heteroaryl group as a substituent is added, and a Suzuki coupling reaction is carried out. By performing such Suzuki coupling reactions in two stages, the corresponding group is introduced into the 4-position of the pyrimidine ring, and the phenyl group having the corresponding heteroaryl group as a substituent is introduced into the 6-position. Furthermore, a Suzuki coupling reaction is performed using an arylboronic acid or an arylboronic ester having a group corresponding to the 2-position, thereby introducing the corresponding group into the 2-position of the pyrimidine ring. In this manner, the pyrimidine derivative of the present invention can be produced.

In the above-described manufacturing method, the sequence of introduction of the respective groups into the 2-position, 4-position and 6-position of the pyrimidine ring may be changed as appropriate.

In the manufacturing method, moreover, a pyrimidine having a halogen atom such as a chloro group substituted at a different position, for example, 2,4,5-trichloropyrimidine, may be used instead of 2,4,6-trichloropyrimidine. In this case, a pyrimidine derivative different in the position of substitution can be produced.

The purification of the resulting compound can be performed, for example, by purification using a column chromatograph, adsorption purification using silica gel, activated carbon, activated clay or the like, recrystallization or crystallization using a solvent, sublimation purification and the like. Identification of the compound can be performed by NMR analysis. As physical property values, a work function and a glass transition point (Tg) can be measured.

The work function serves as an index to hole blocking properties. The work function can be measured by preparing a 100 nm thin film on an ITO substrate and using an ionization potential measuring device (PYS-202, produced by Sumitomo Heavy Industries, Ltd.) on the sample.

The glass transition point (Tg) serves as an index to stability in a thin film state. The glass transition point (Tg) can be measured, for example, with a high sensitivity differential scanning calorimeter (DSC3100S, produced by Bruker AXS K.K.) using a powder.

Of the pyrimidine derivatives of the present invention, concrete examples of the preferred compounds will be shown below as compounds 76, 78 to 80, 82, 83, 92, 93, 95, 97 to 99, 125, 127, 128, 130 to 133, 135, 136, 138 to 14o, 143, 147, 150, 152 to 156, 158, 159, 161, 164, 172, 173, 176, 229, 230 and 233, but the present invention is in no way limited to these compounds. Compounds 1 to 75, 77, 81, 84 to 91, 94, 96, 100 to 124, 126, 129, 134, 137, 141, 142, 144 to 146, 148, 149, 151, 157, 160, 162, 163, 165 to 171, 174, 175, 177 to 228, 231, 232 and 234 to 264 are reference compounds not within the scope of the present invention.

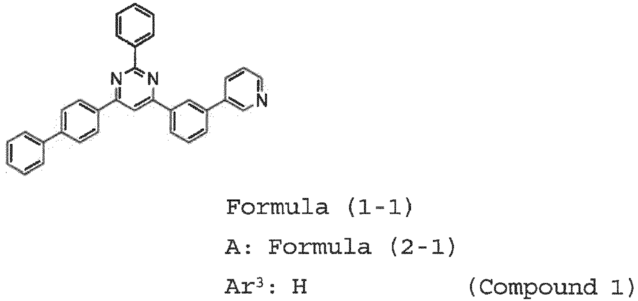

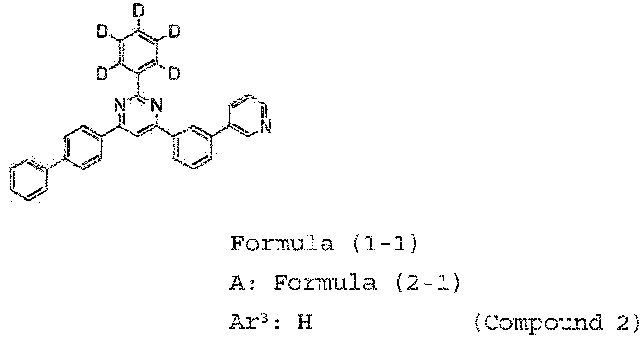

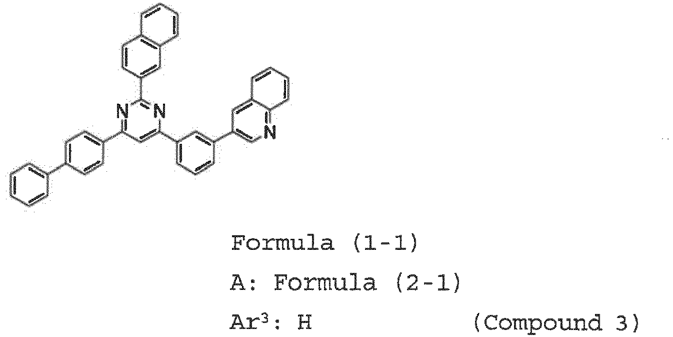

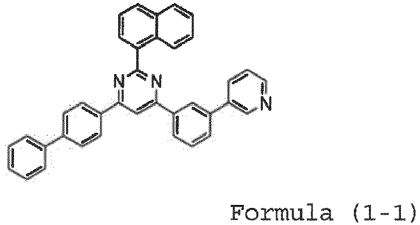

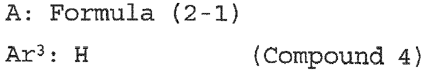

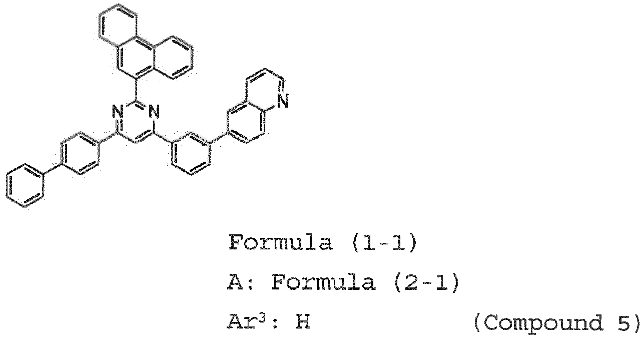

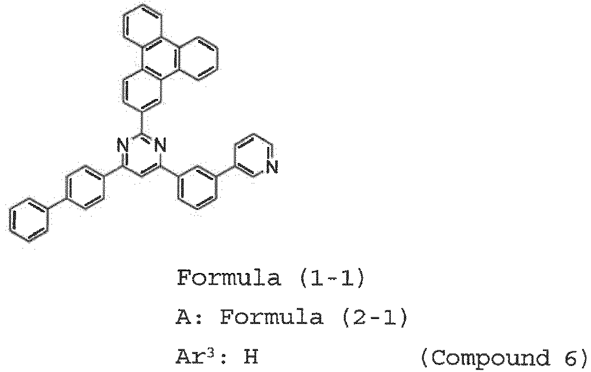

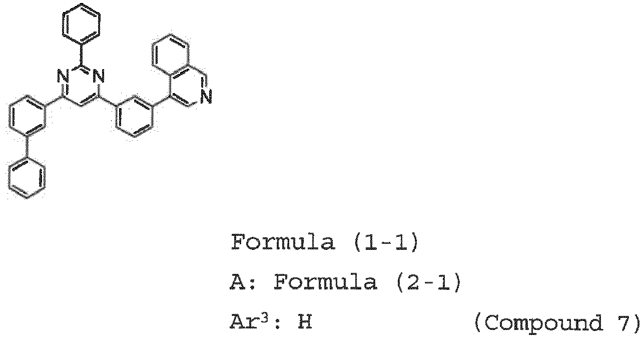

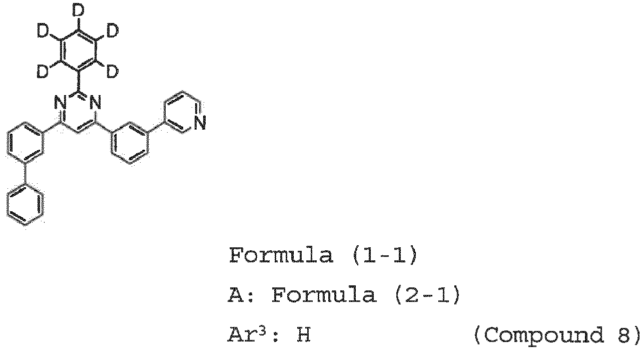

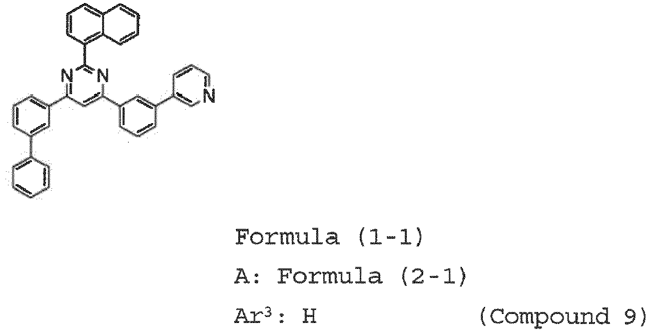

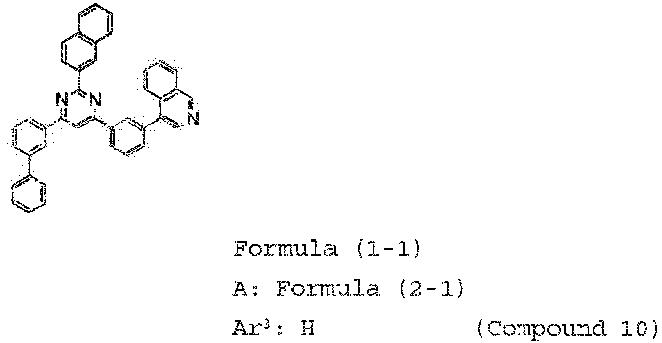

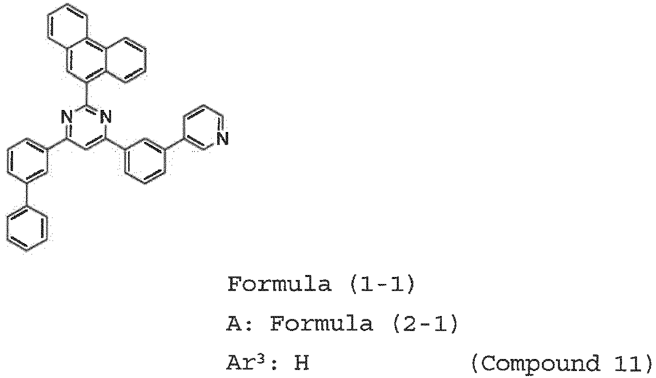

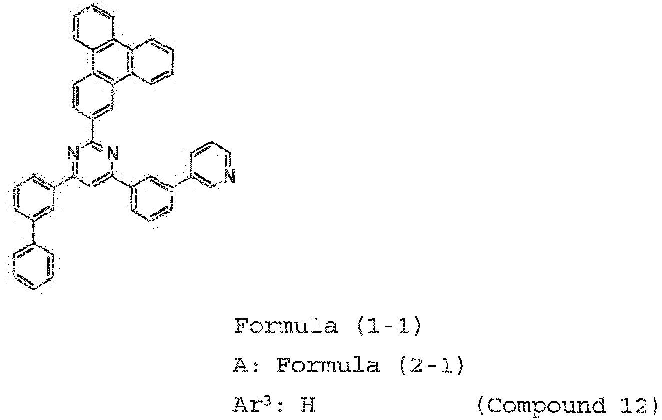

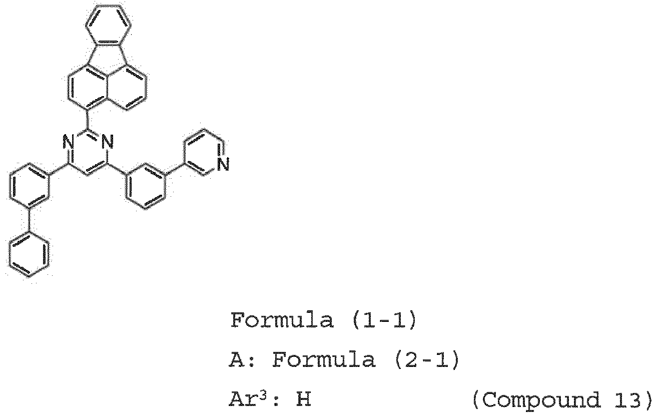

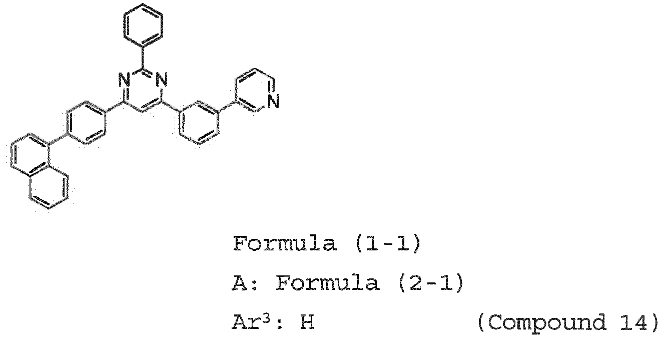

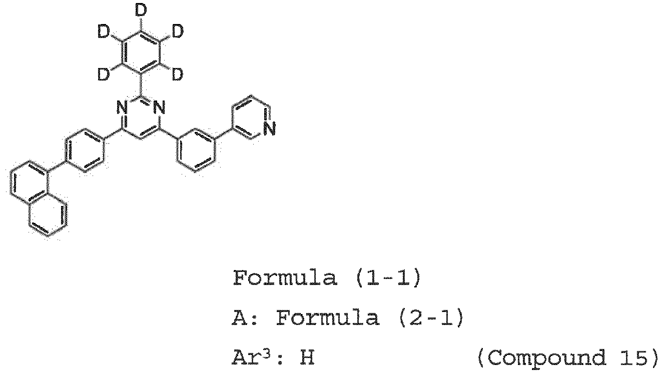

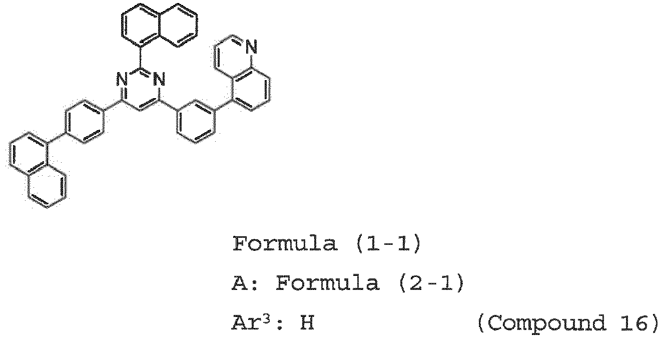

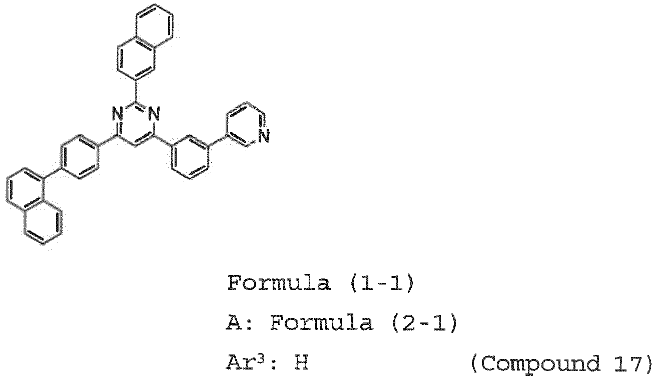

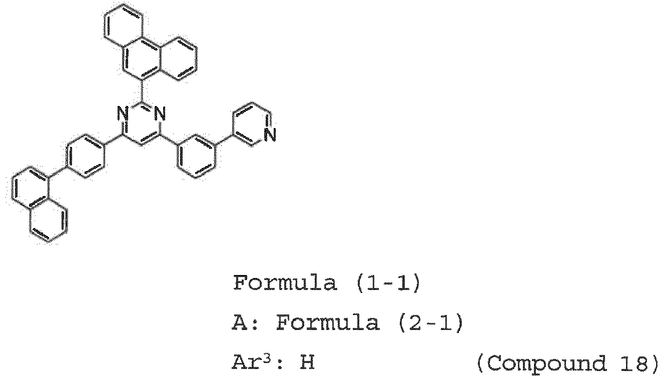

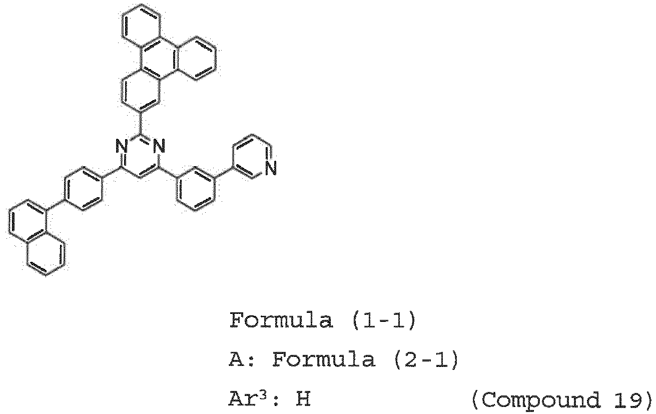

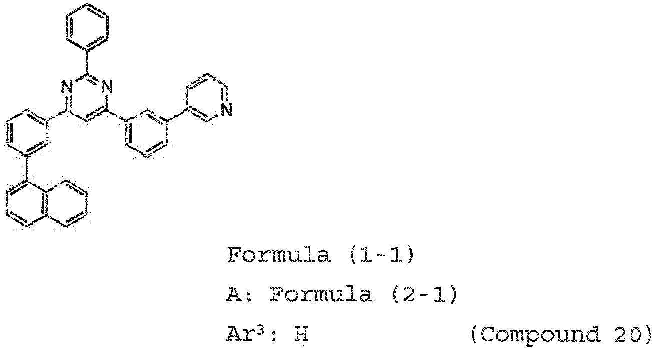

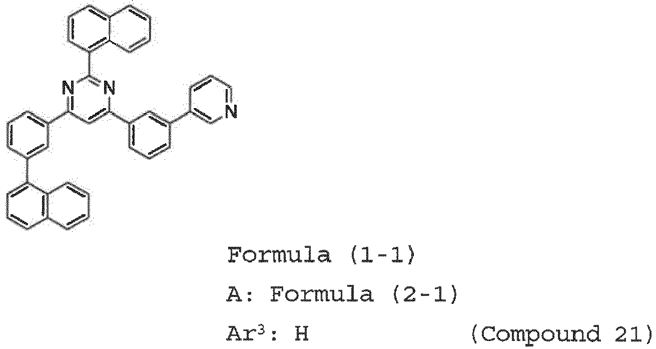

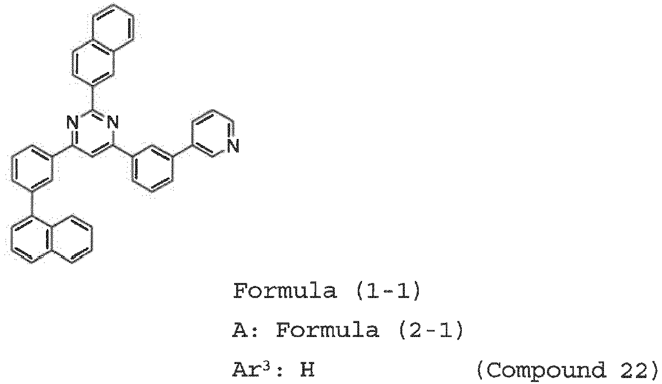

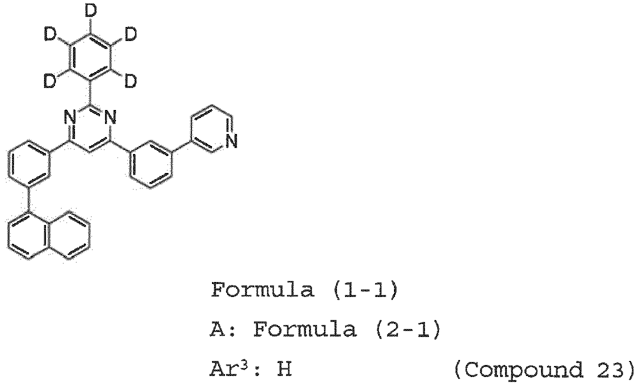

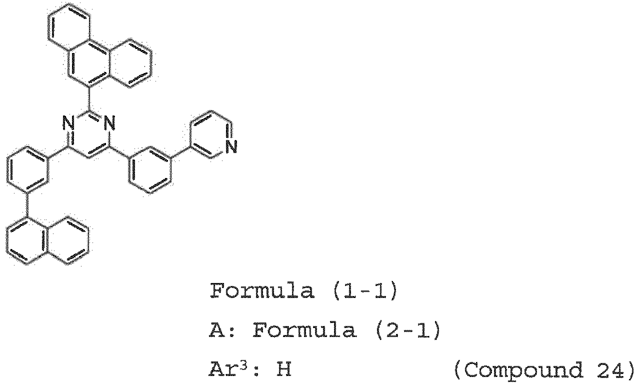

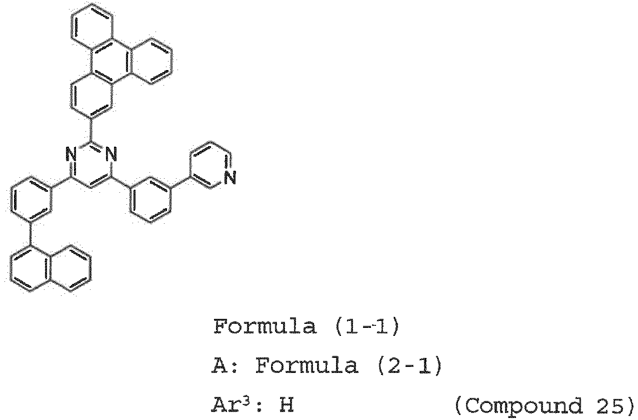

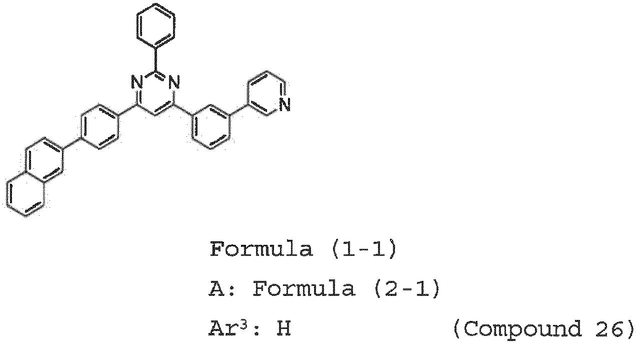

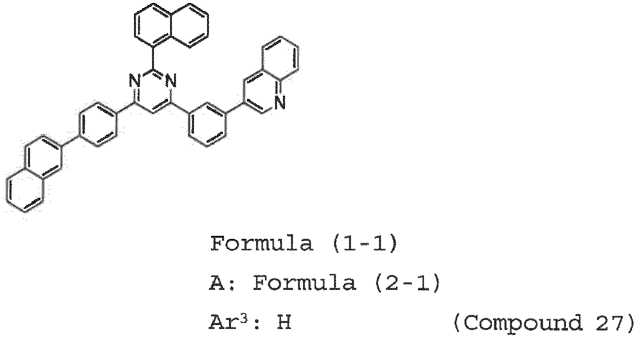

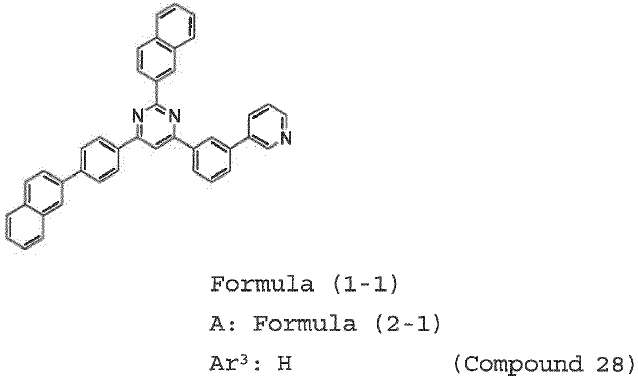

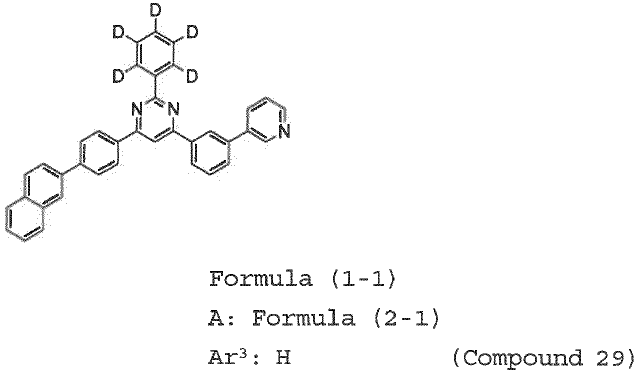

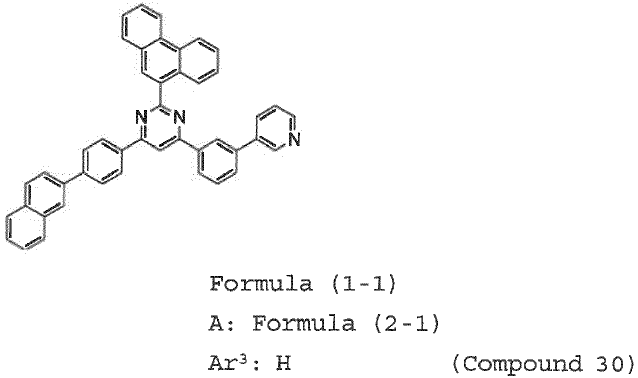

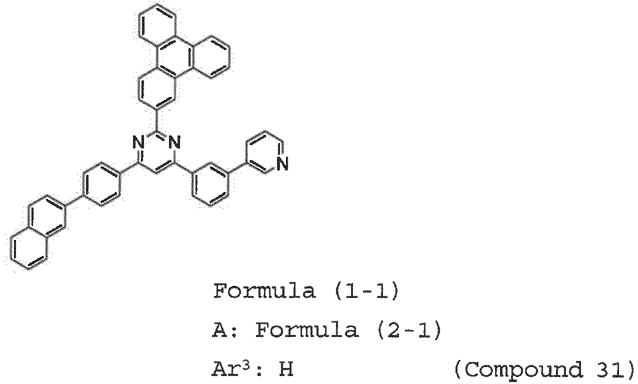

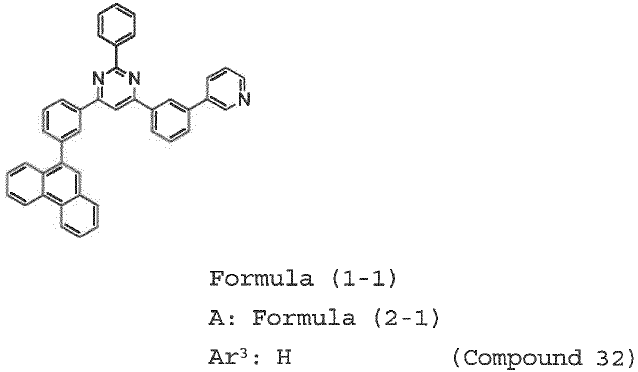

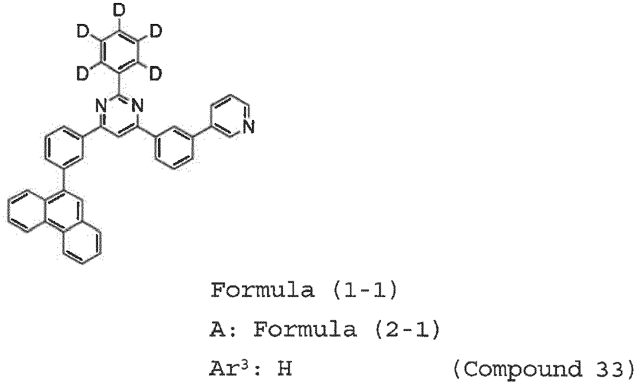

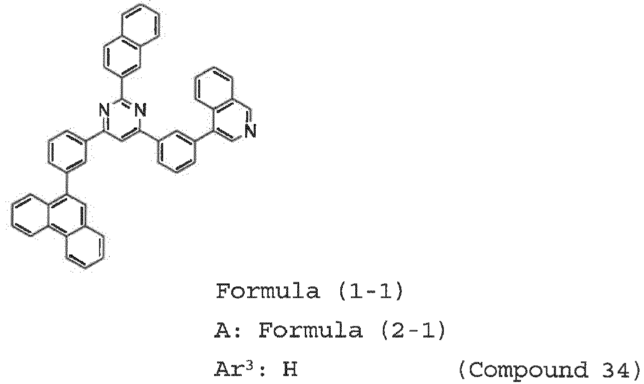

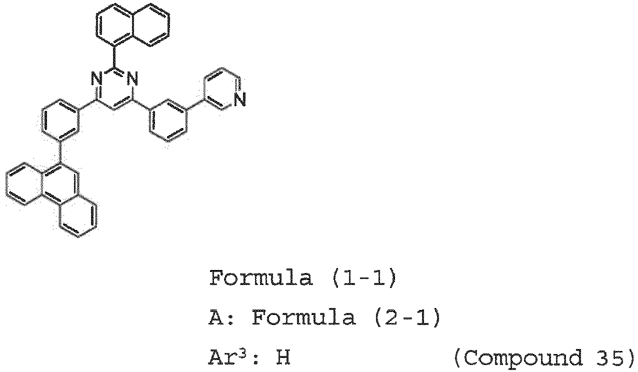

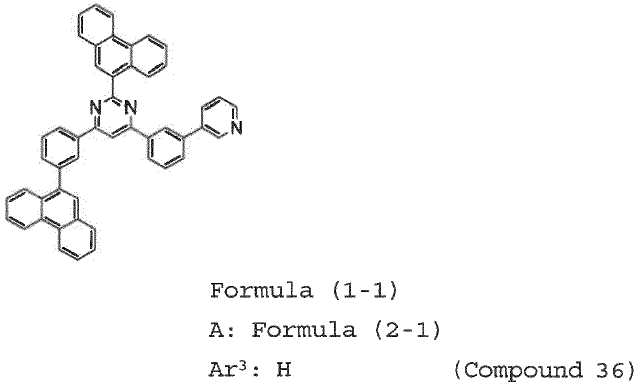

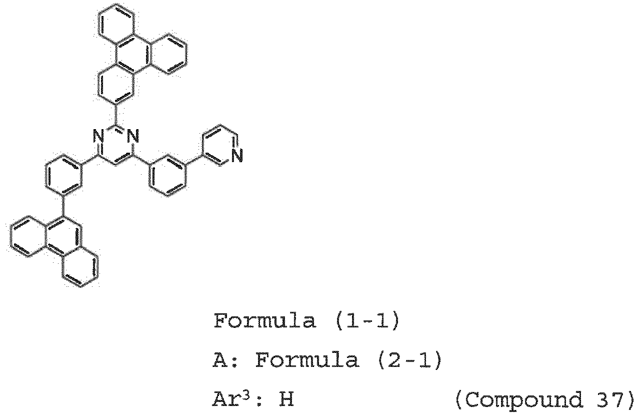

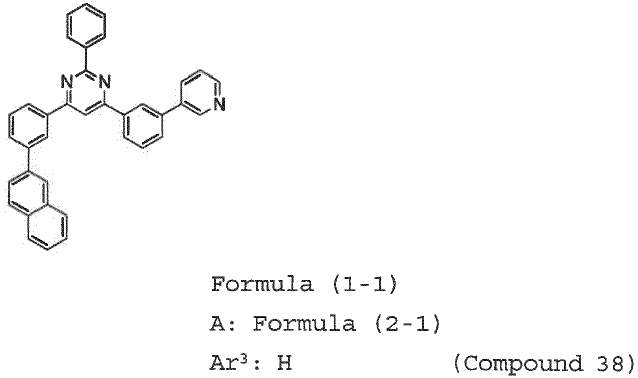

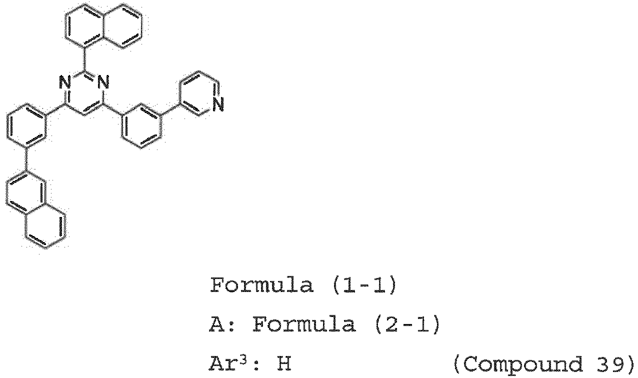

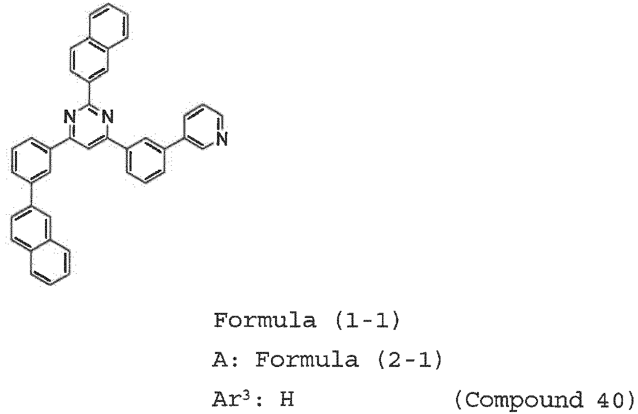

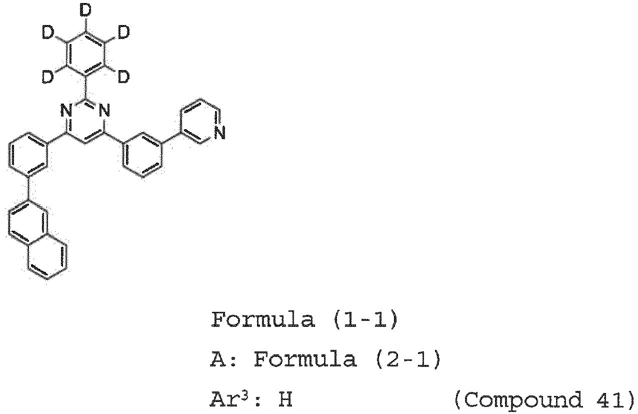

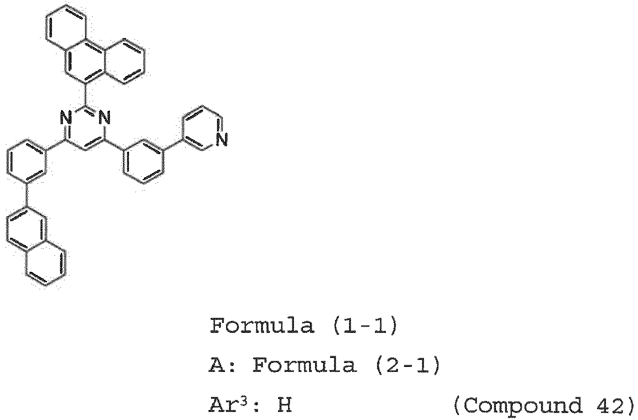

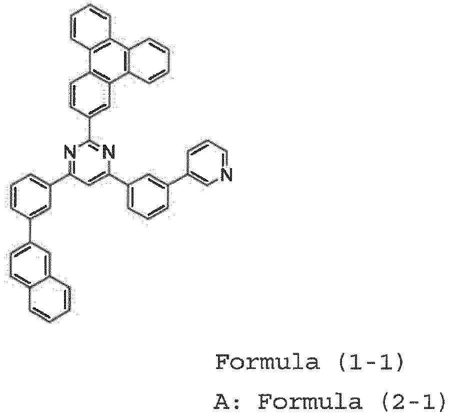

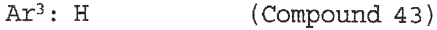

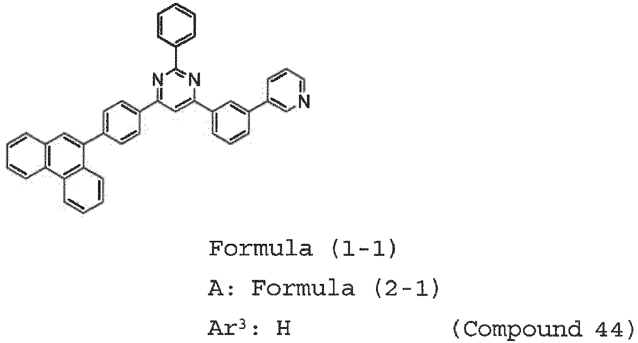

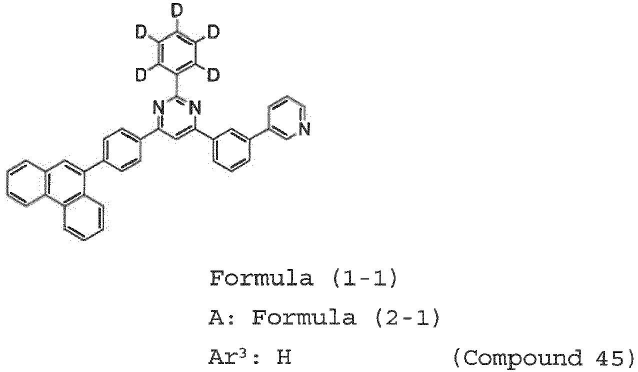

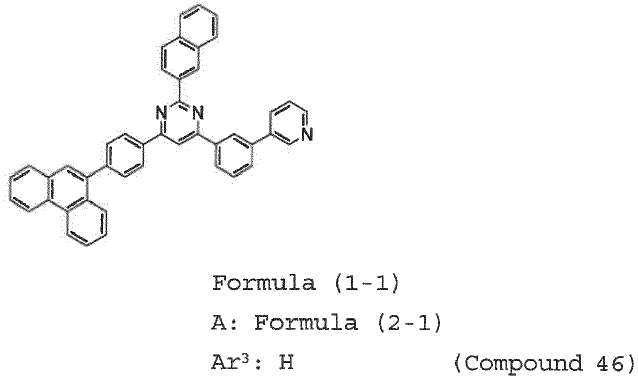

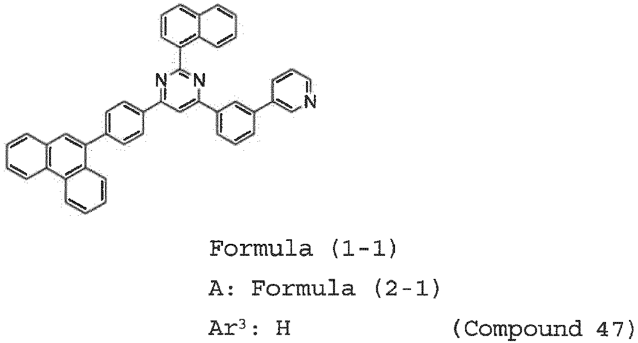

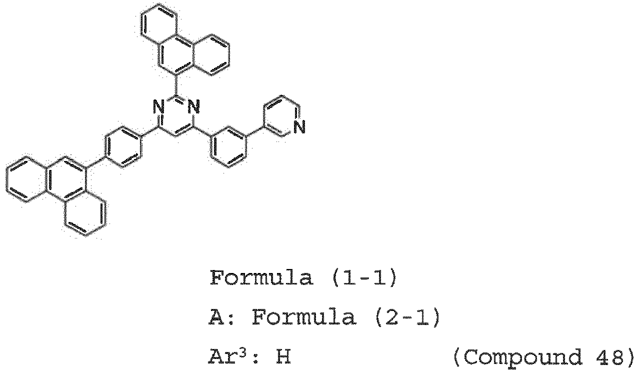

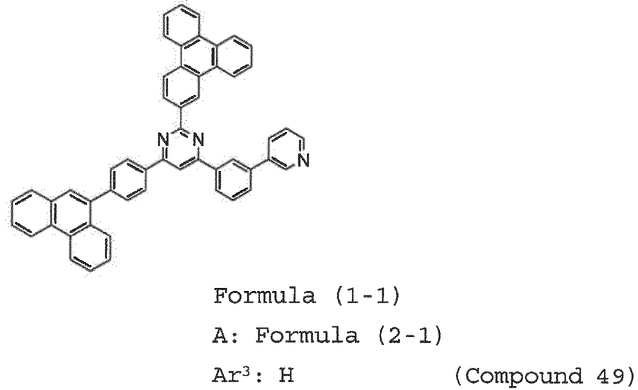

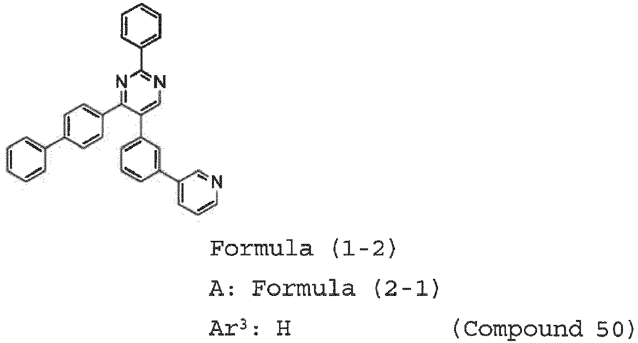

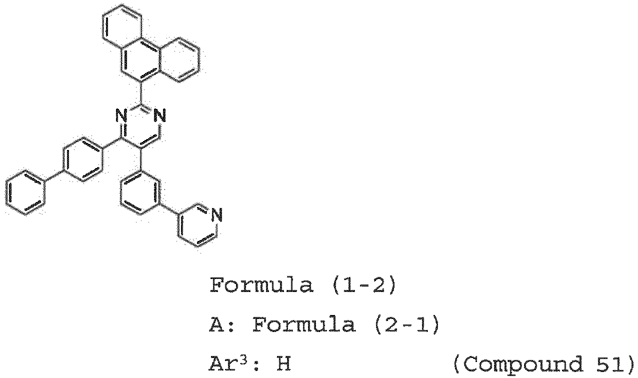

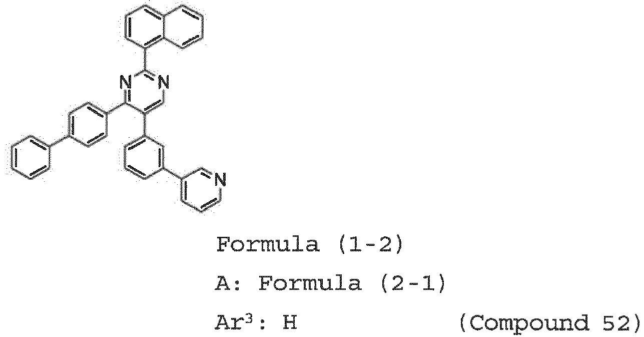

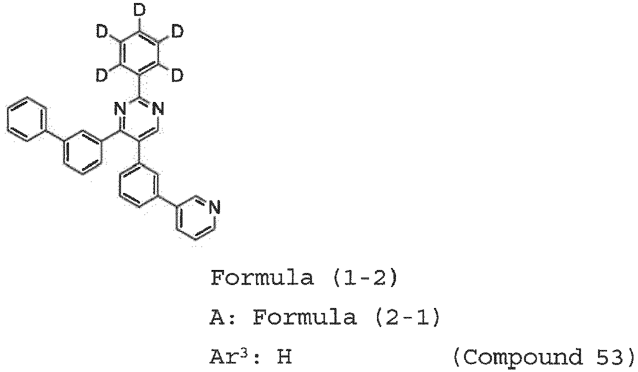

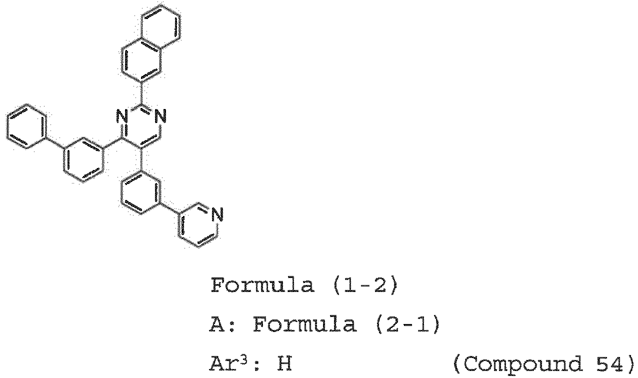

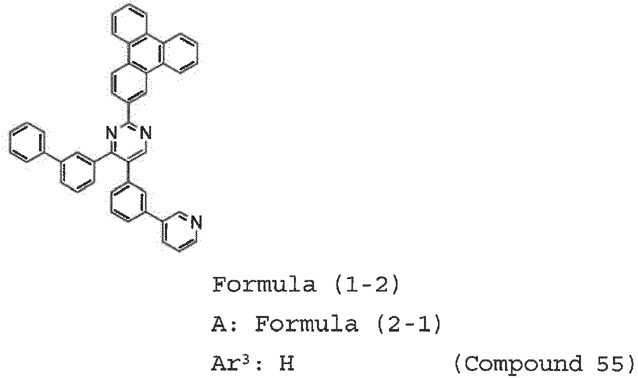

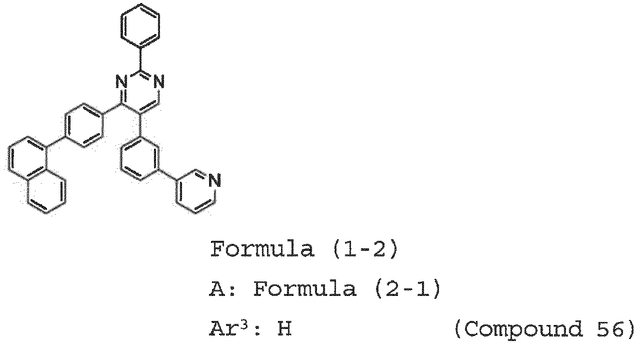

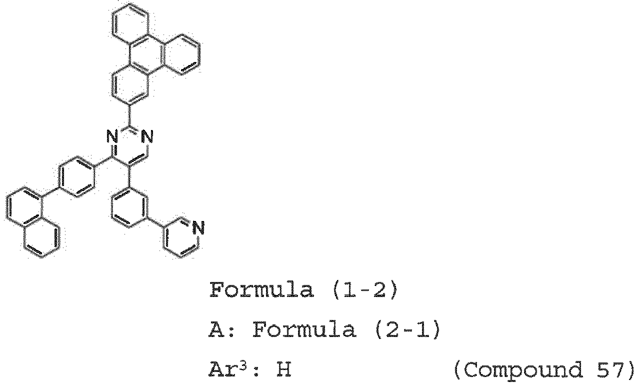

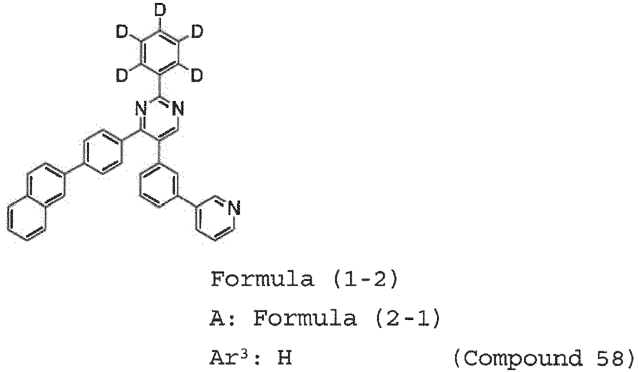

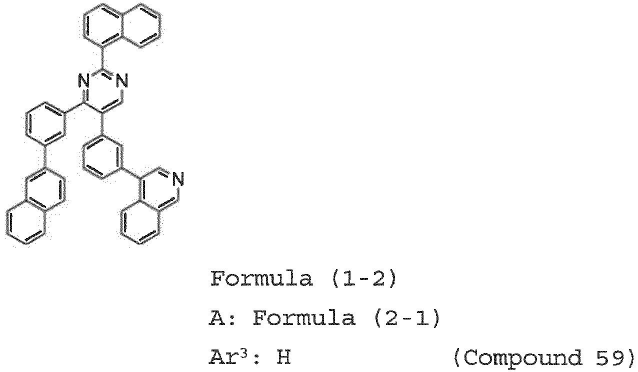

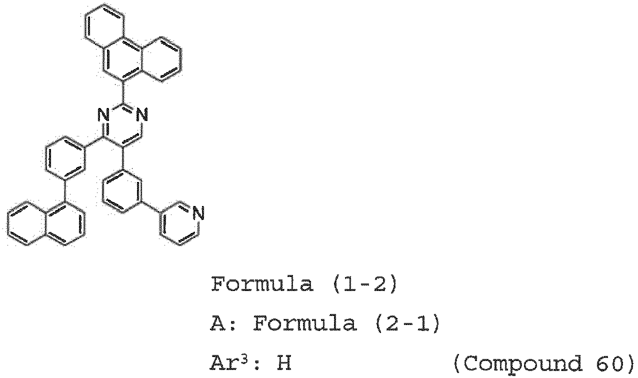

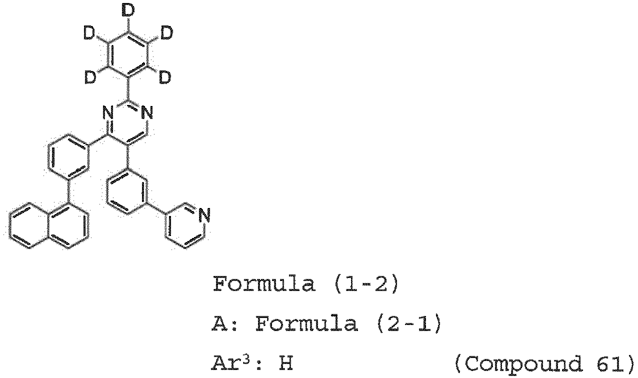

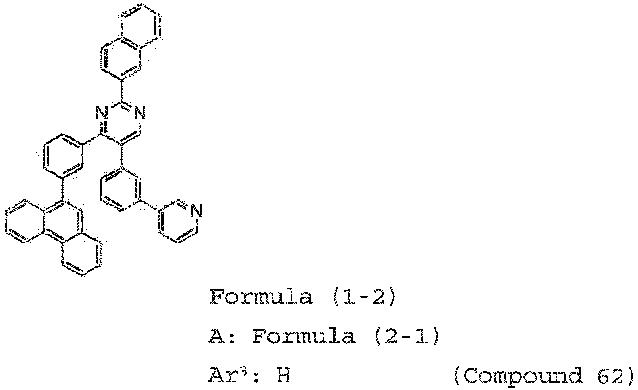

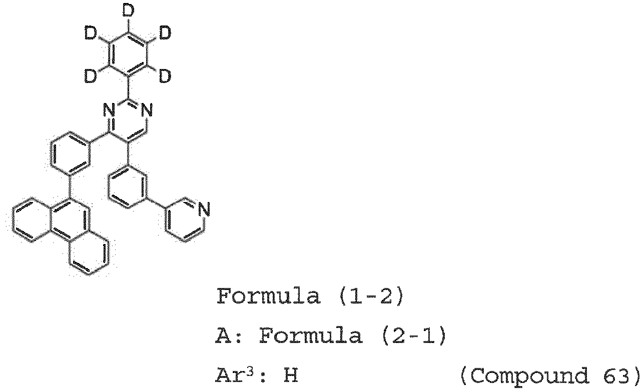

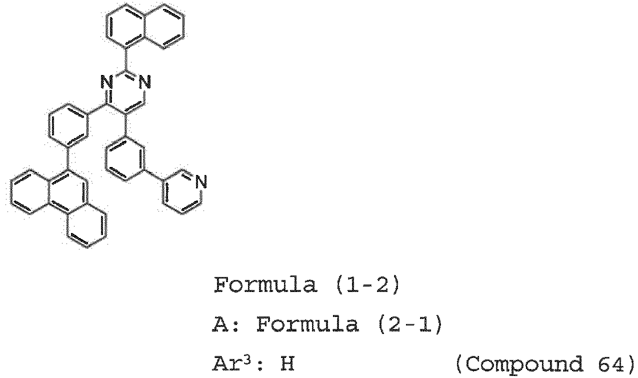

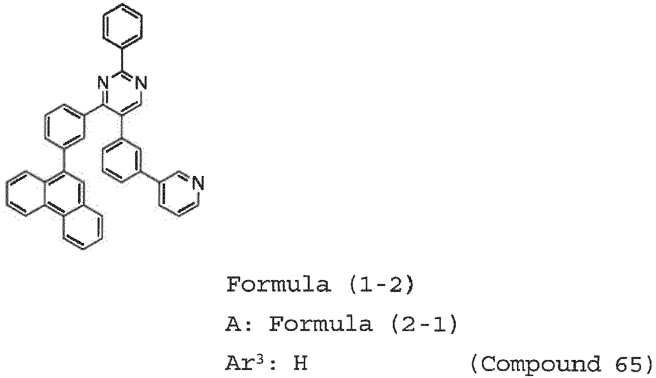

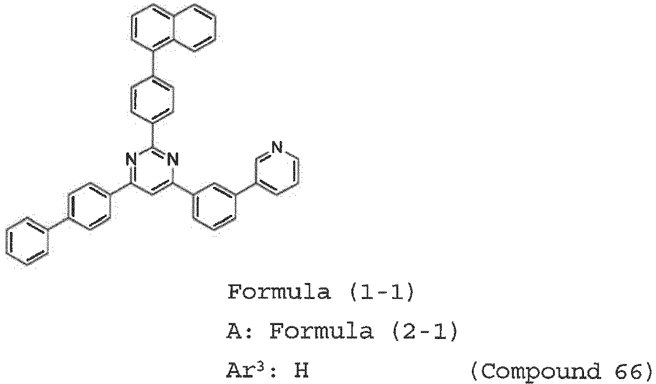

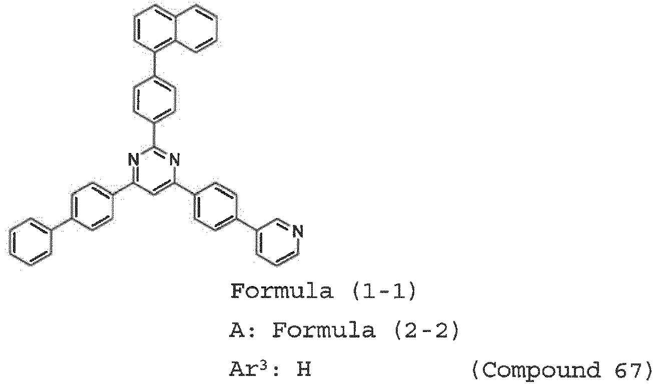

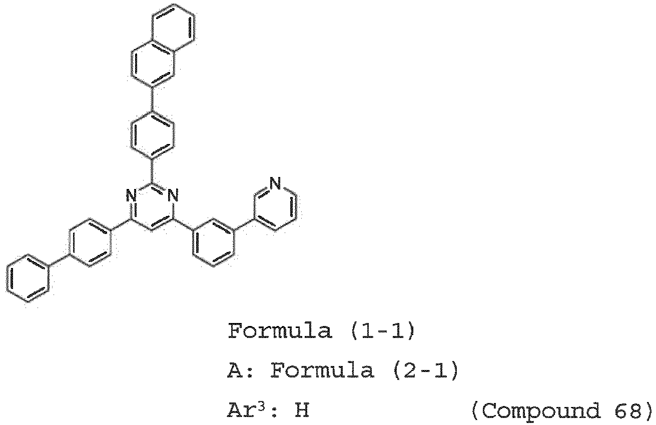

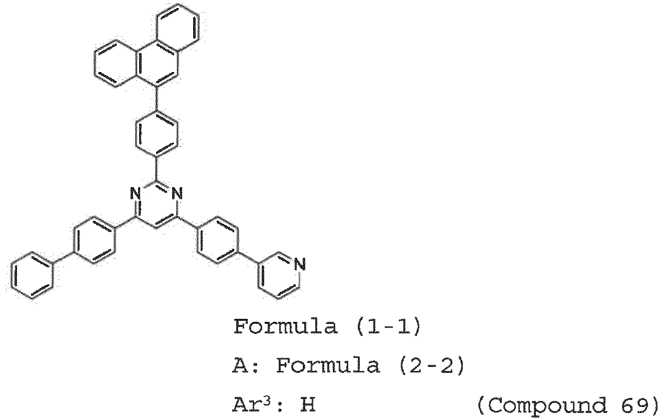

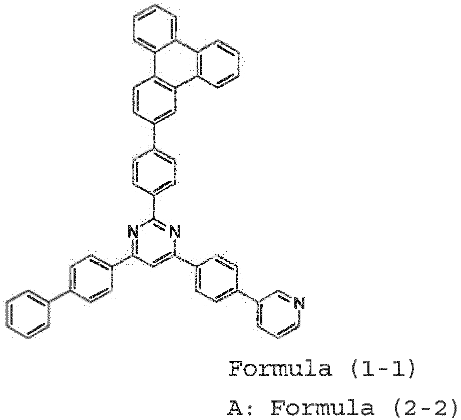

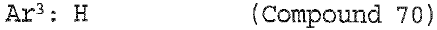

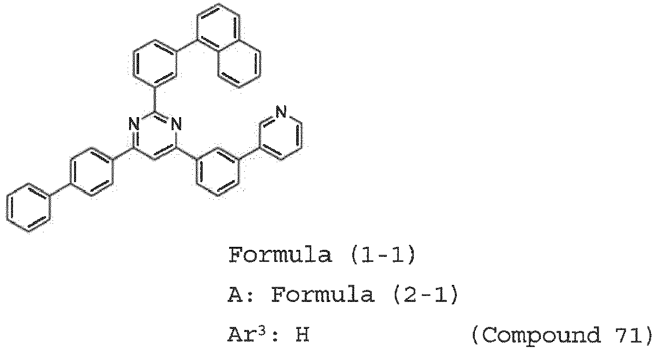

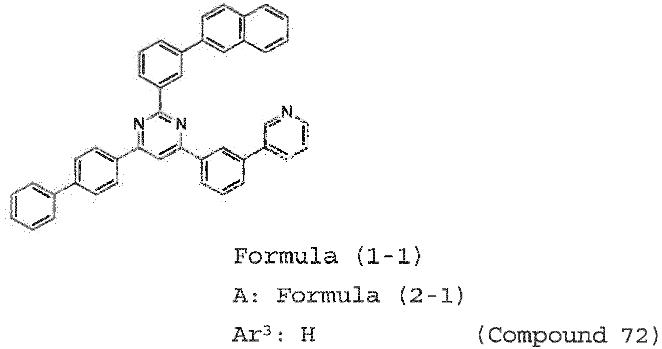

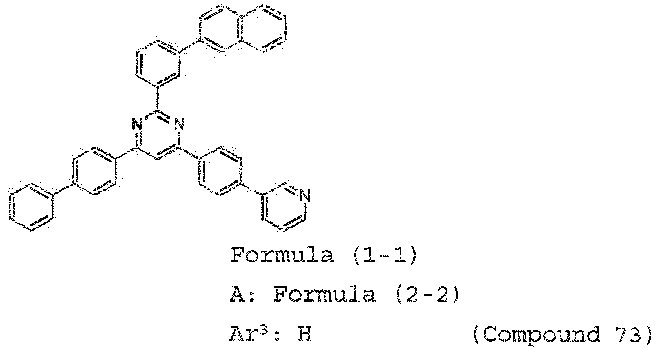

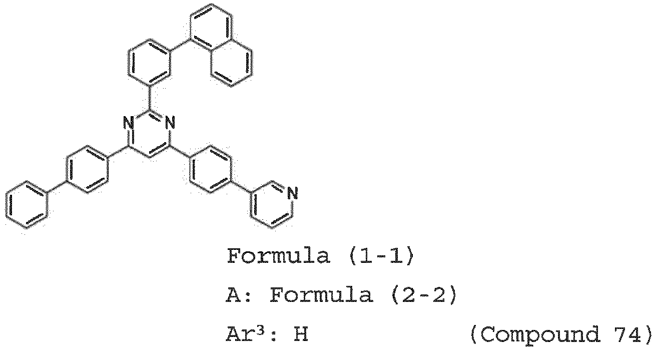

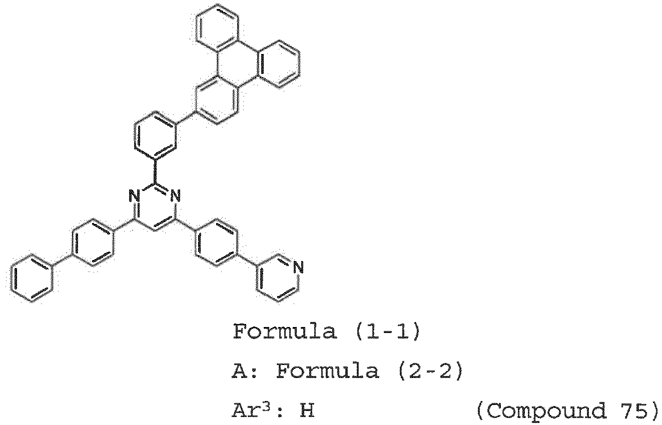

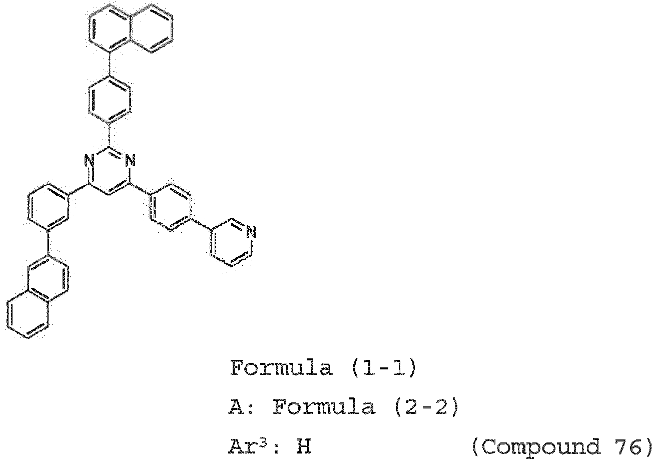

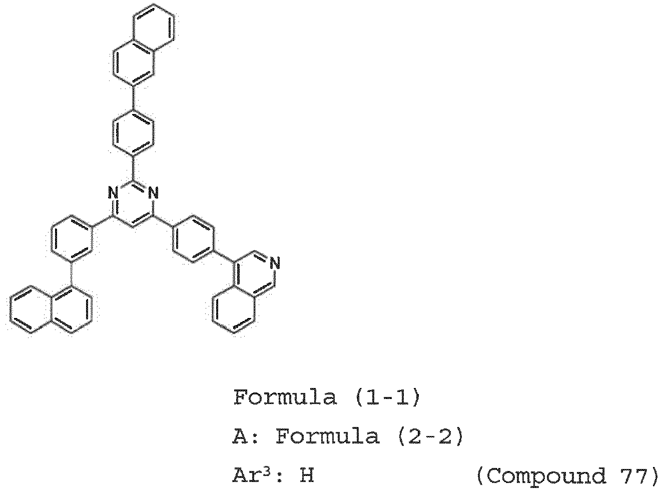

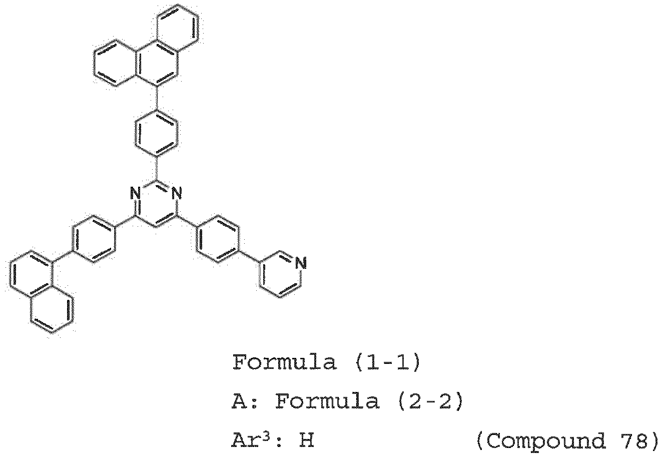

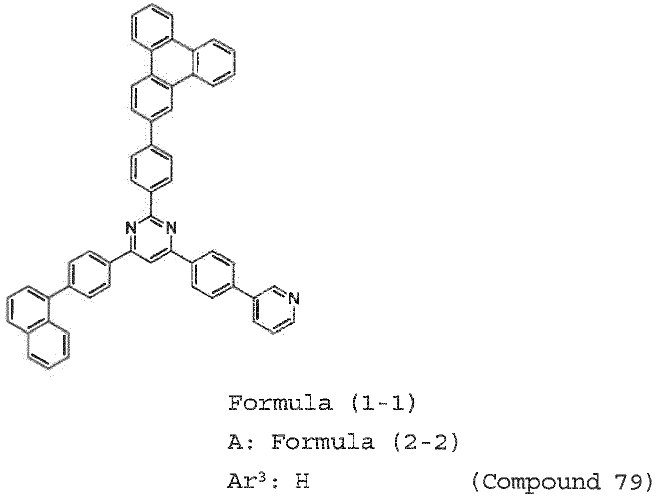

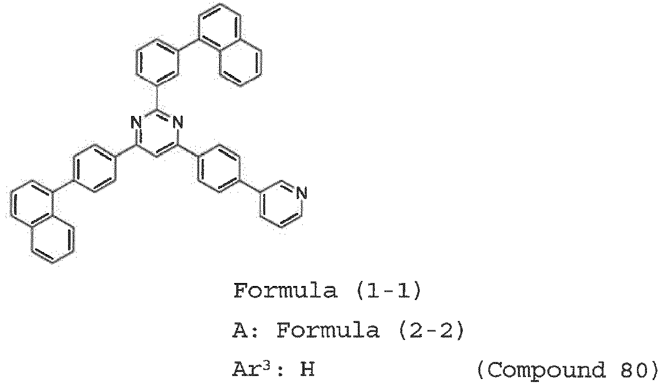

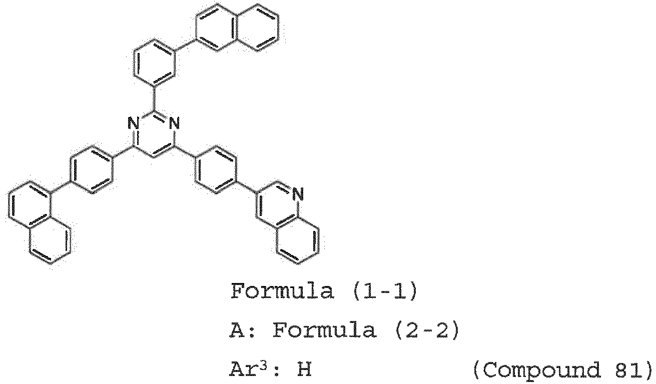

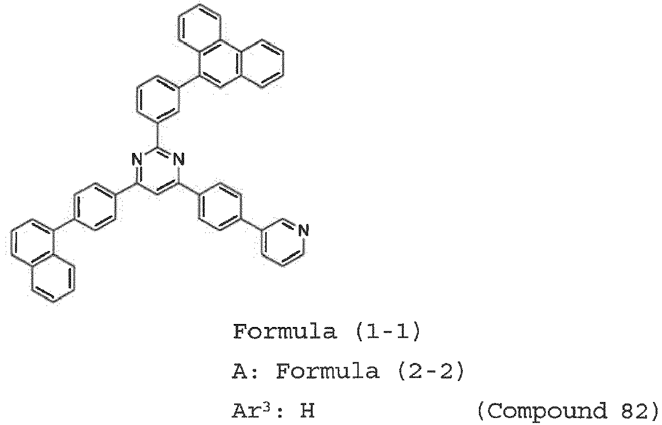

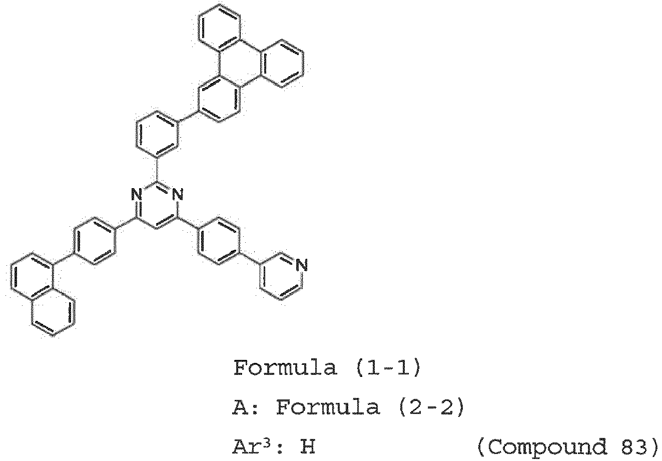

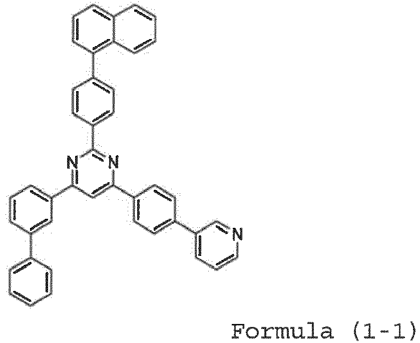

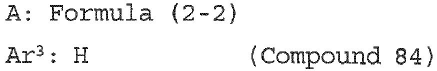

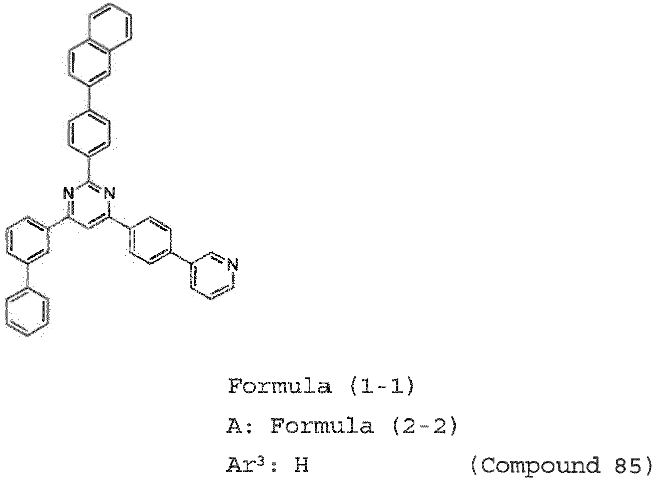

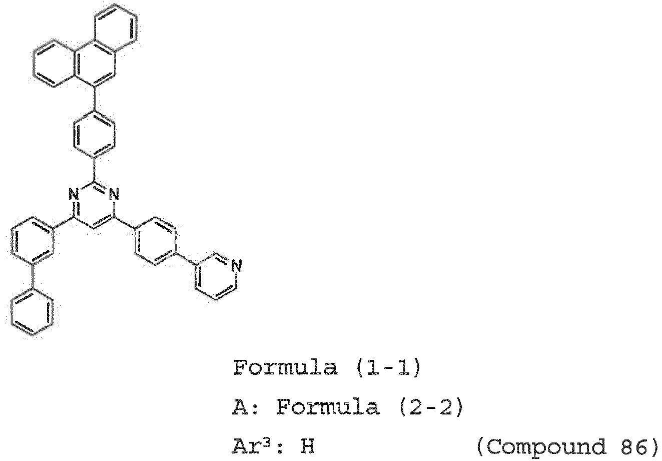

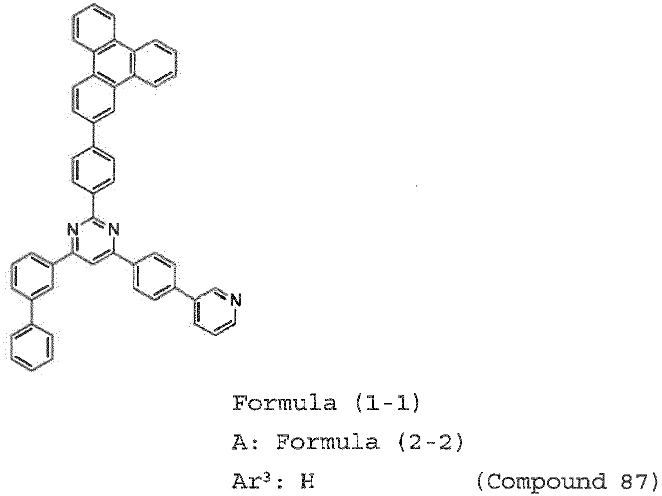

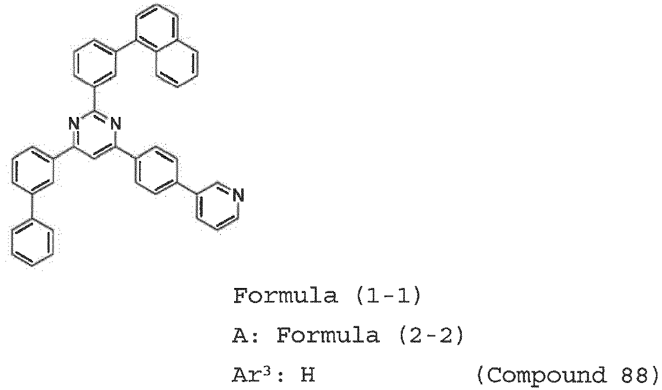

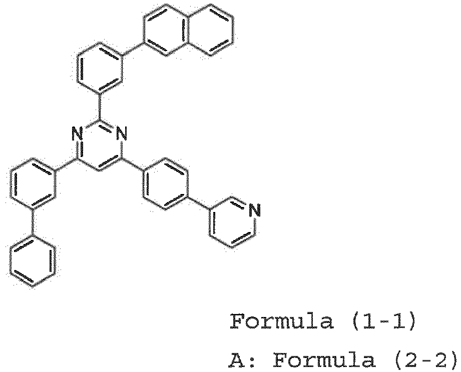

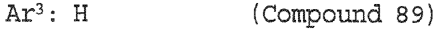

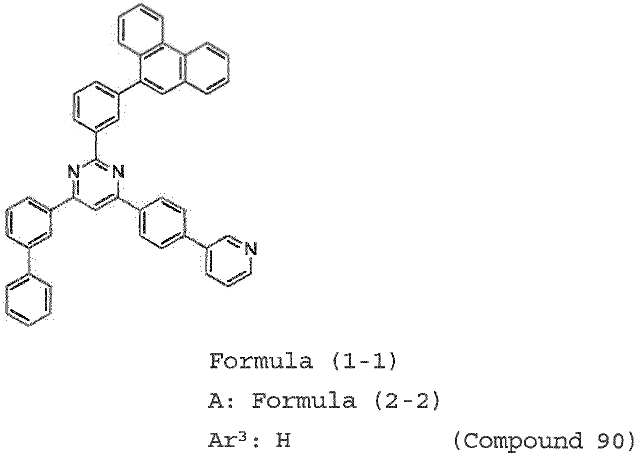

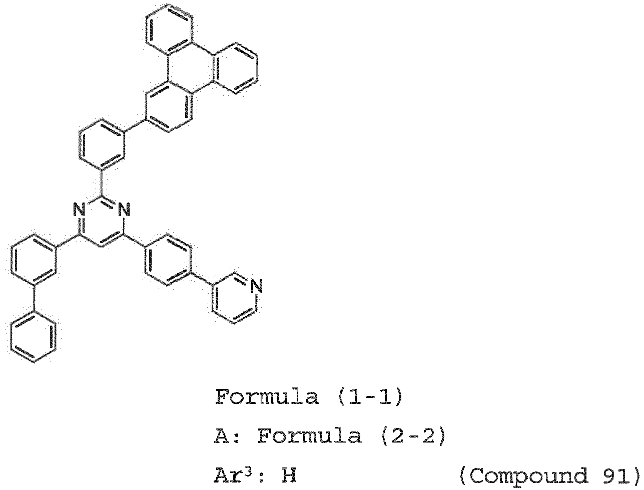

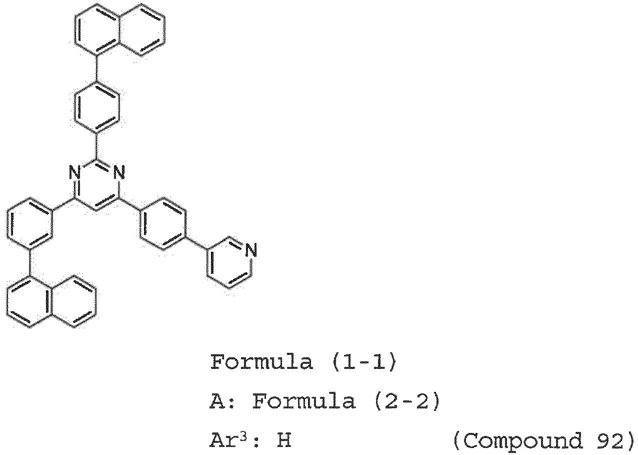

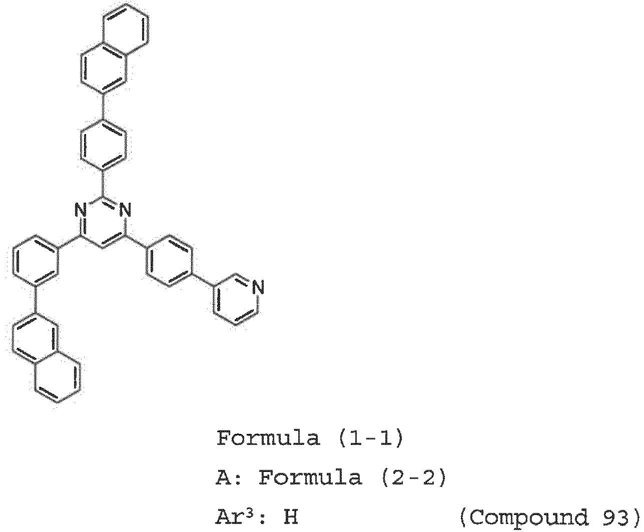

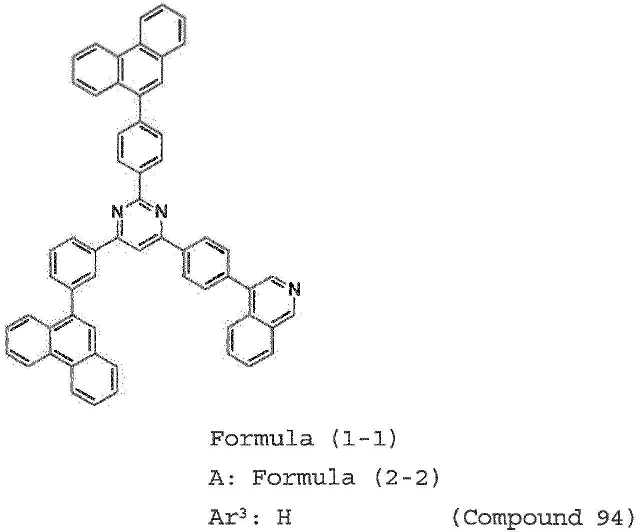

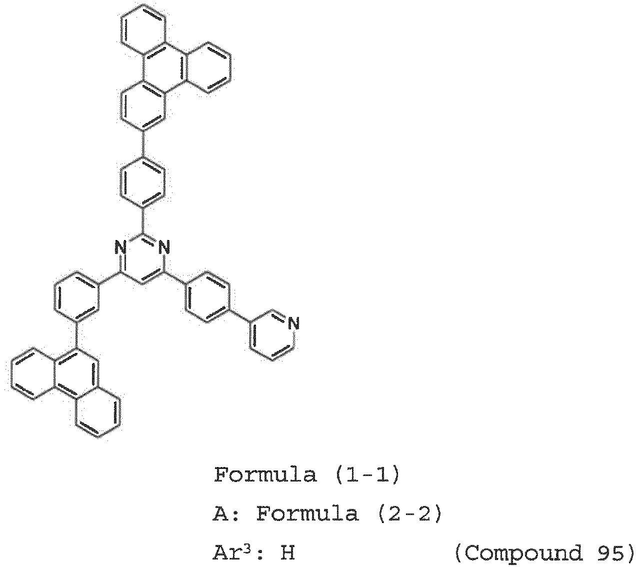

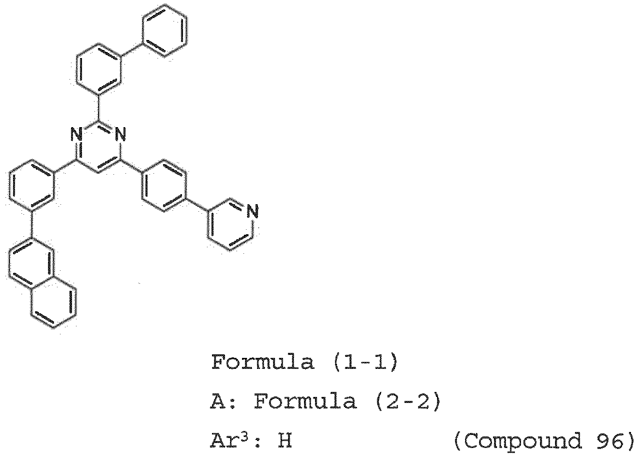

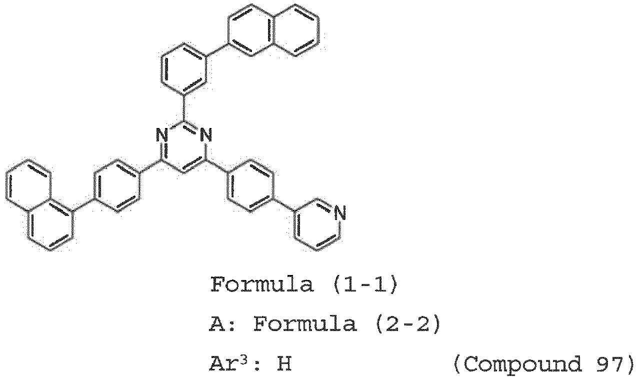

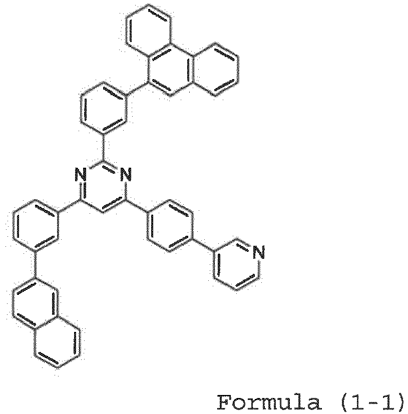

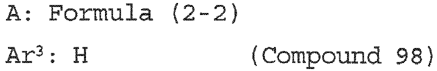

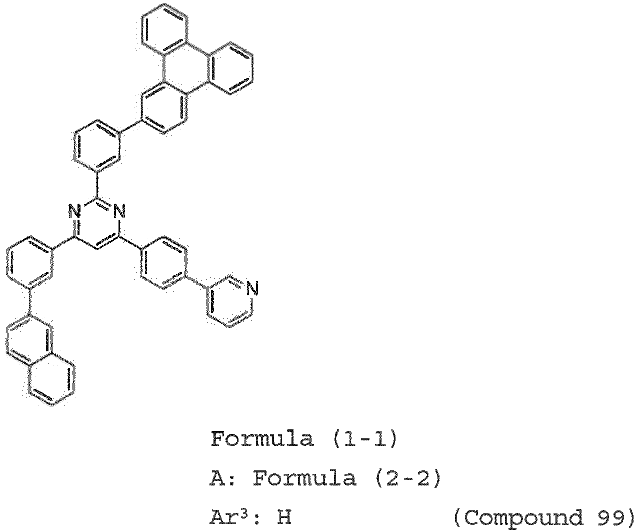

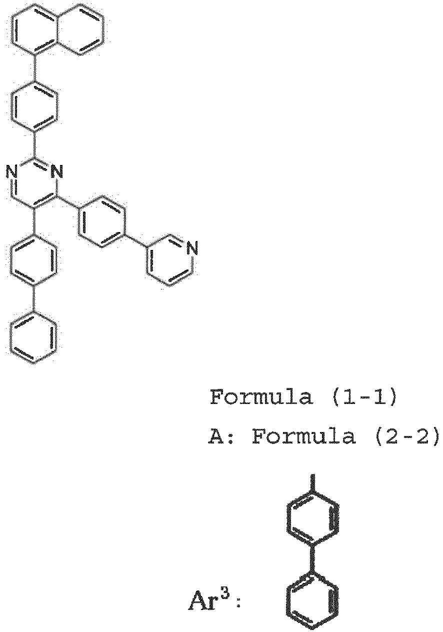

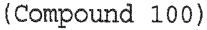

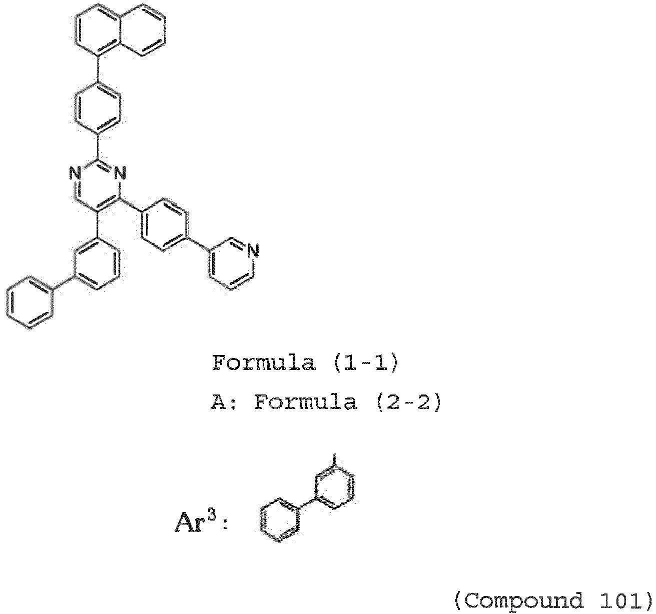

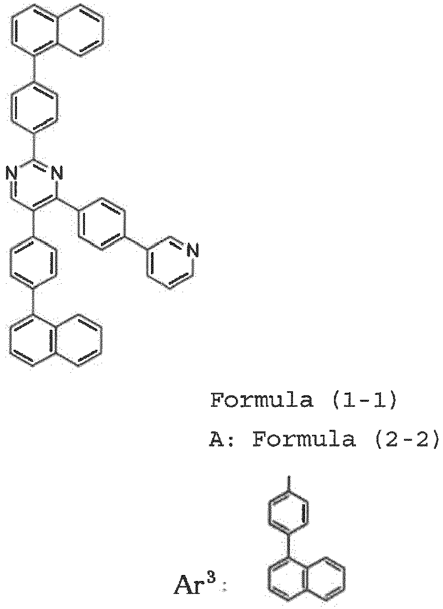

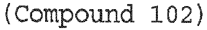

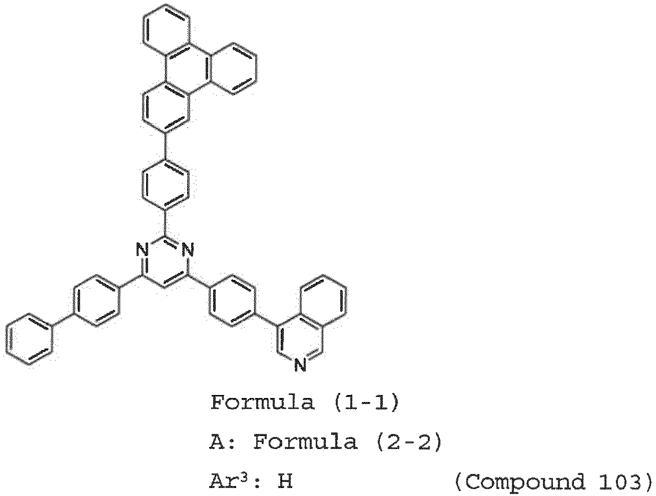

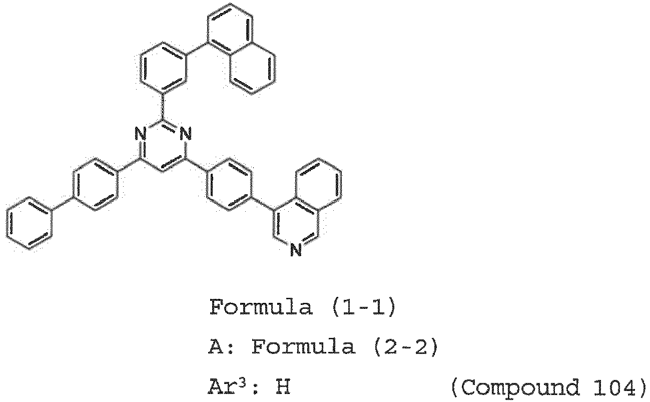

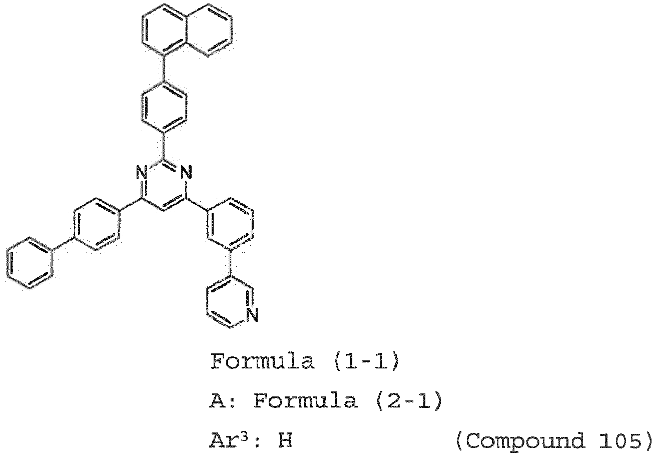

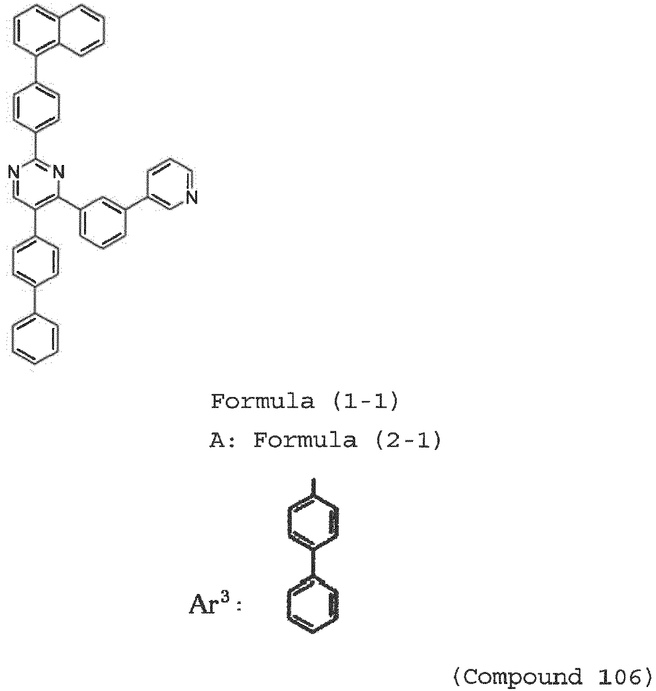

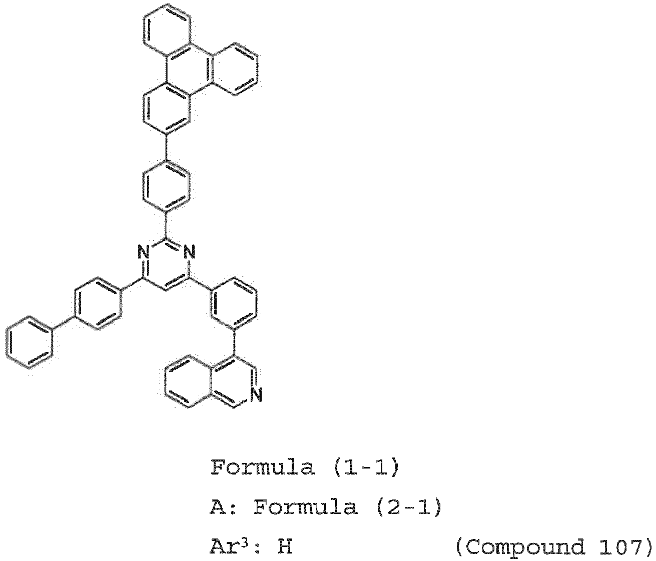

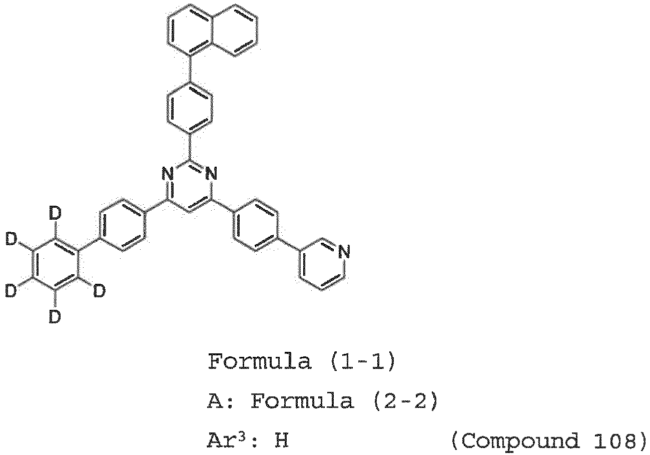

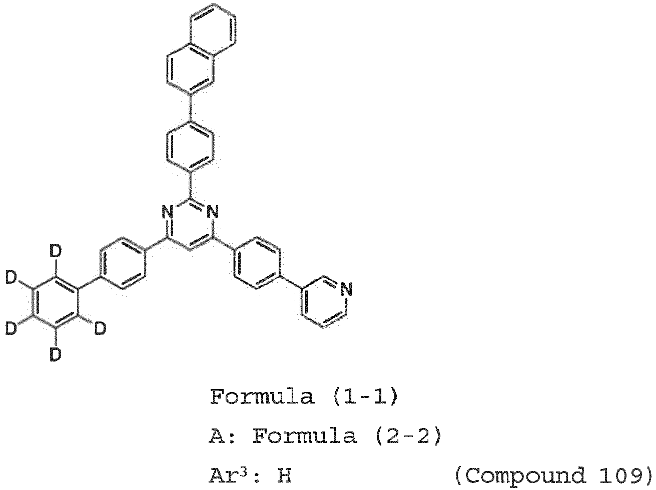

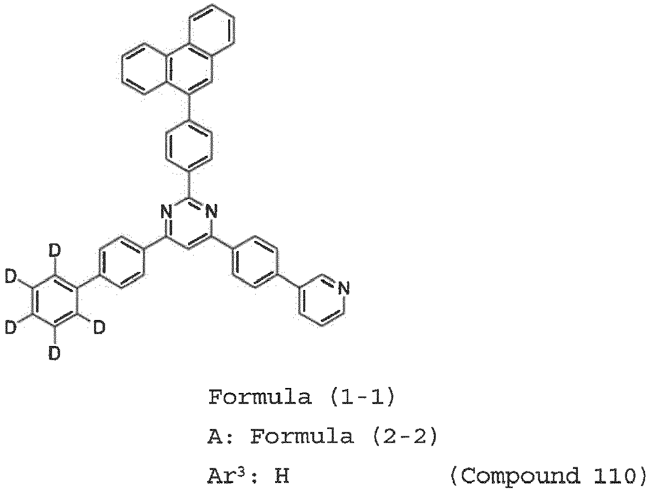

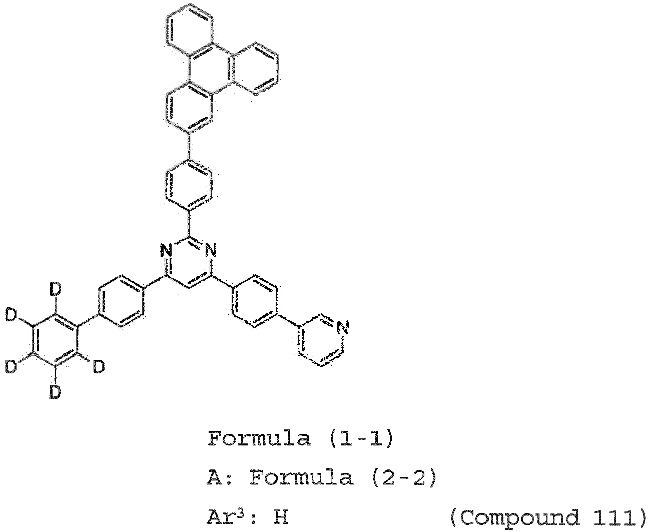

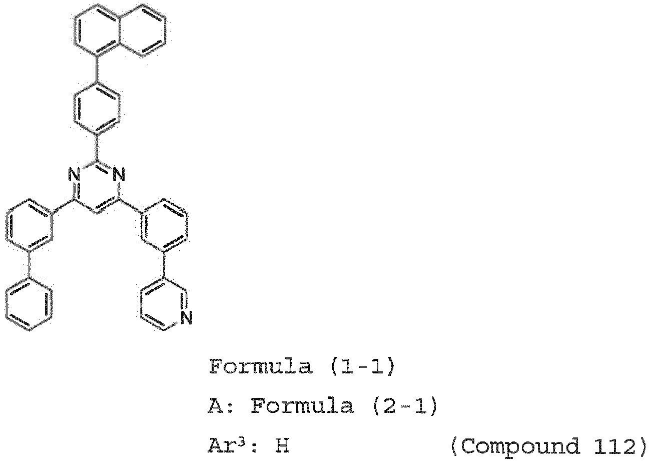

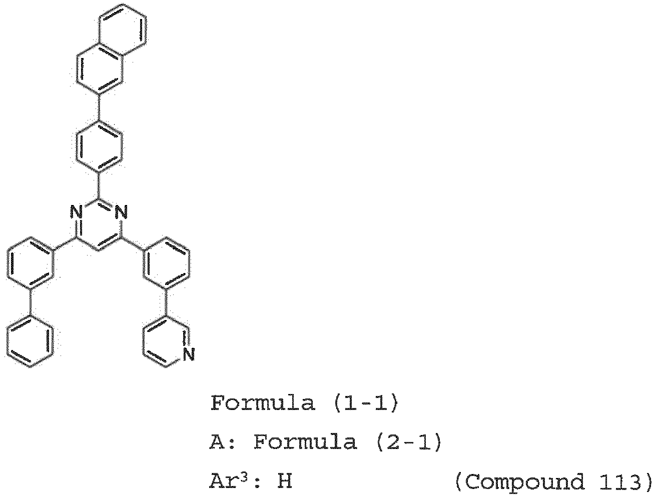

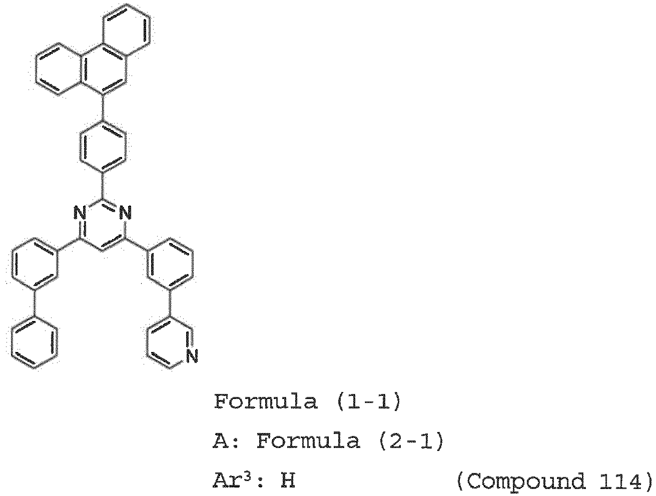

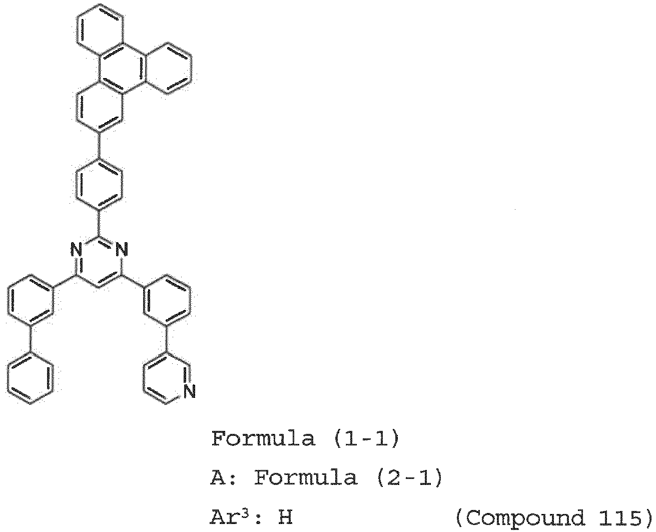

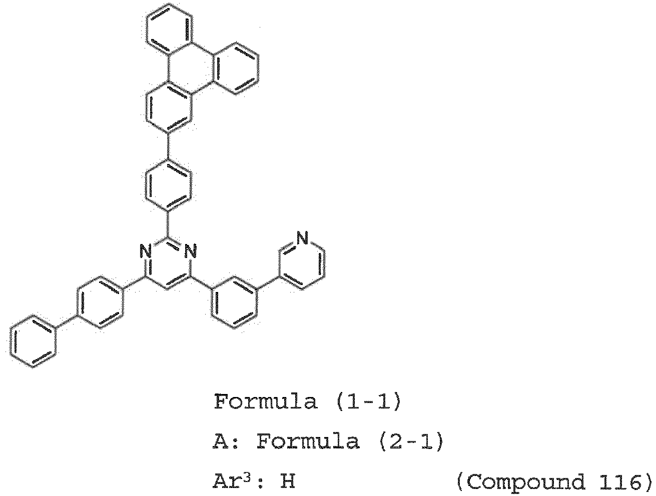

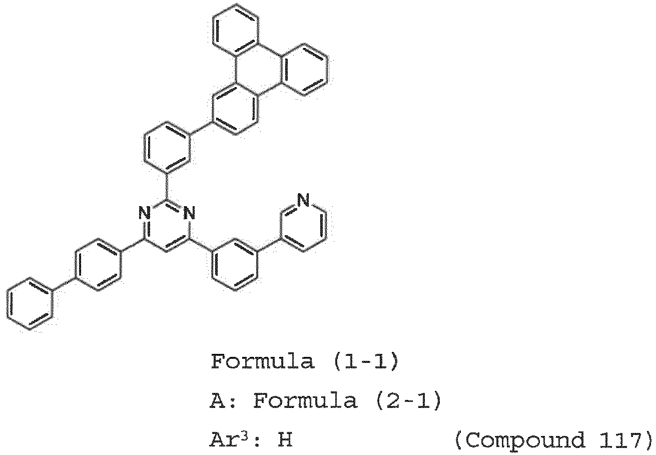

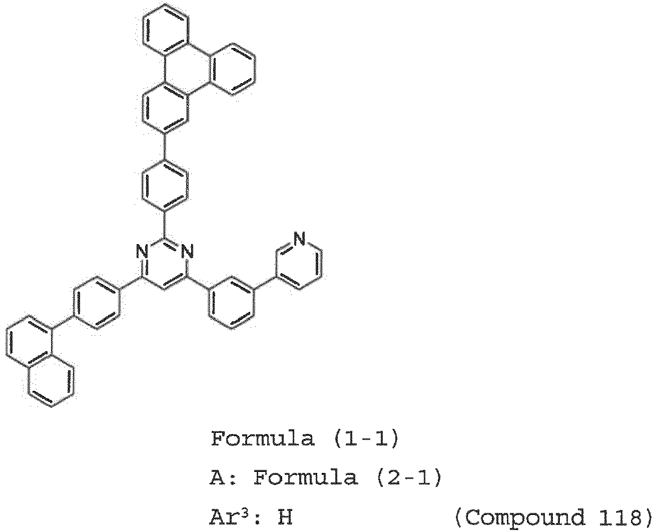

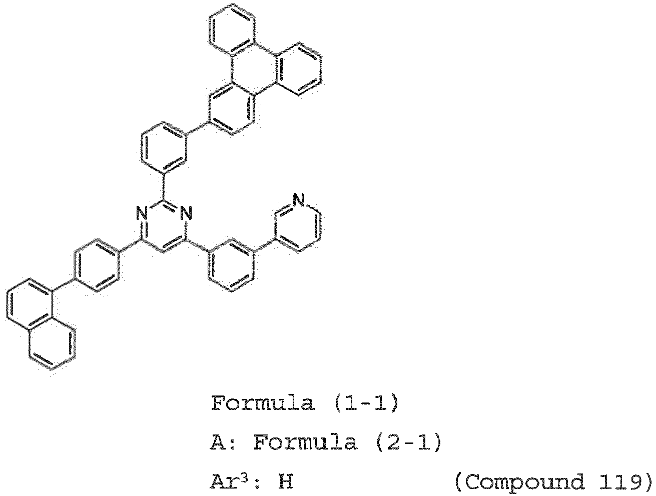

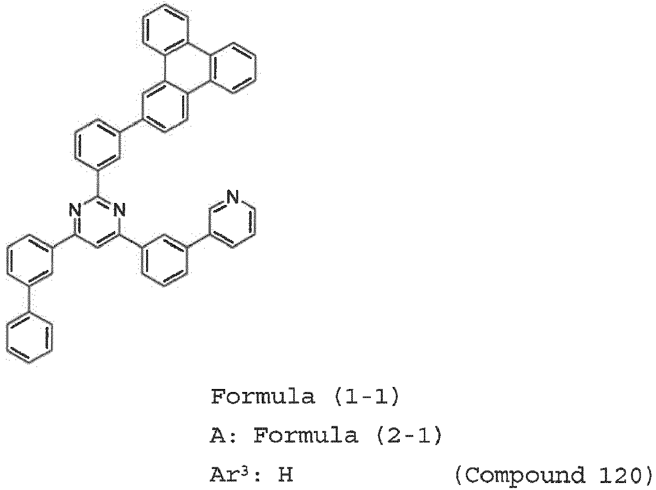

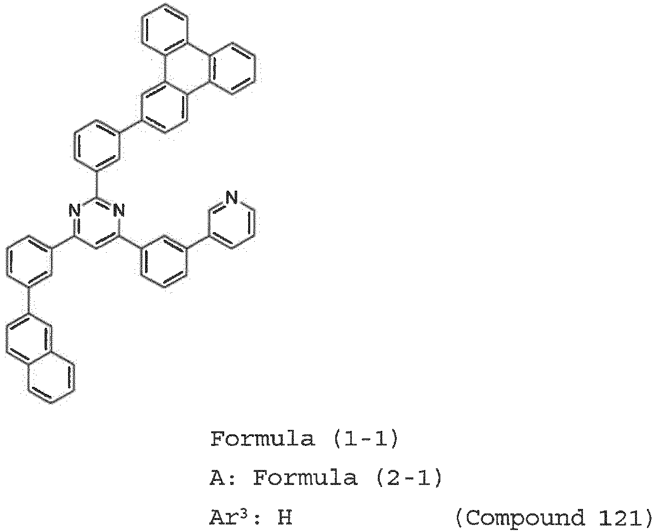

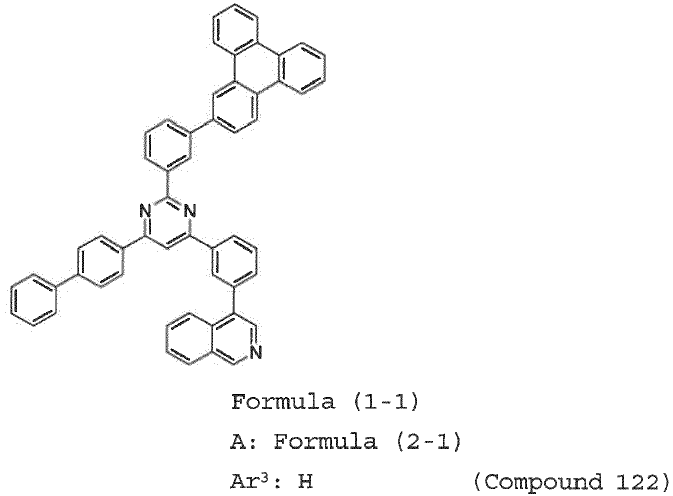

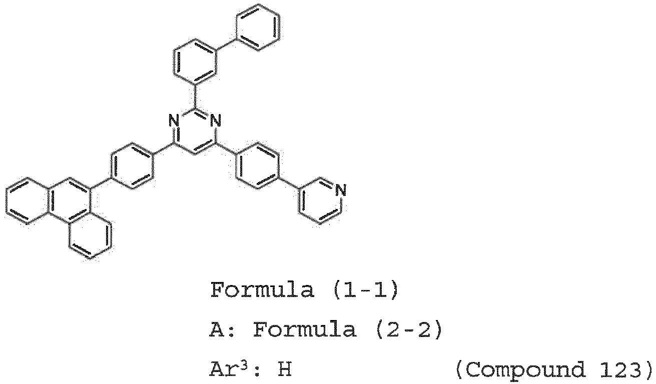

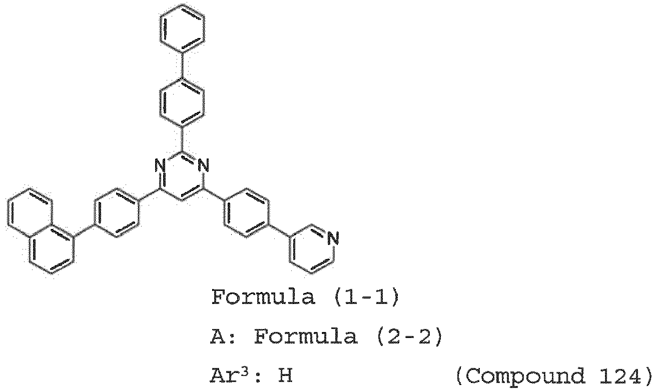

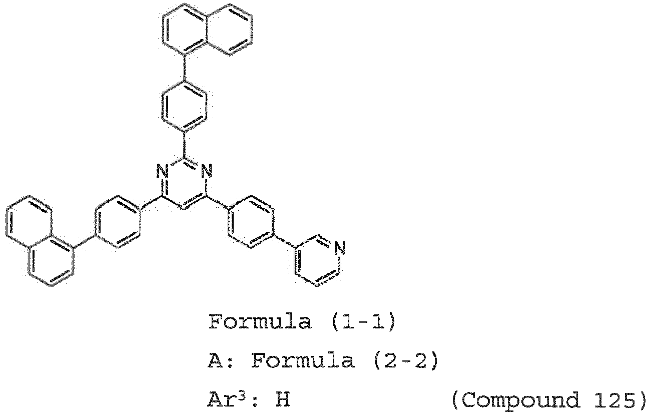

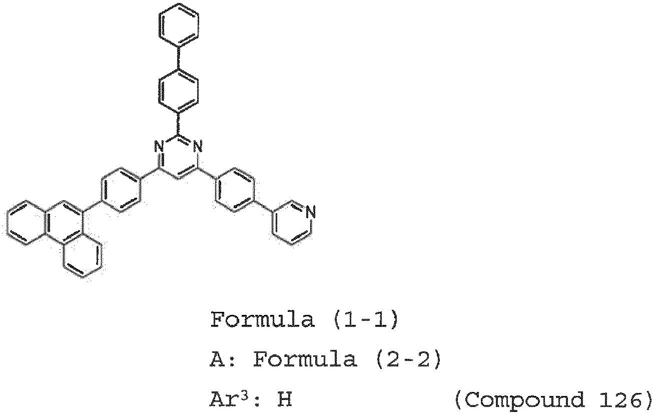

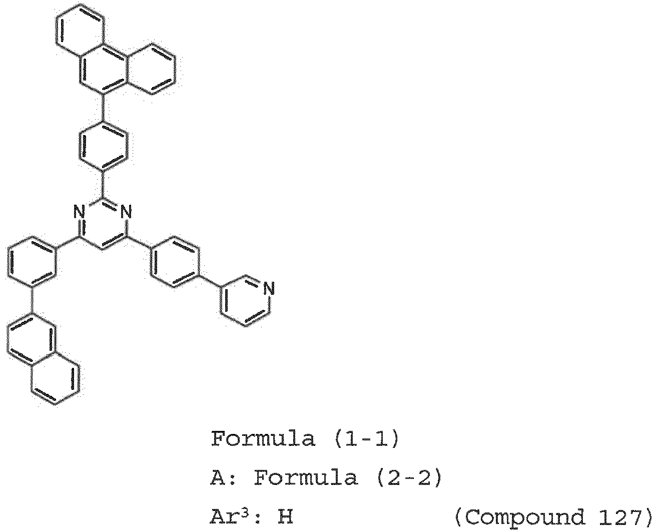

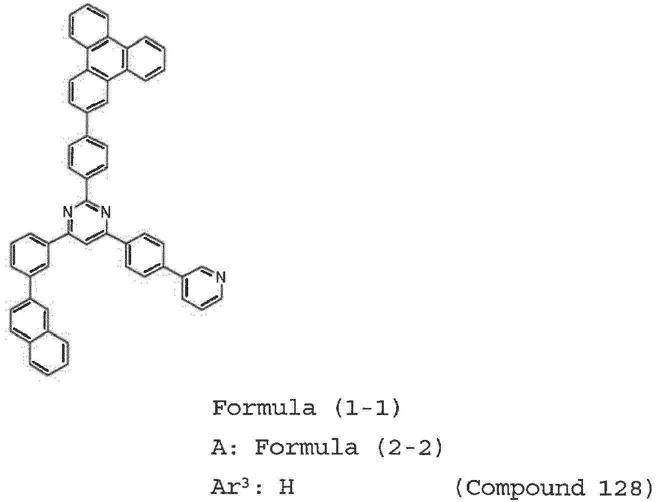

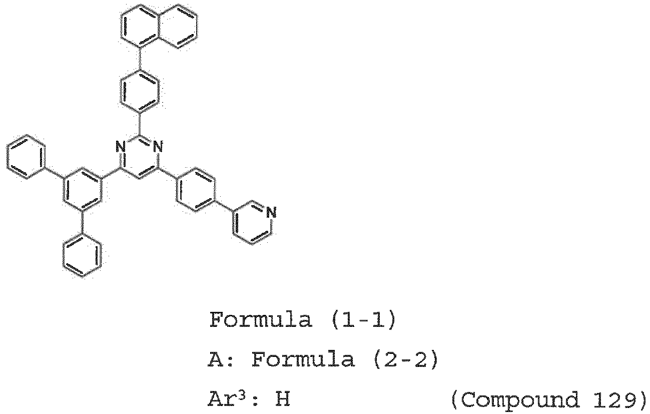

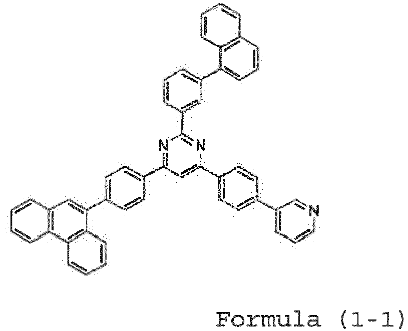

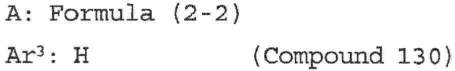

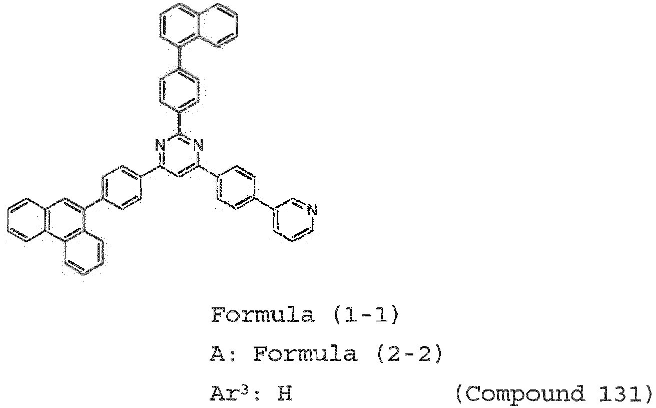

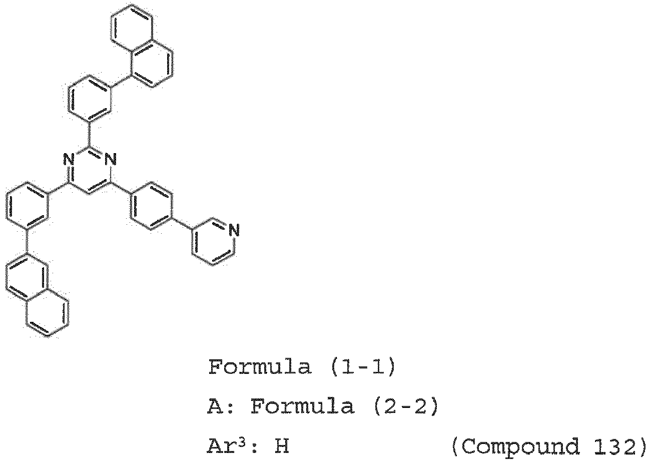

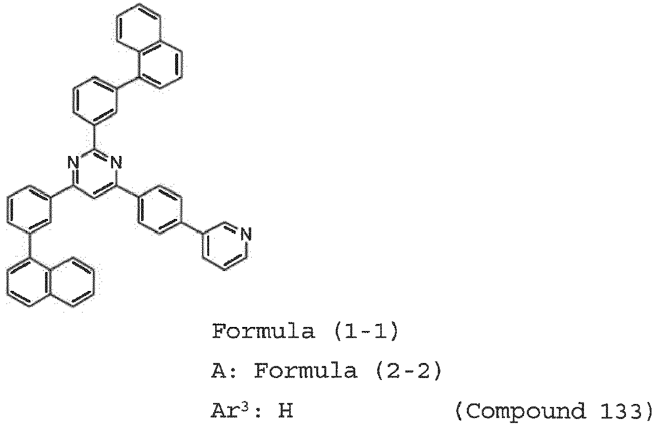

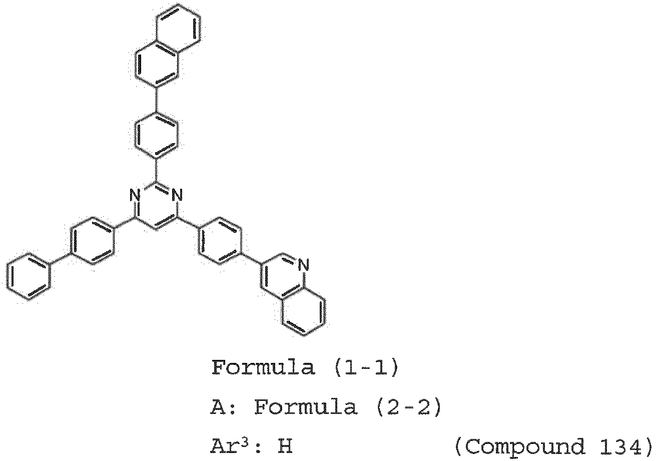

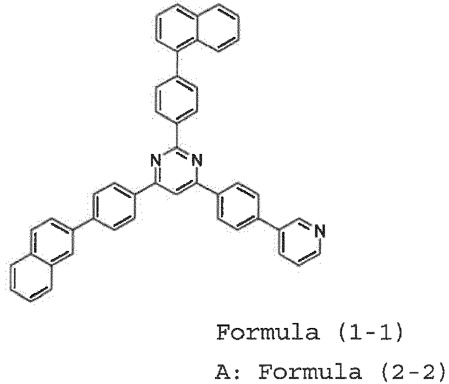

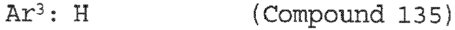

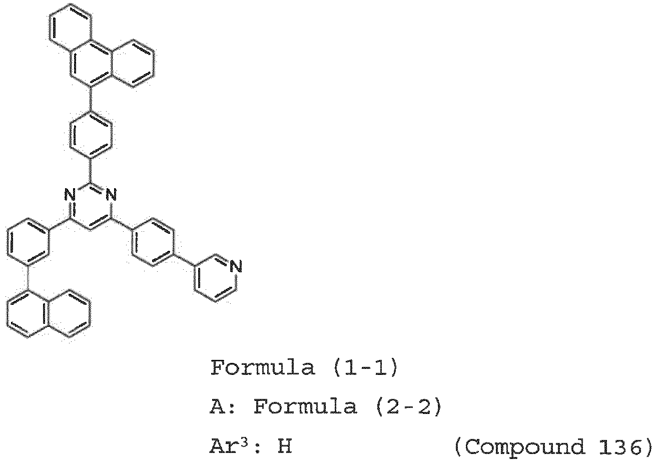

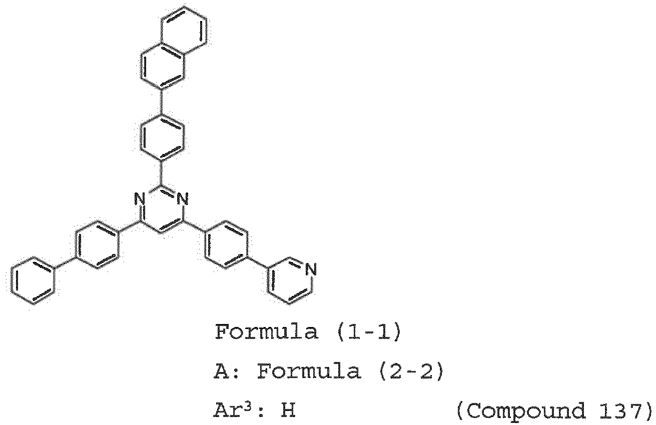

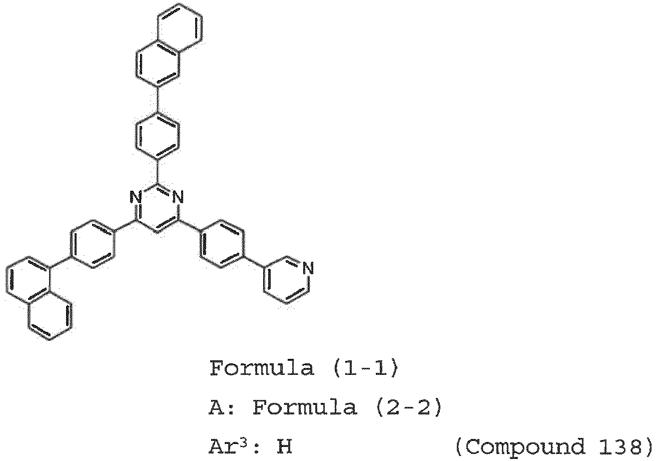

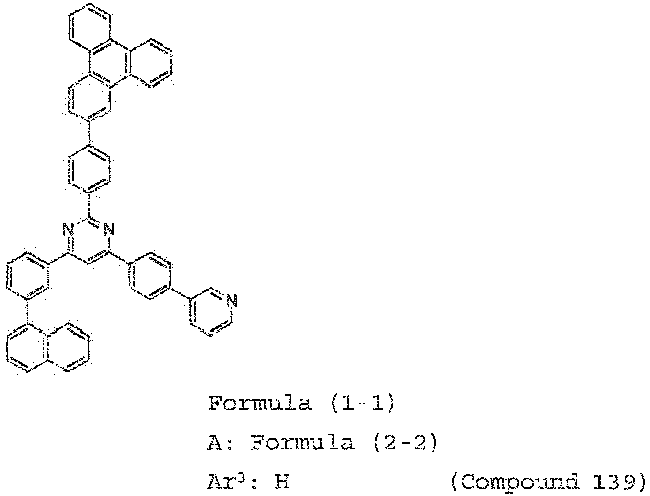

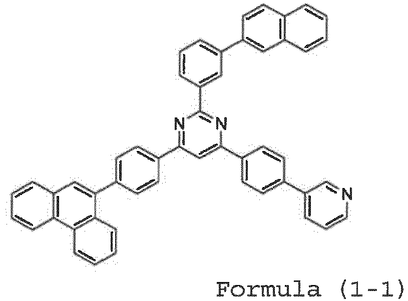

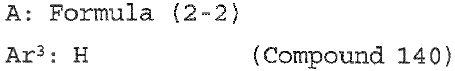

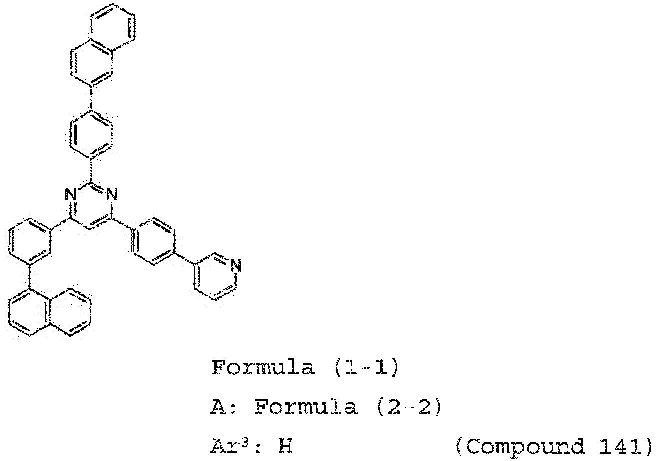

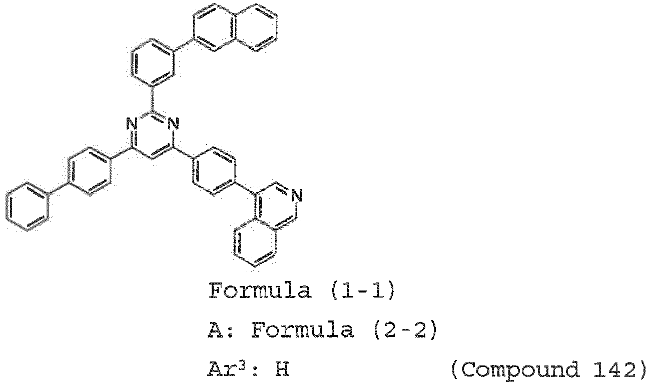

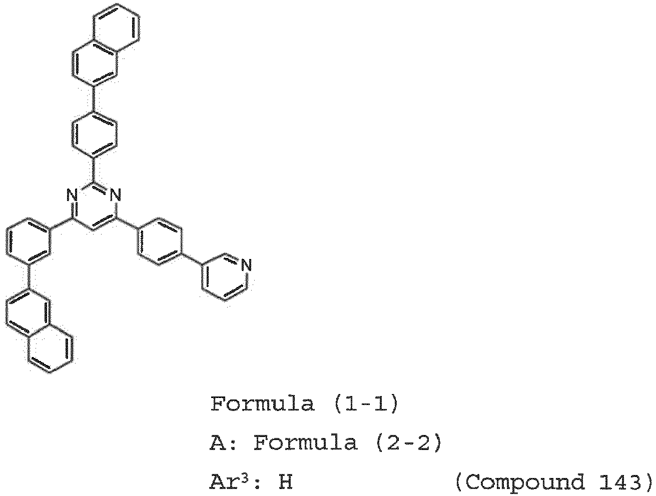

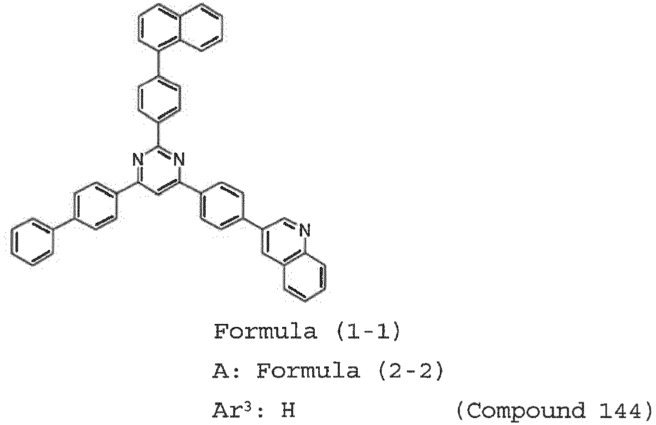

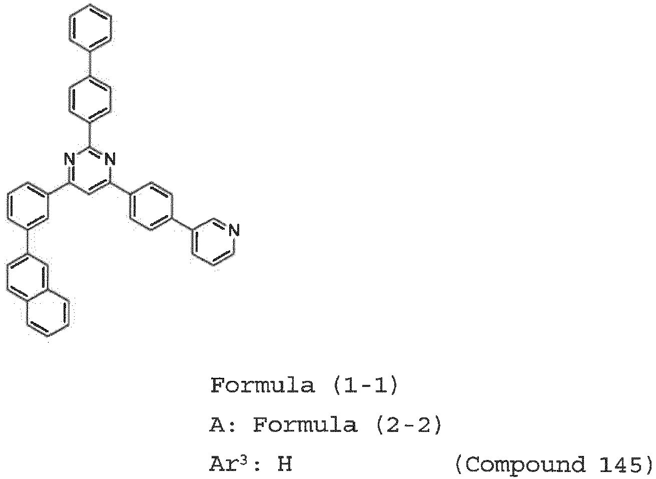

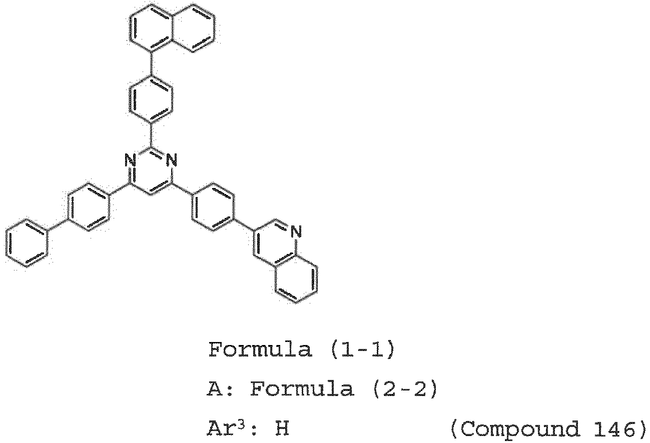

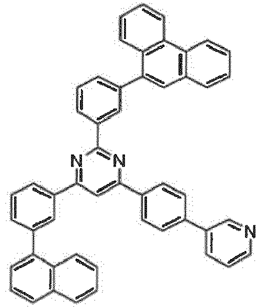

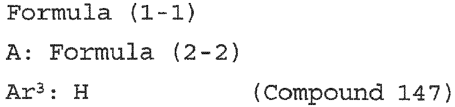

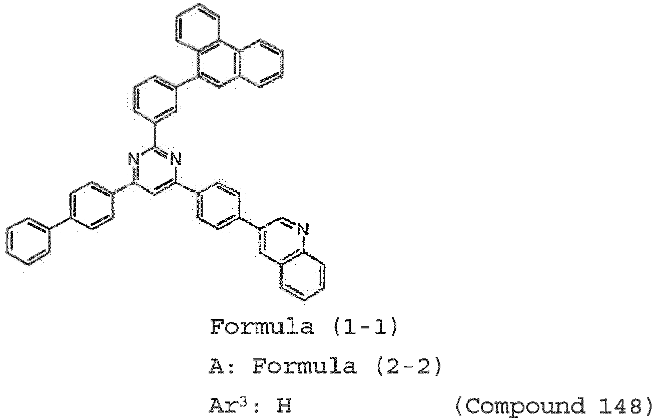

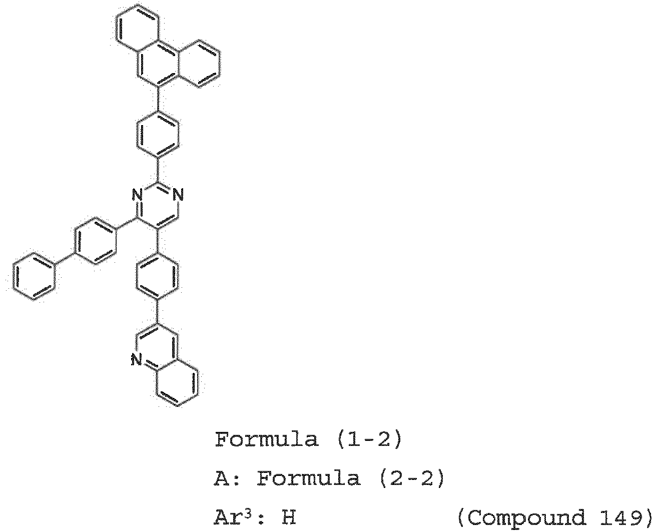

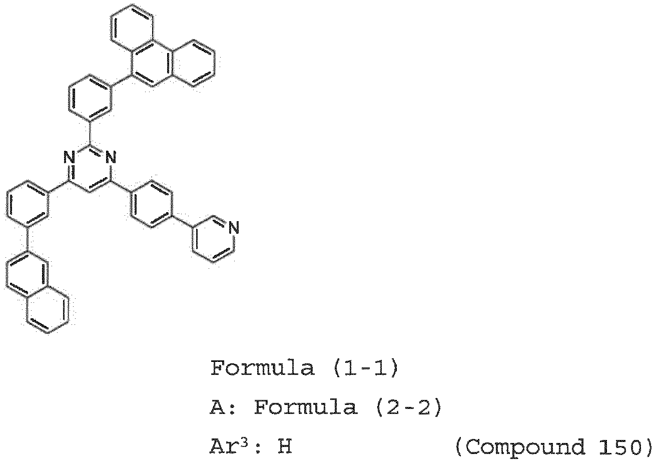

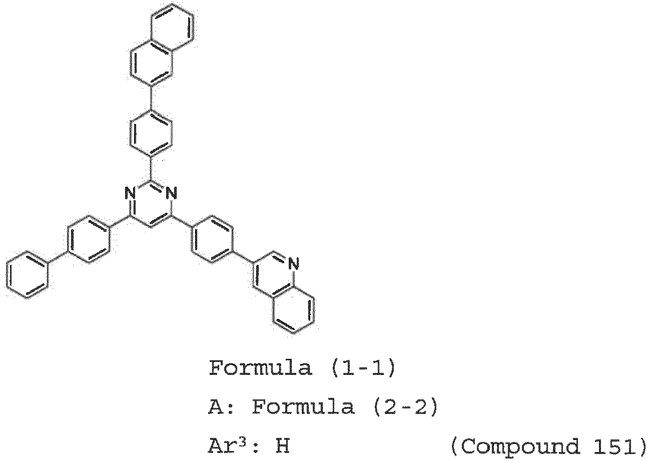

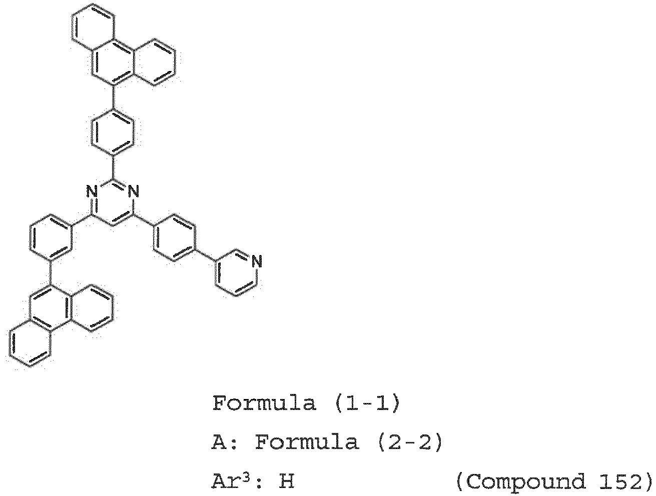

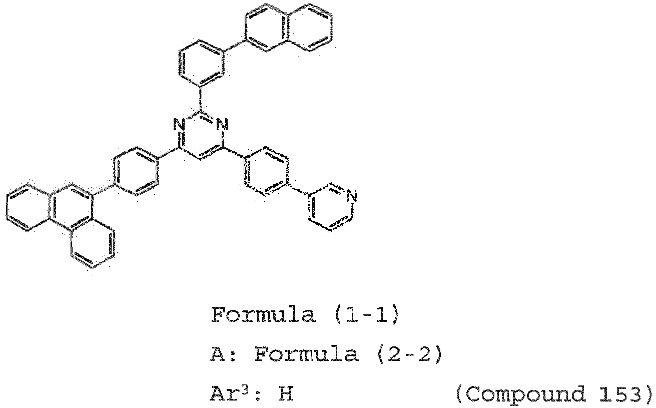

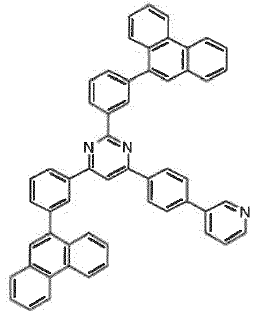

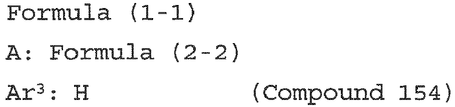

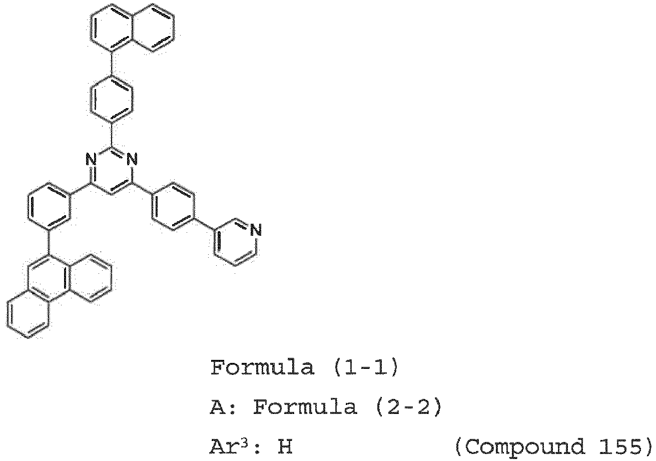

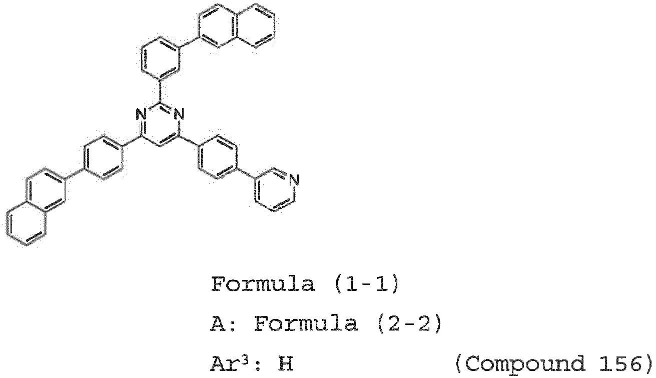

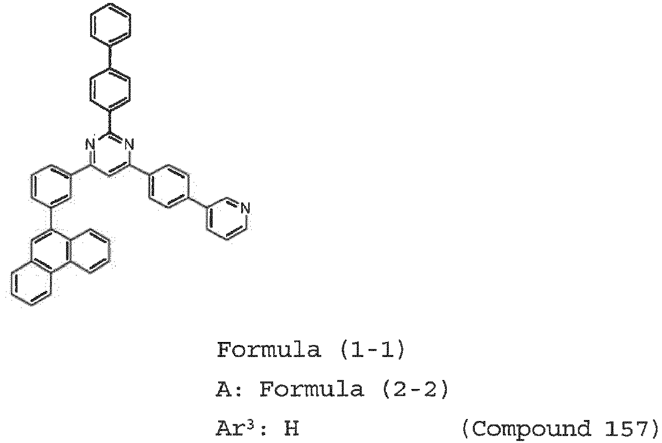

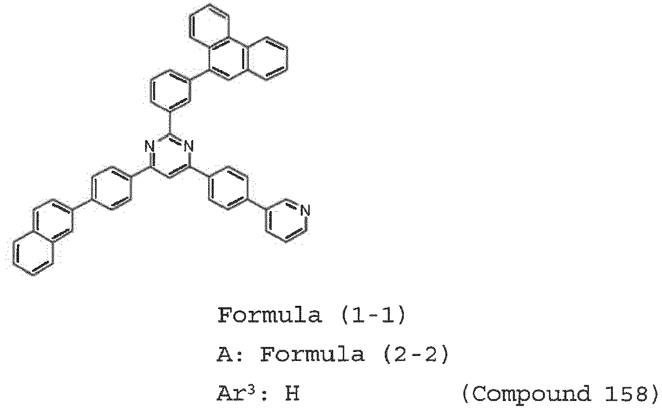

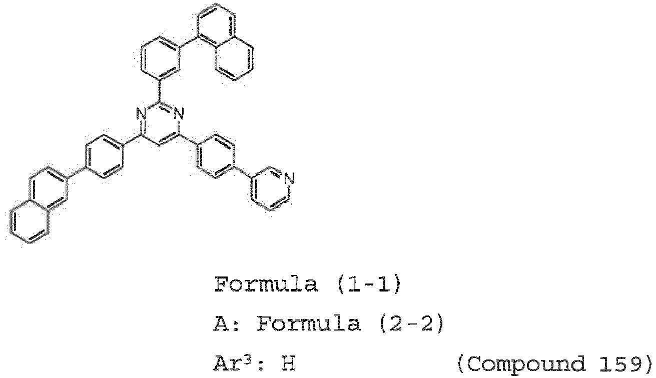

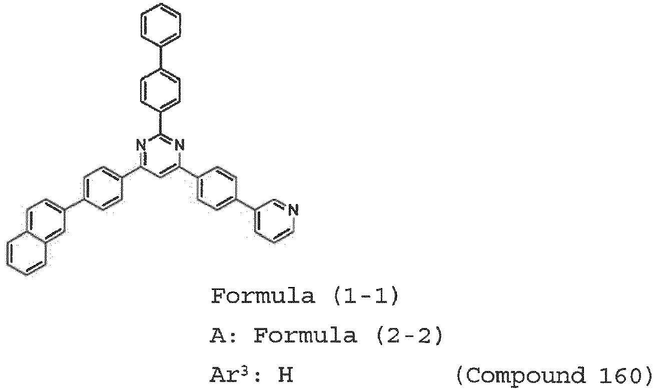

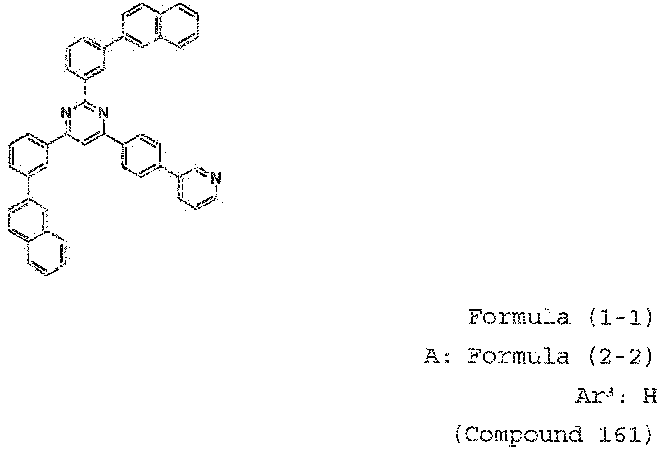

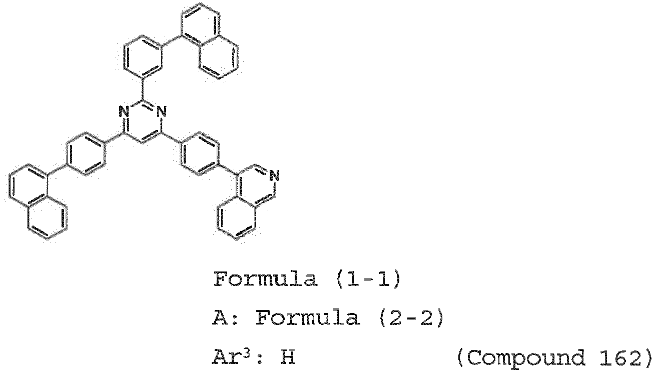

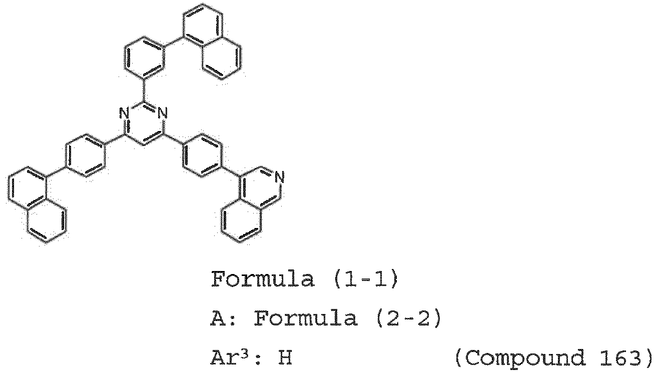

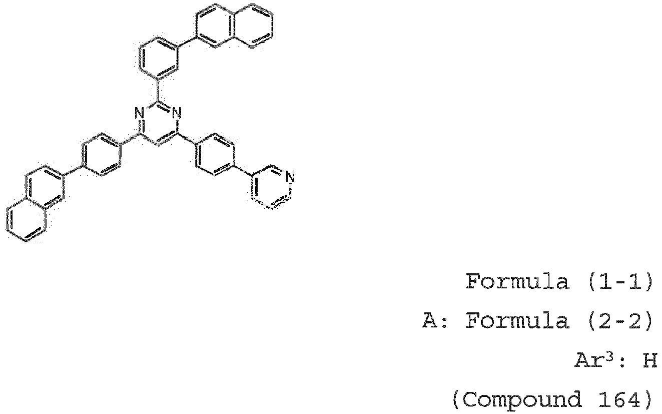

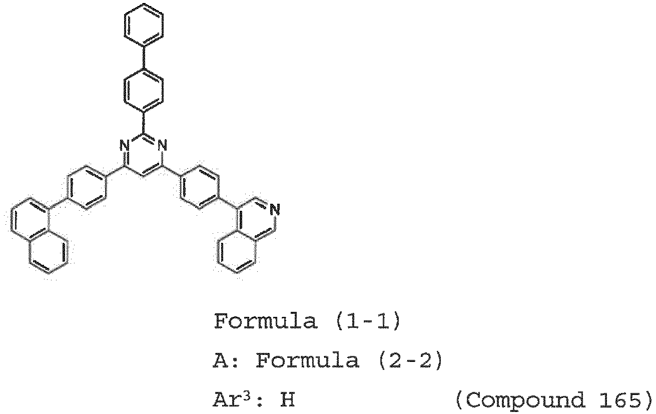

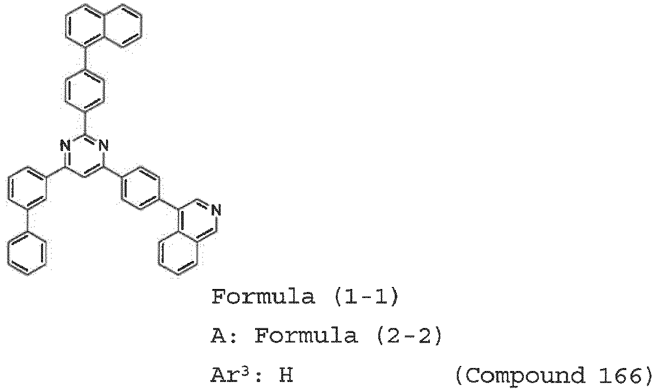

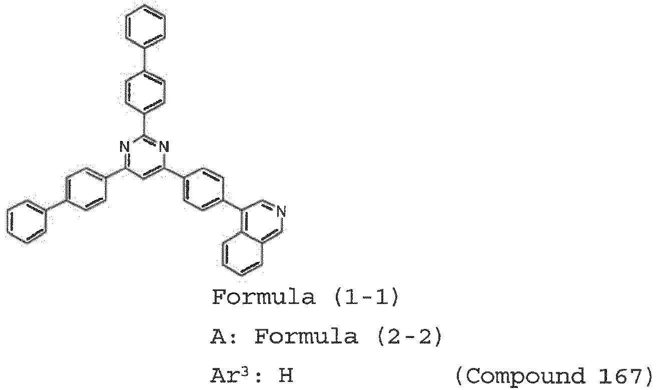

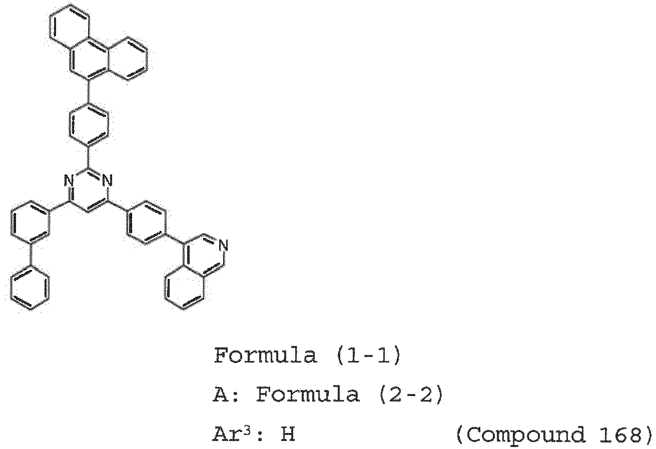

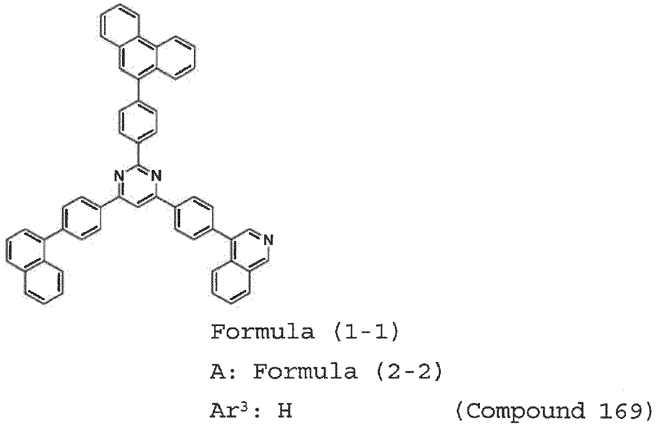

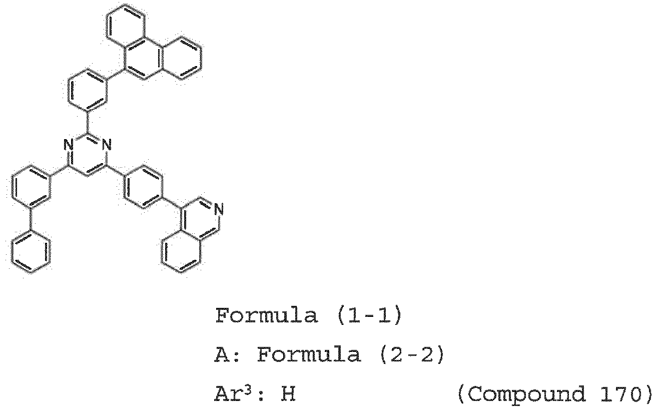

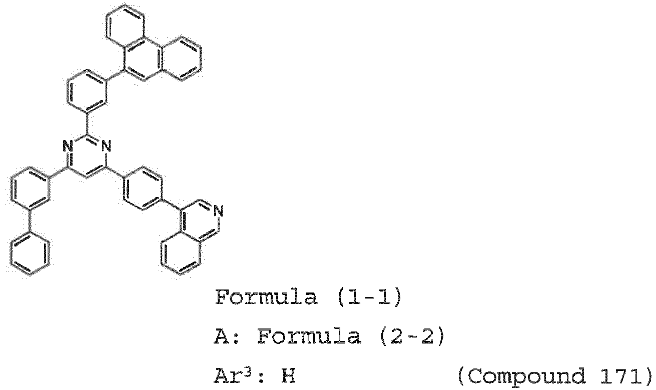

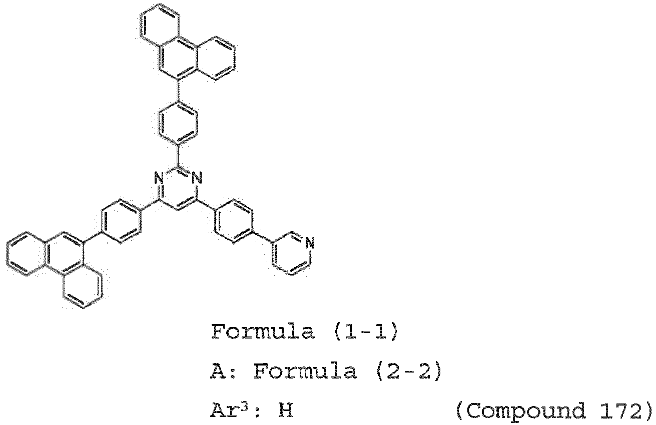

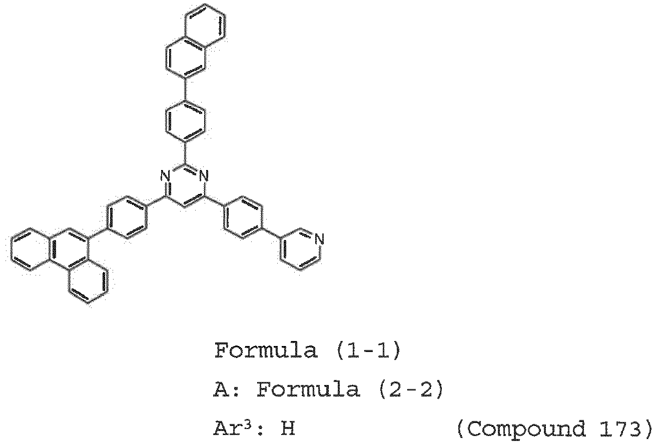

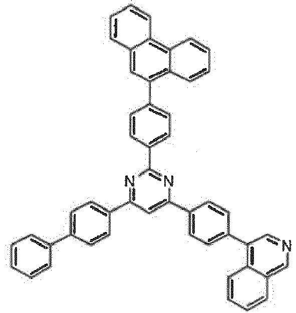

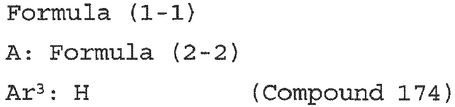

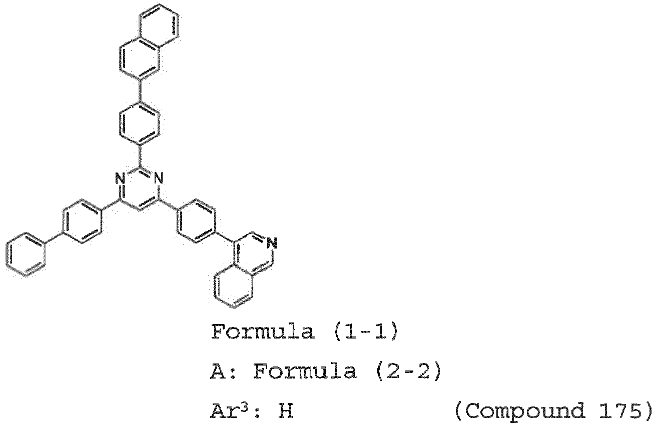

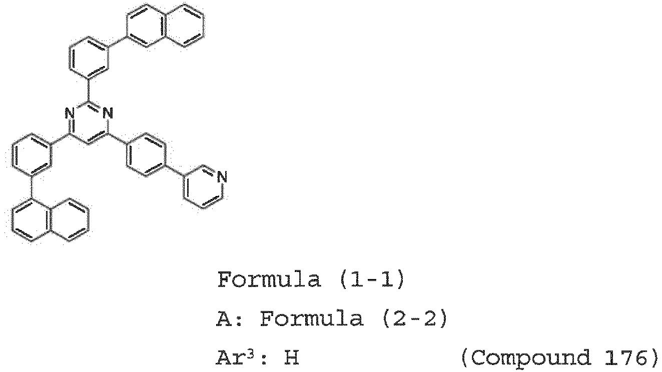

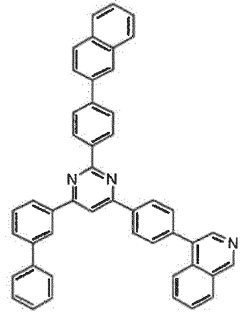

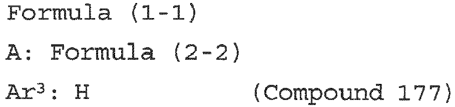

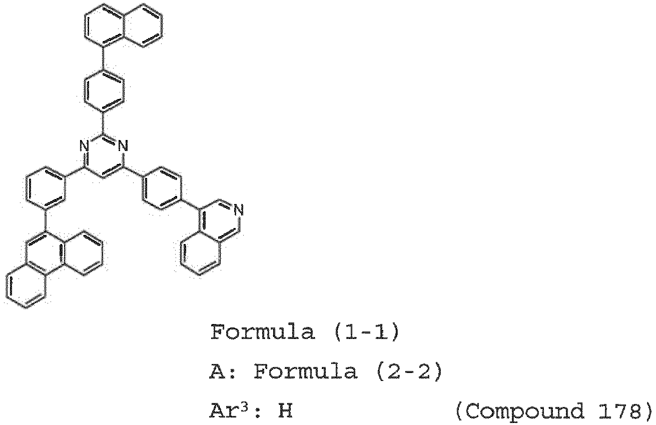

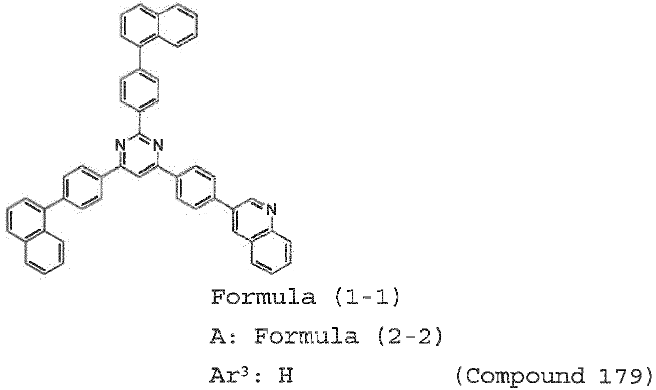

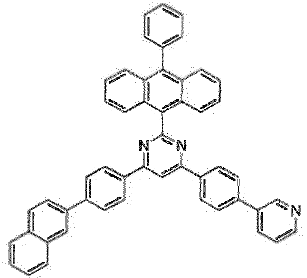

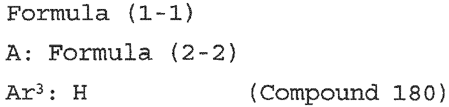

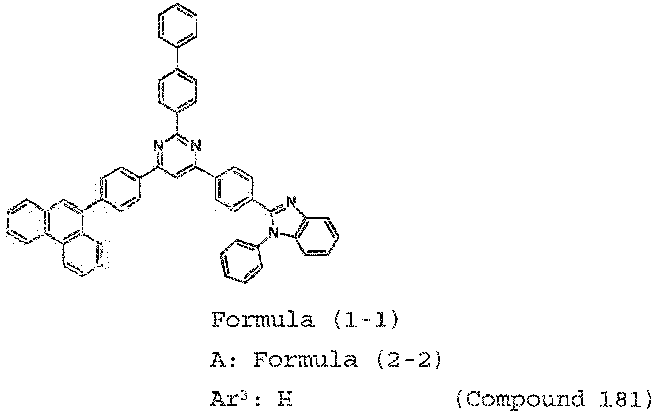

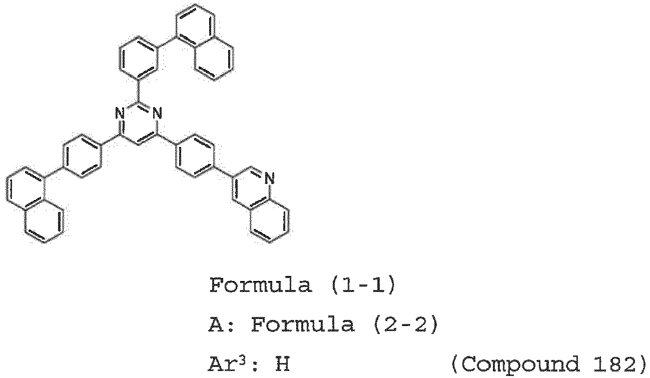

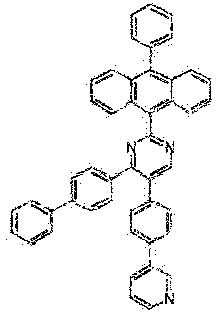

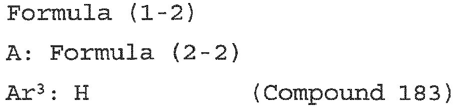

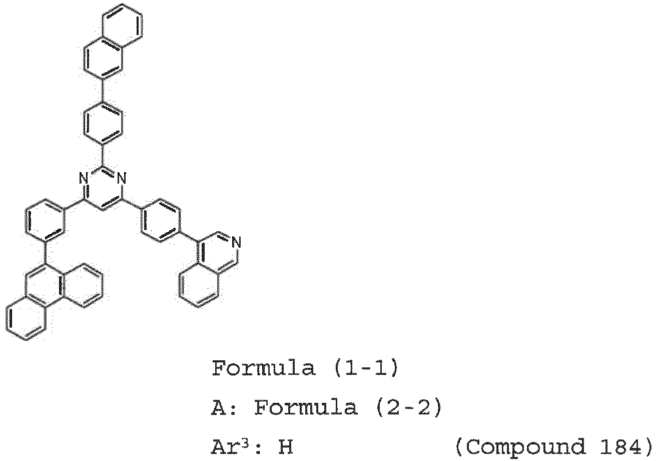

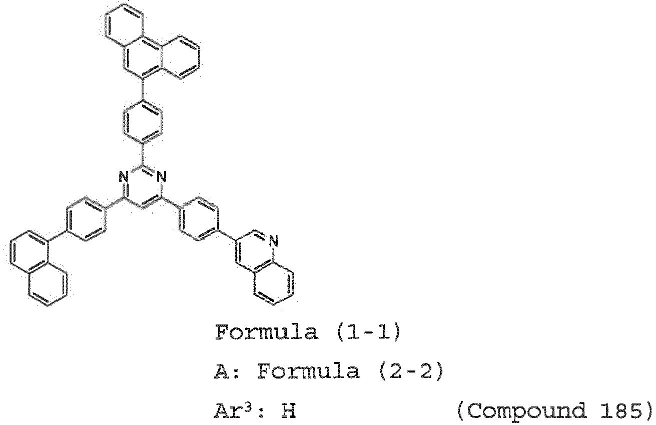

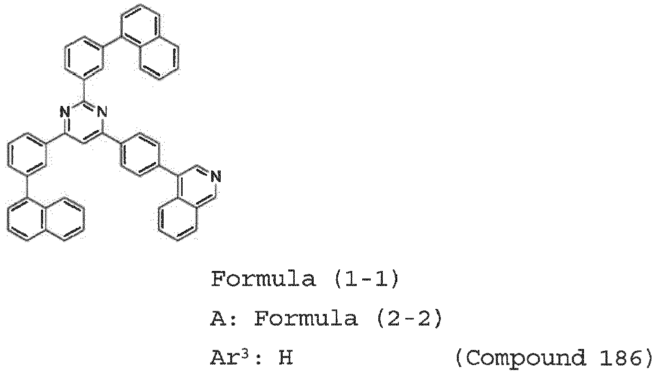

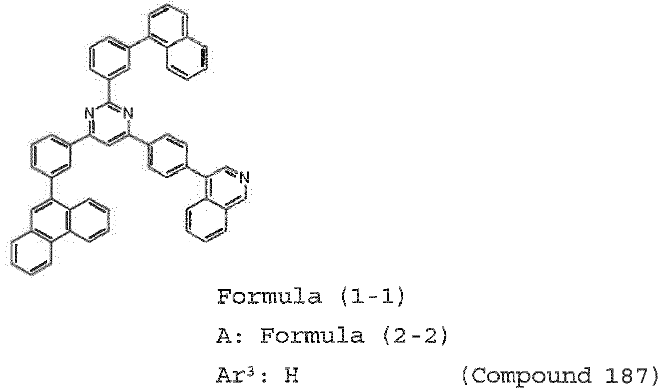

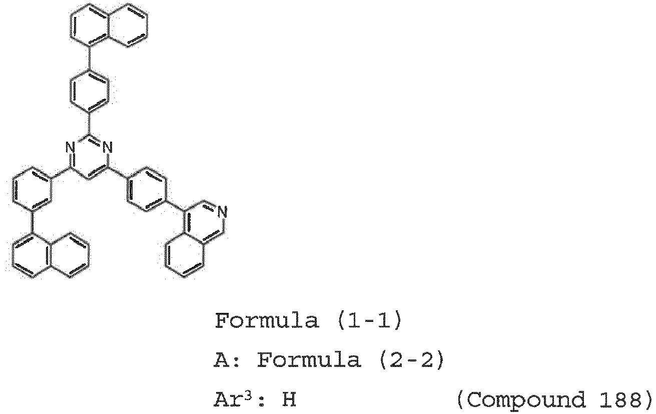

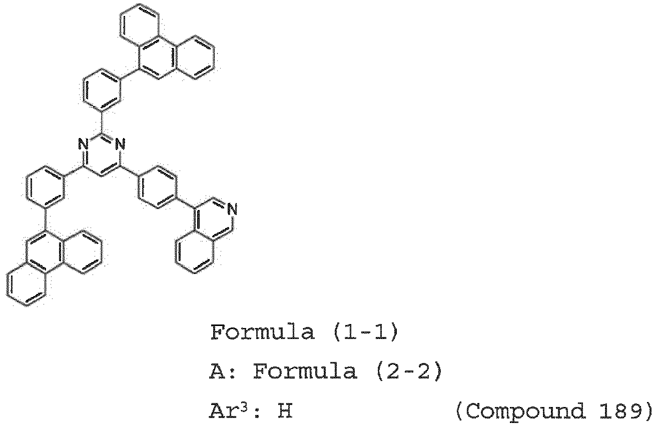

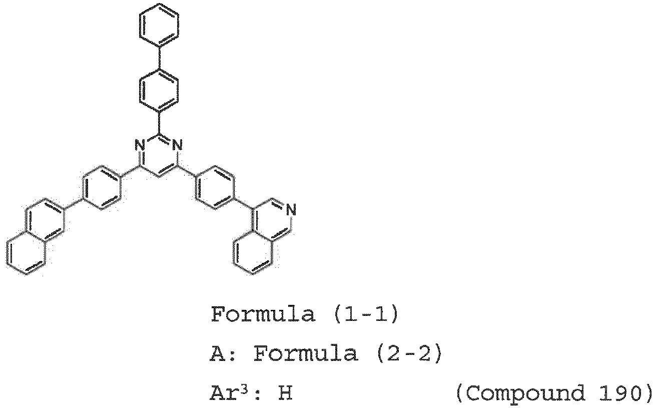

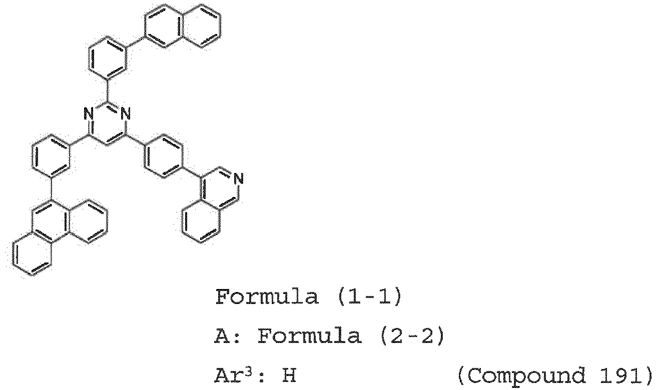

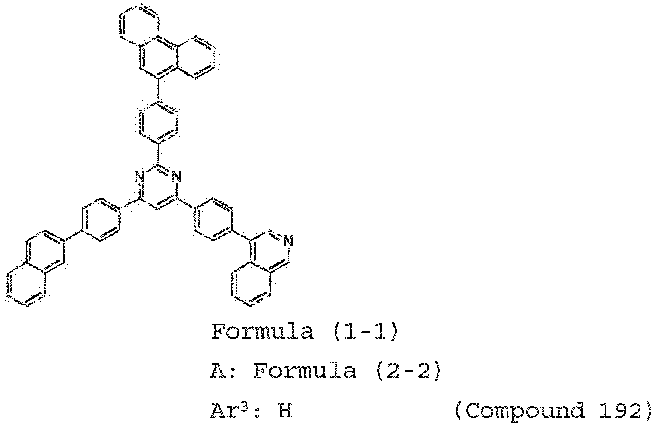

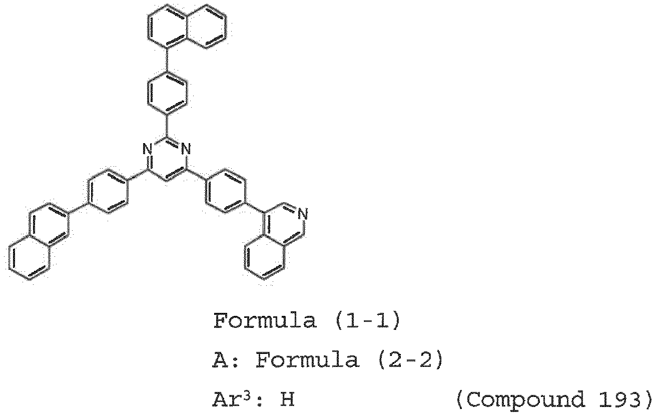

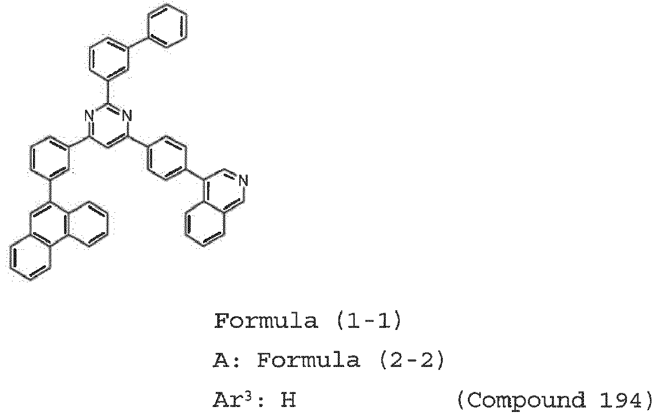

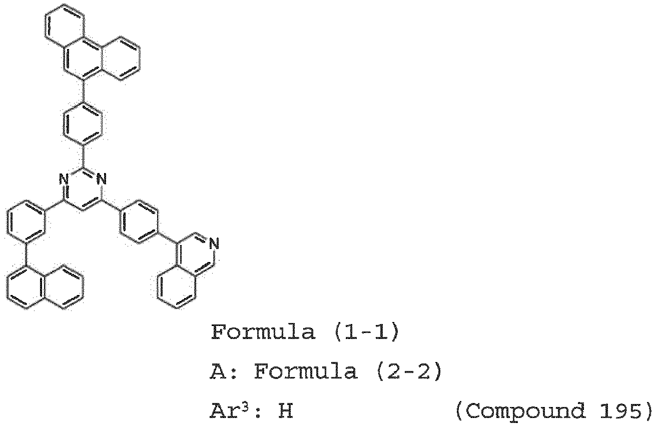

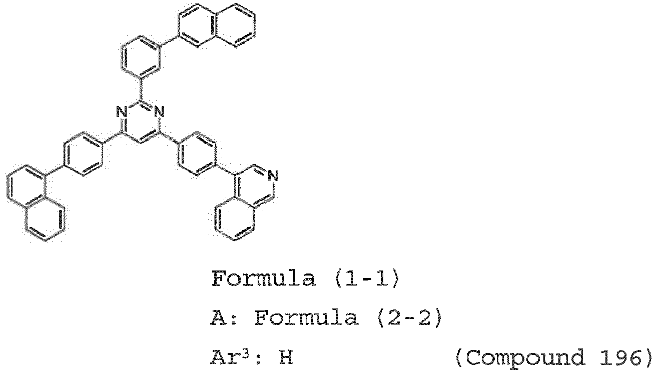

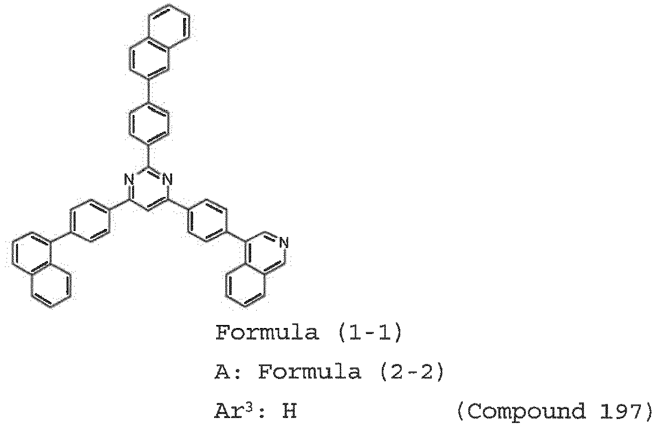

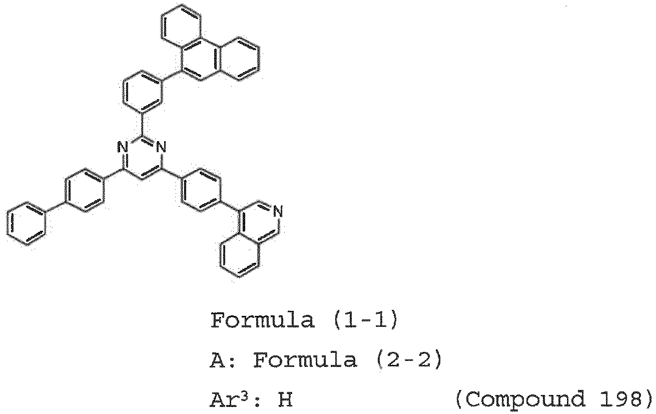

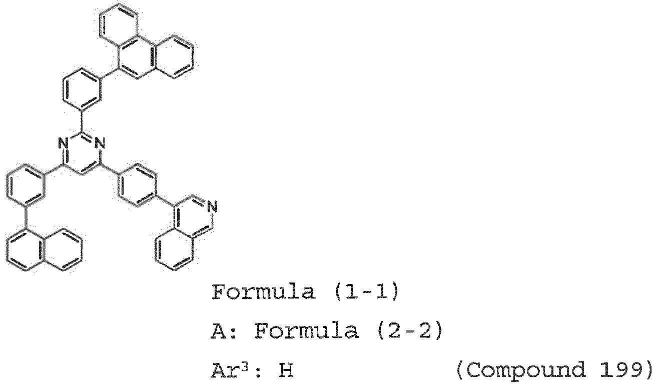

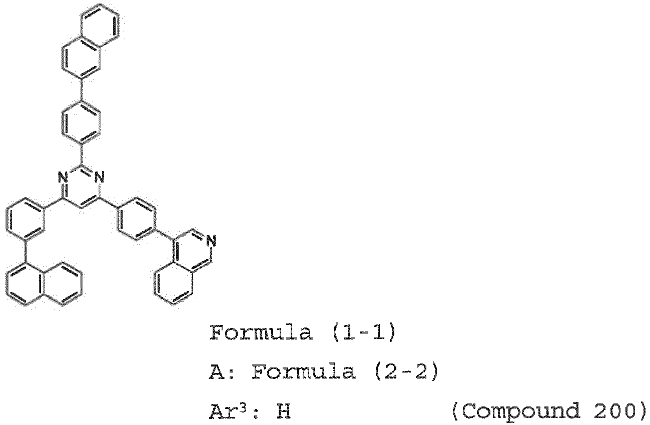

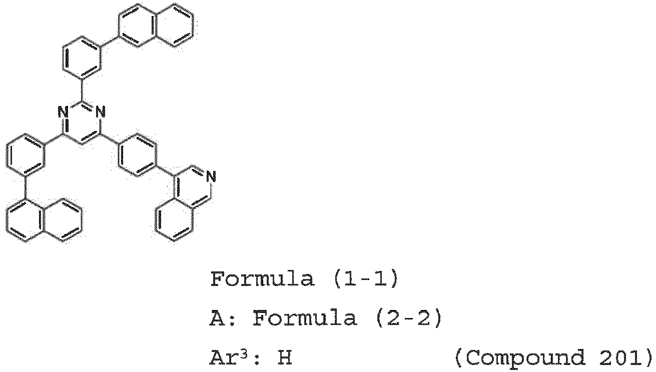

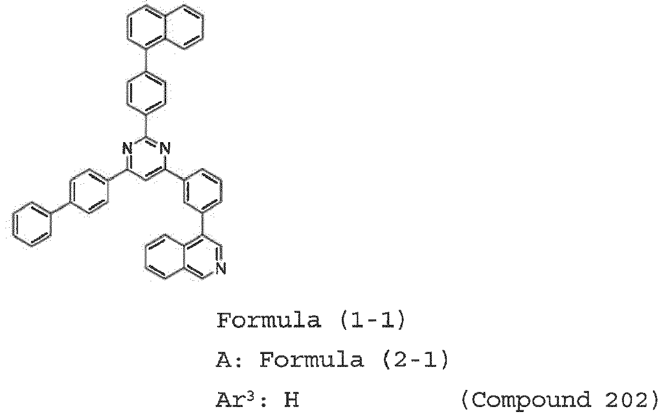

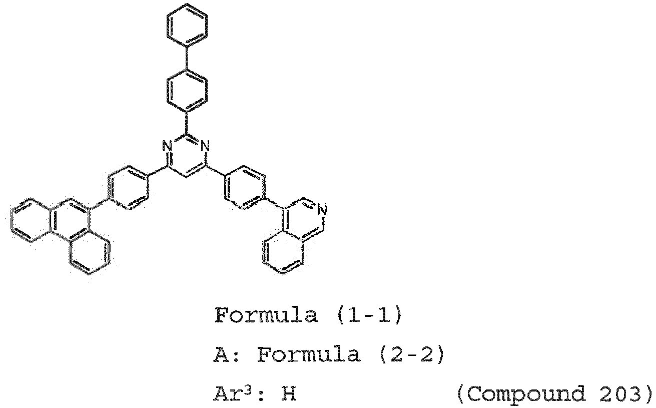

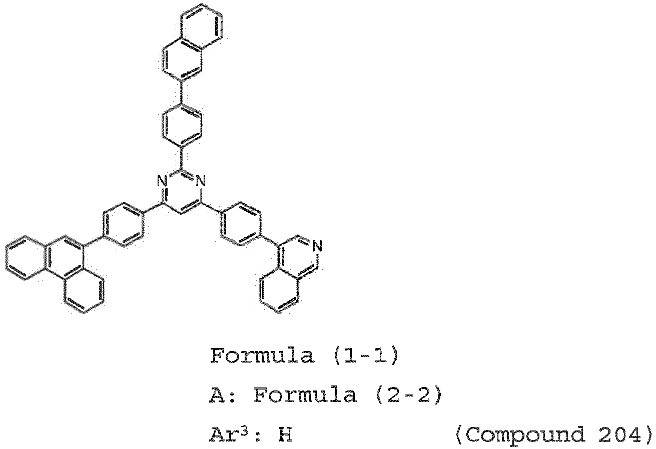

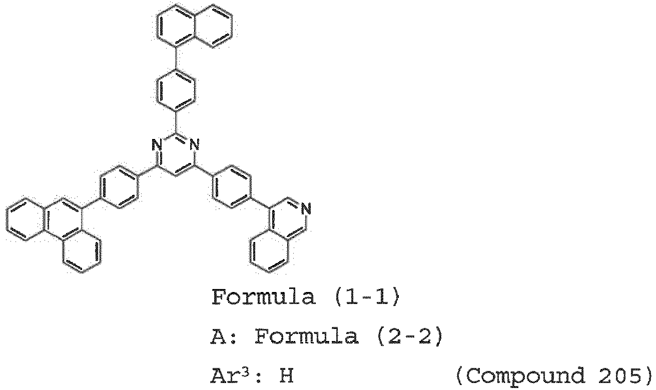

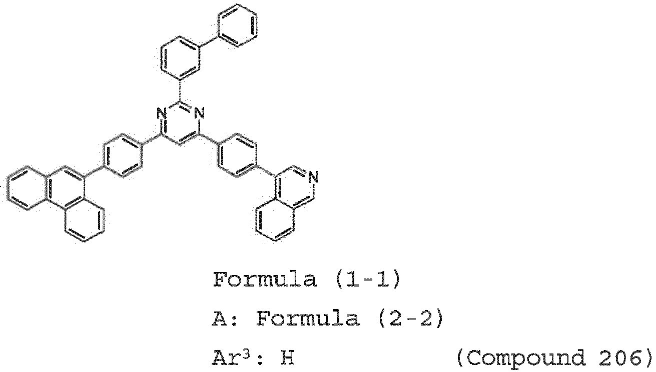

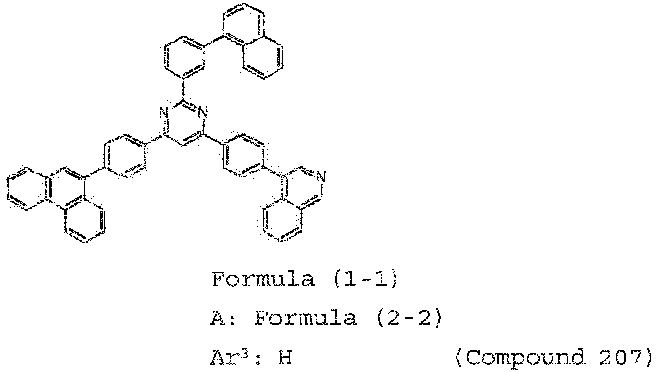

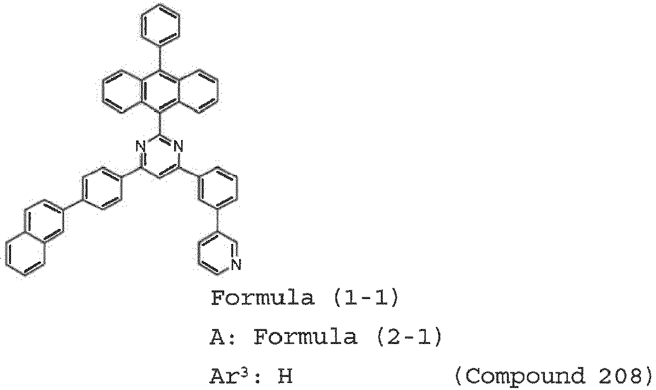

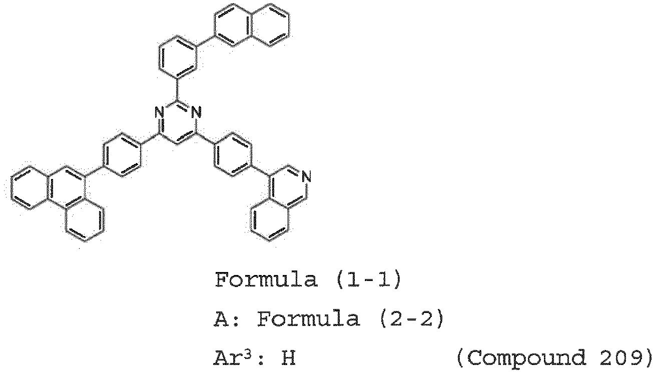

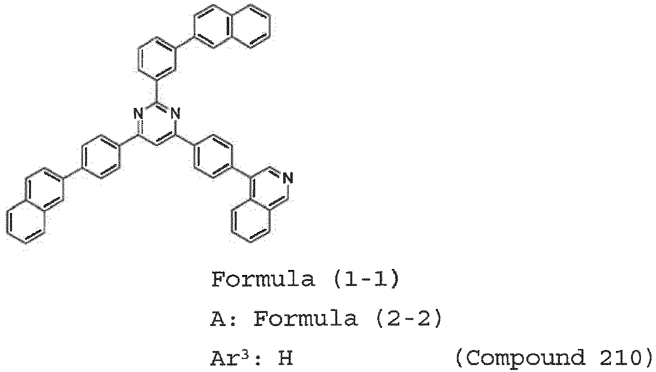

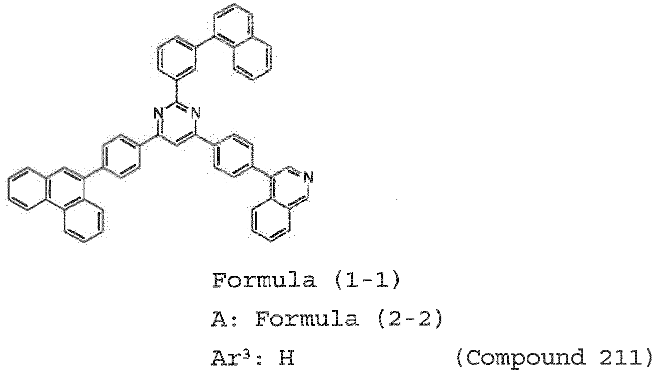

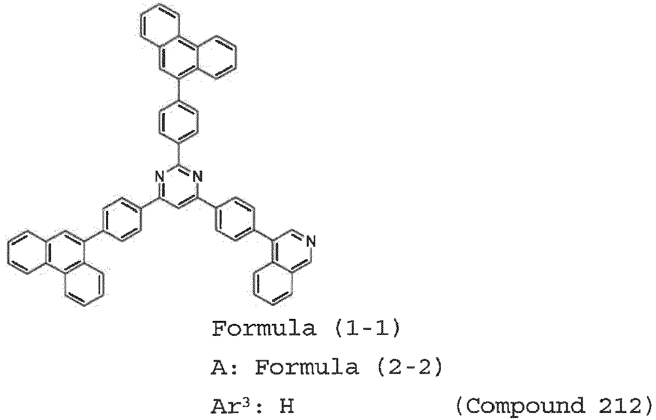

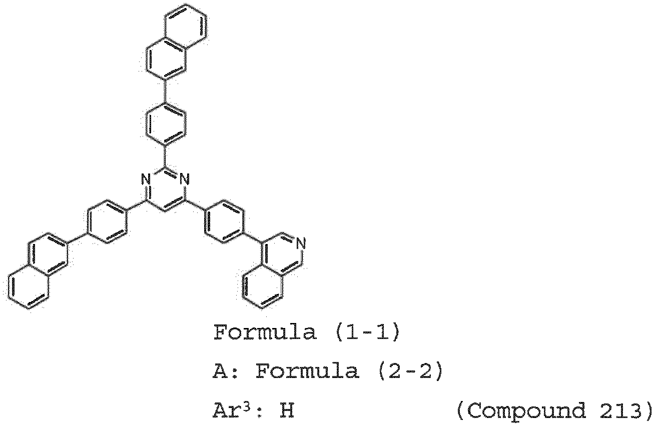

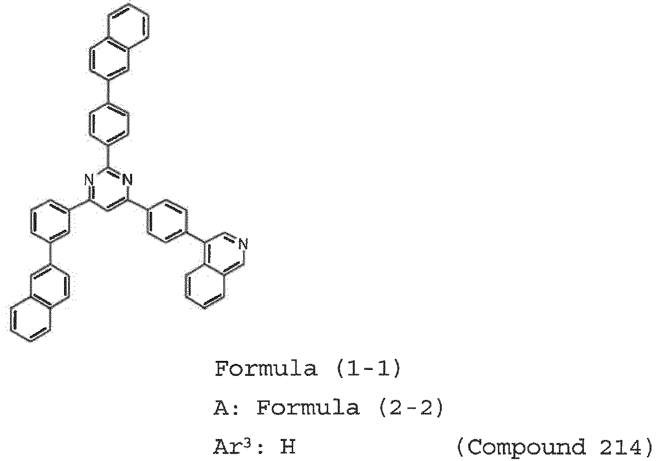

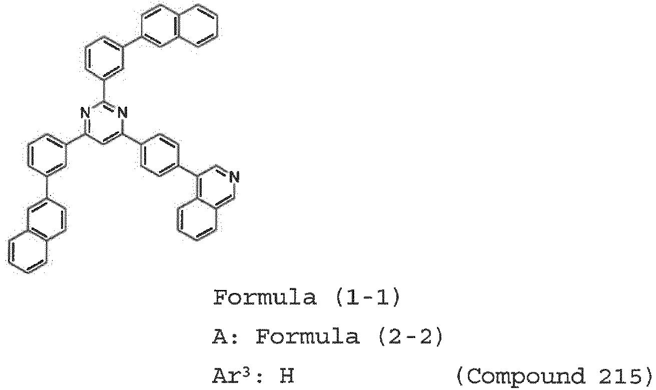

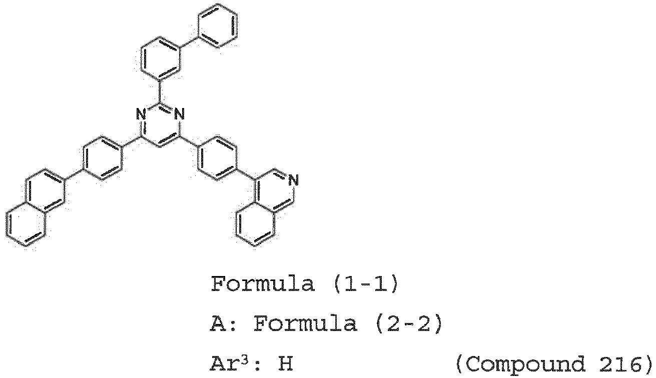

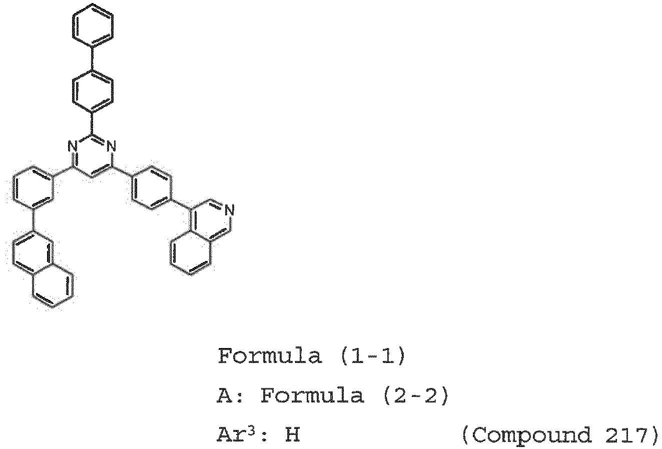

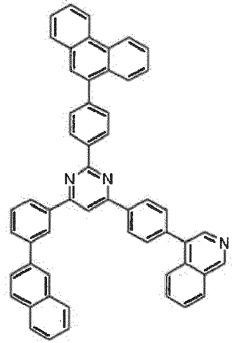

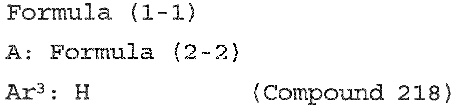

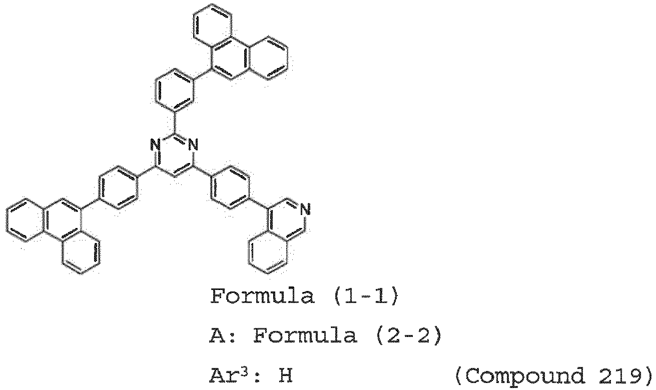

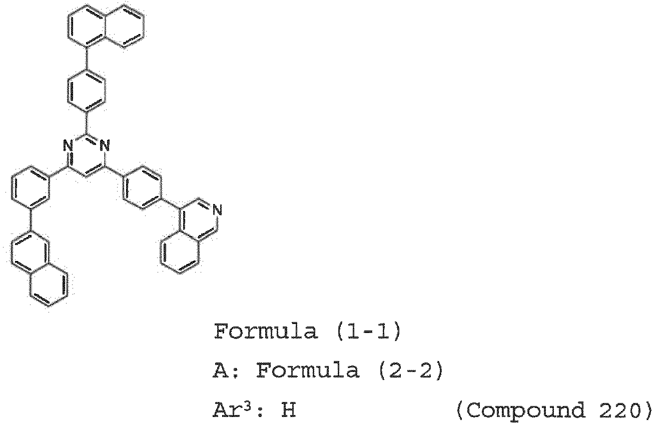

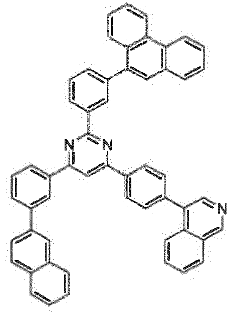

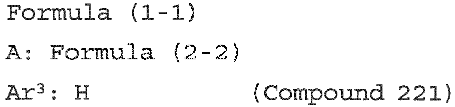

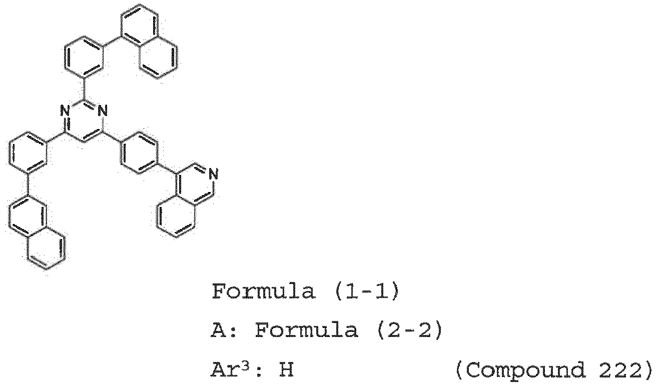

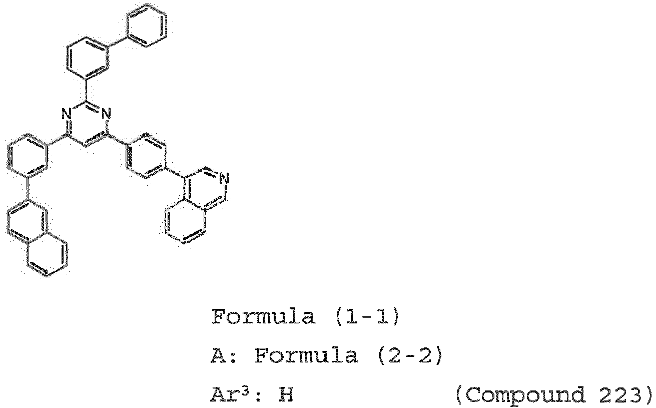

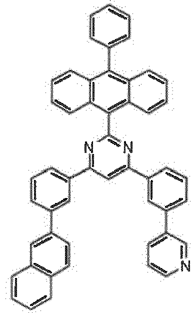

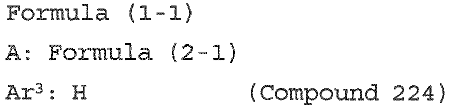

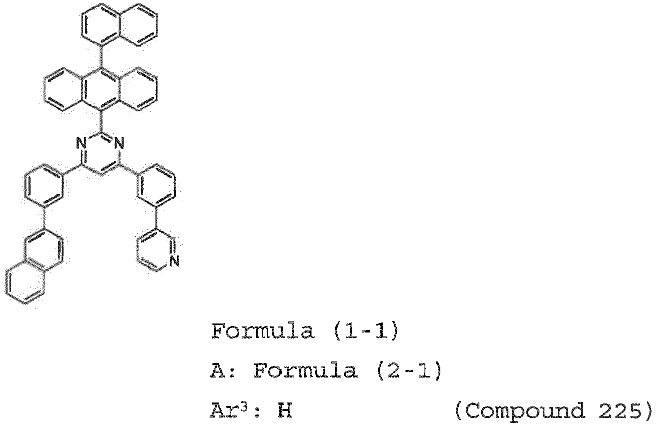

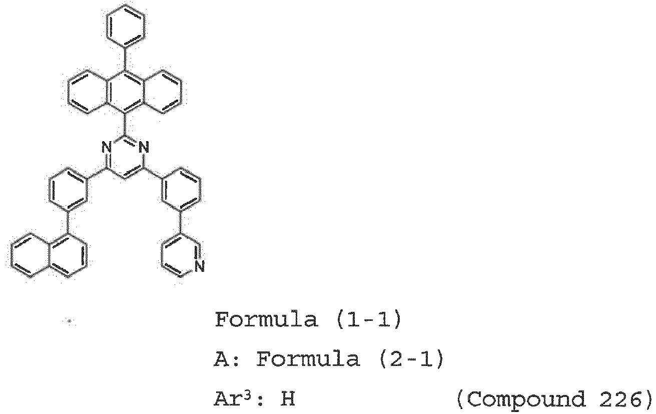

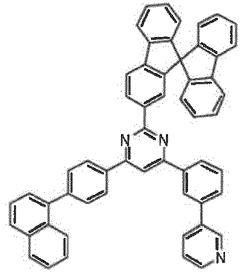

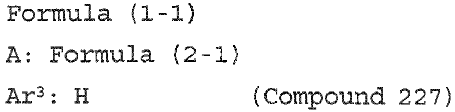

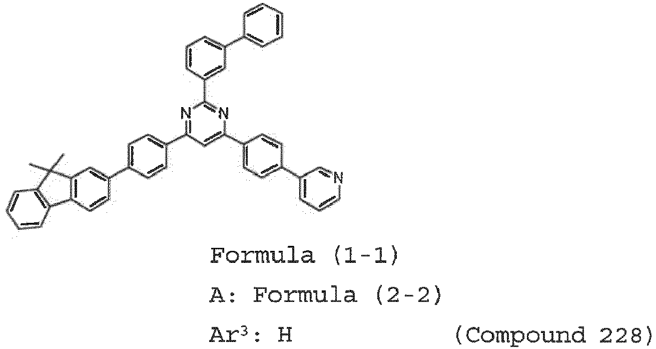

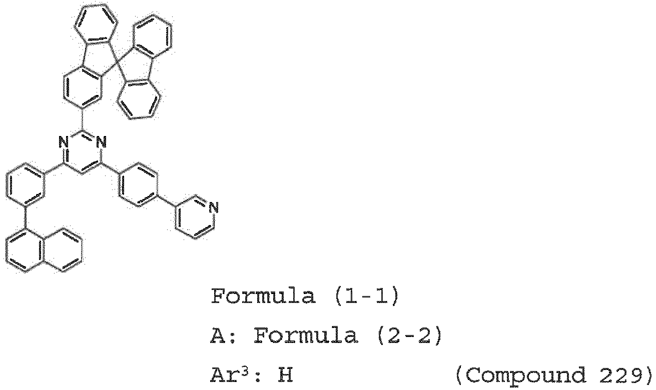

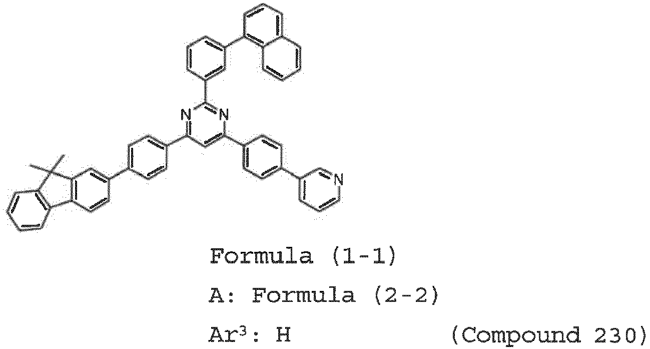

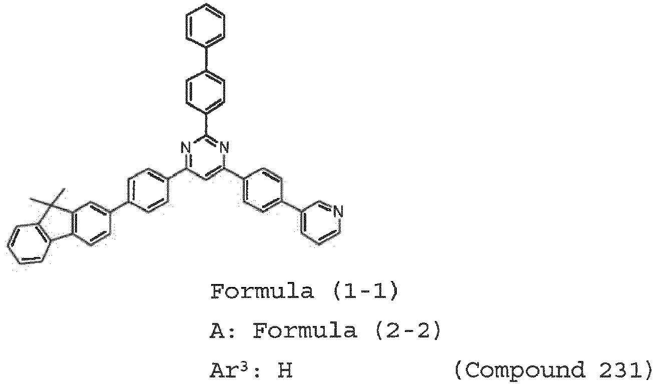

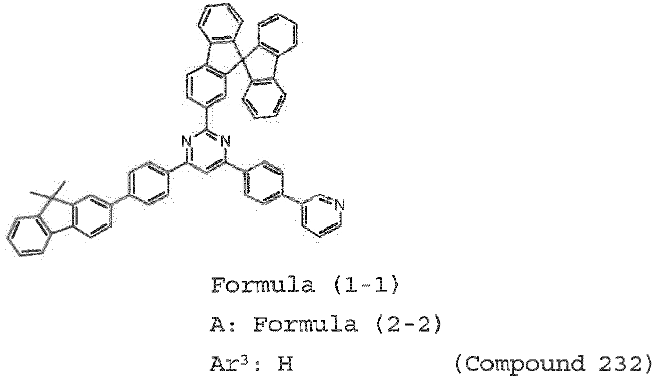

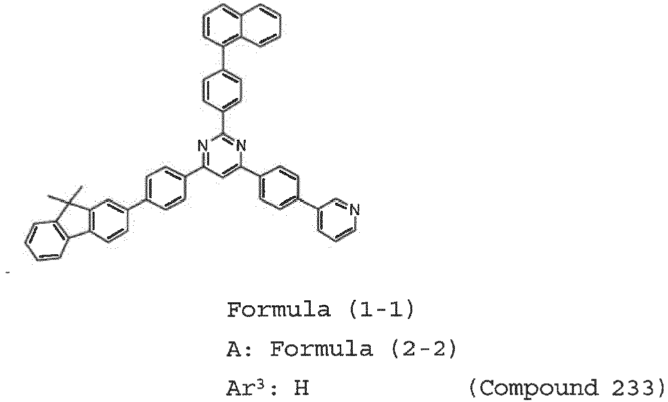

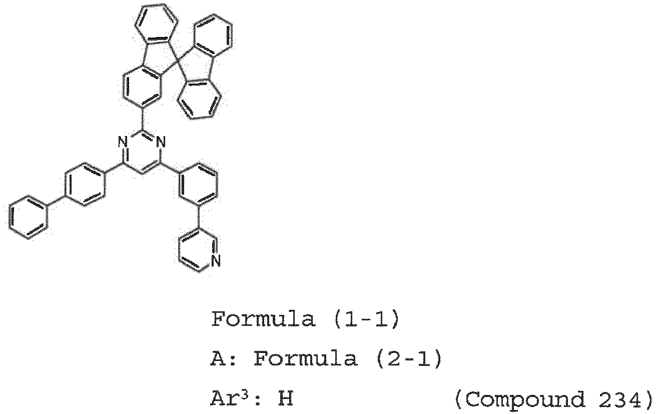

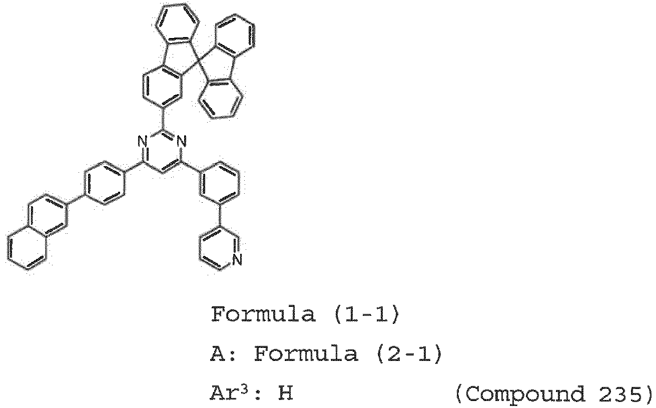

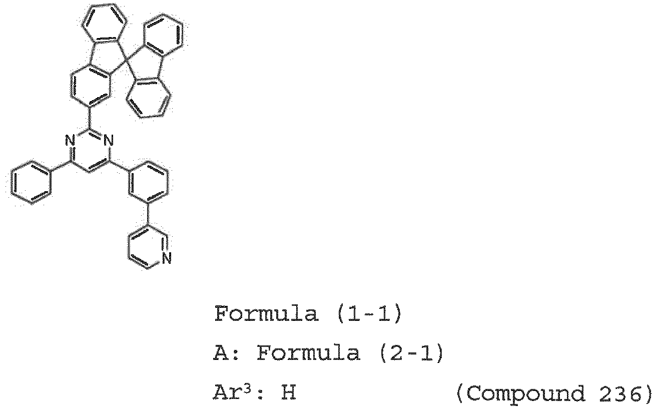

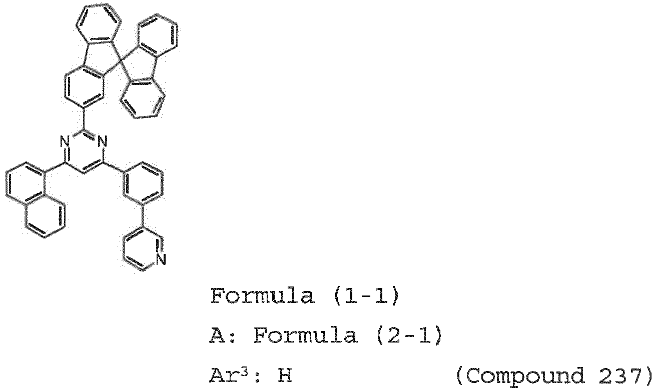

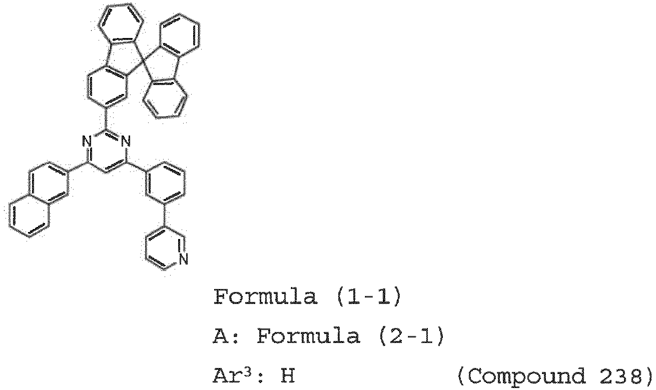

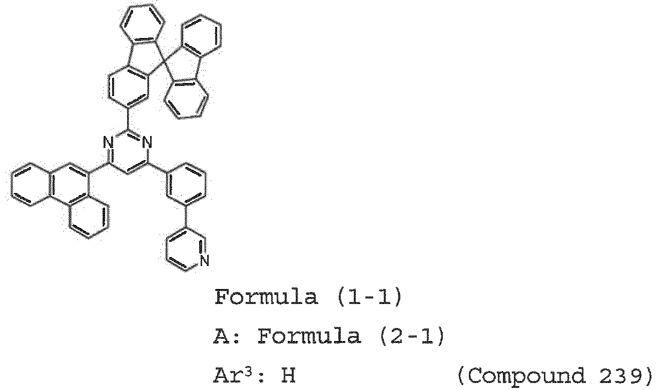

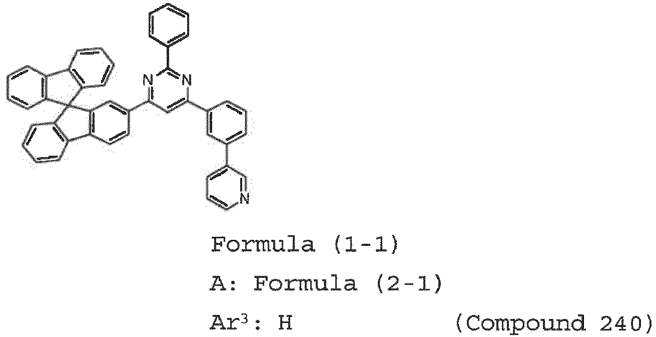

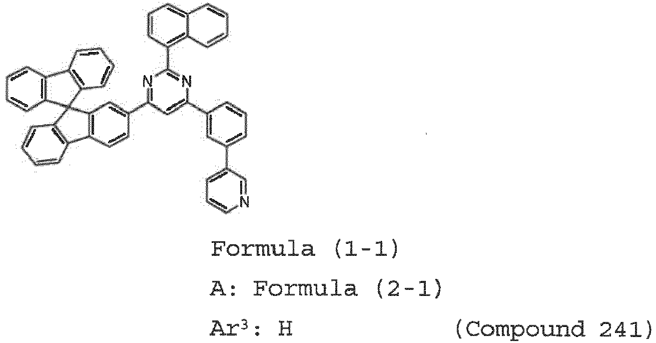

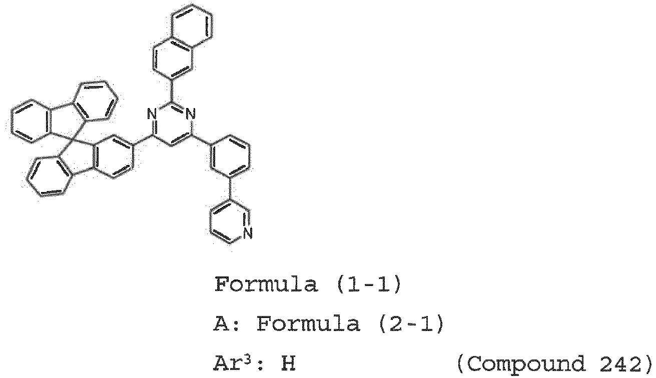

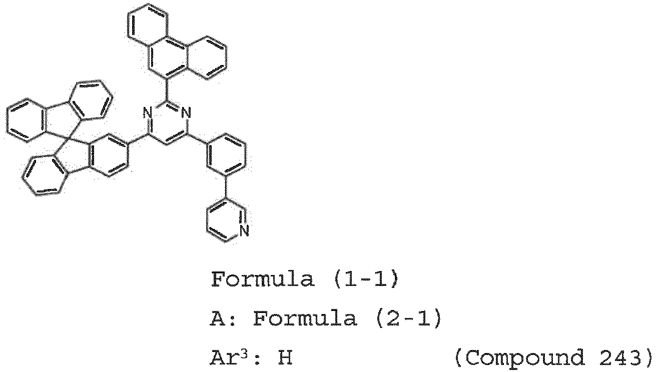

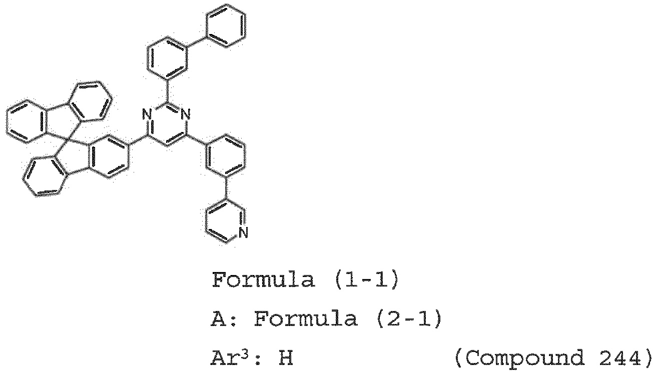

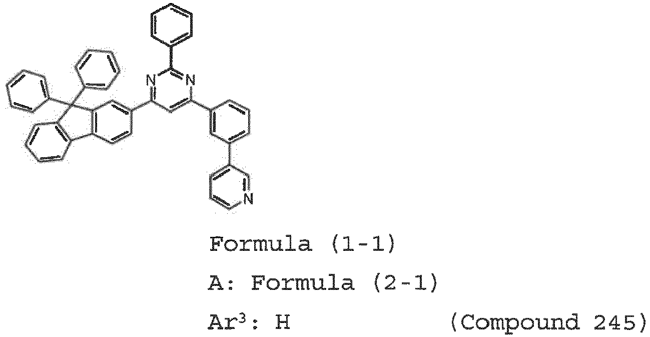

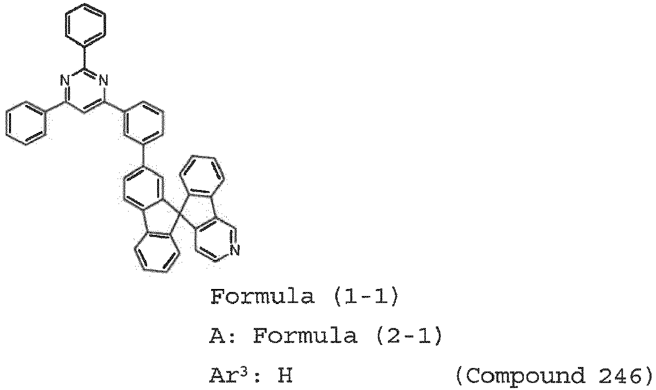

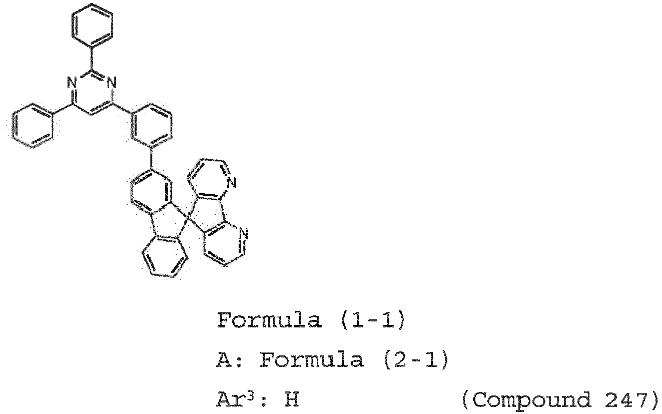

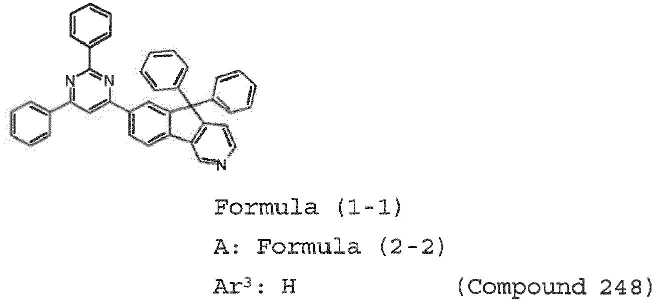

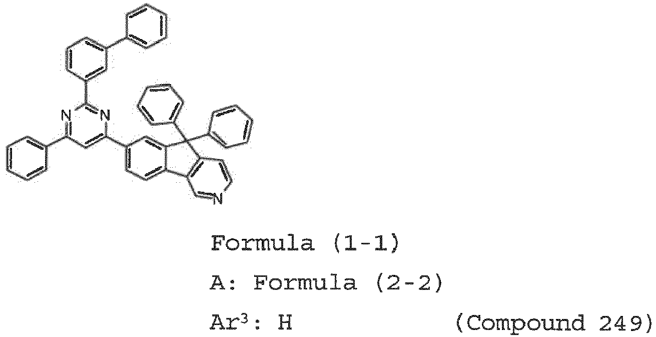

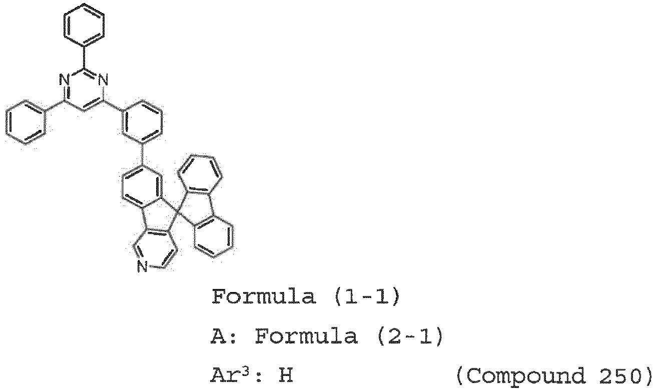

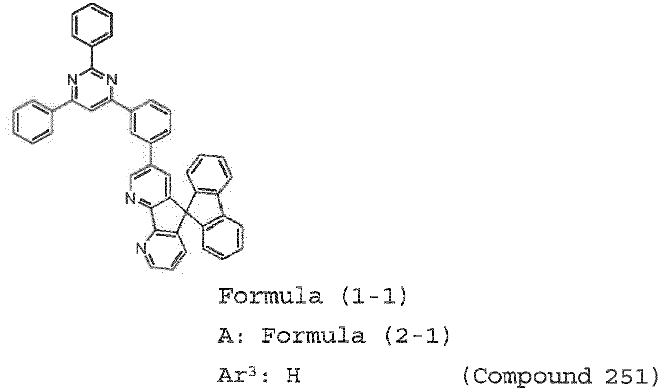

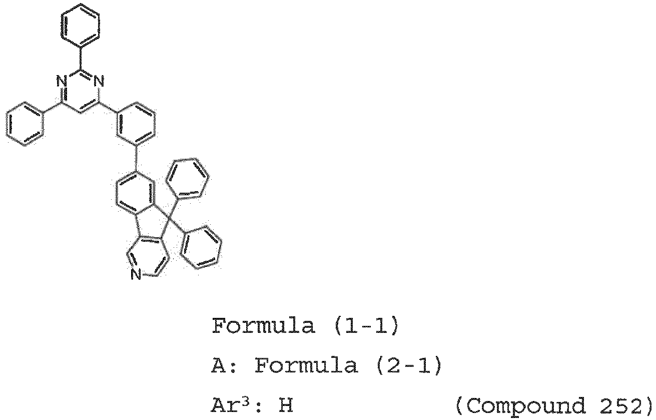

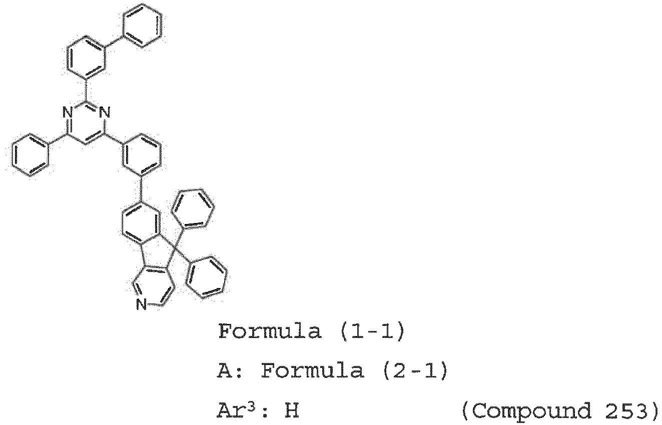

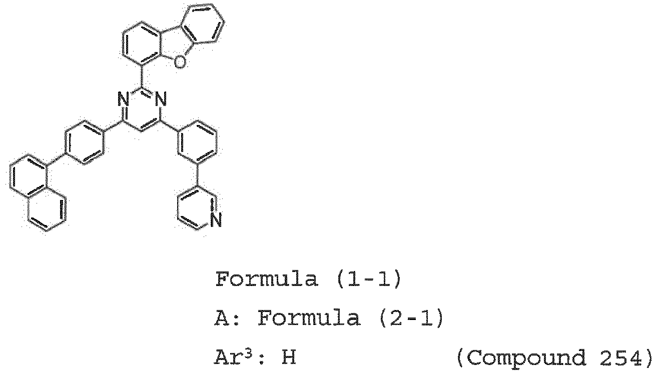

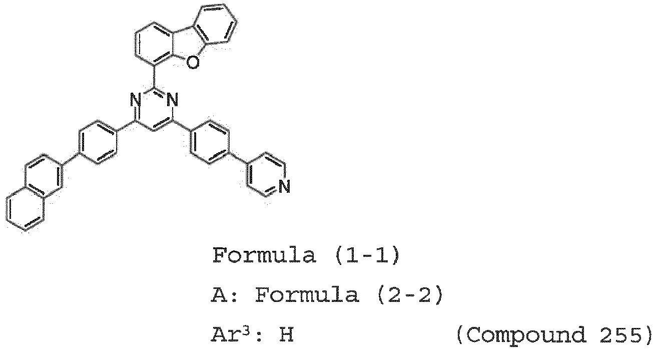

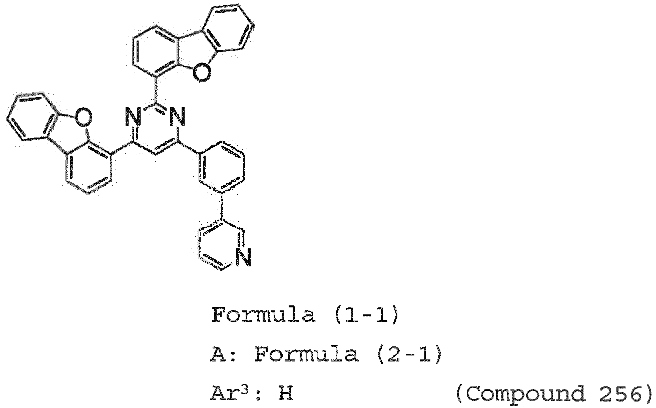

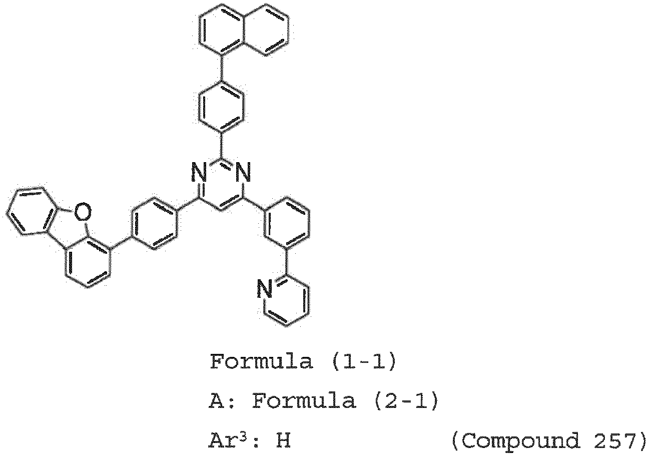

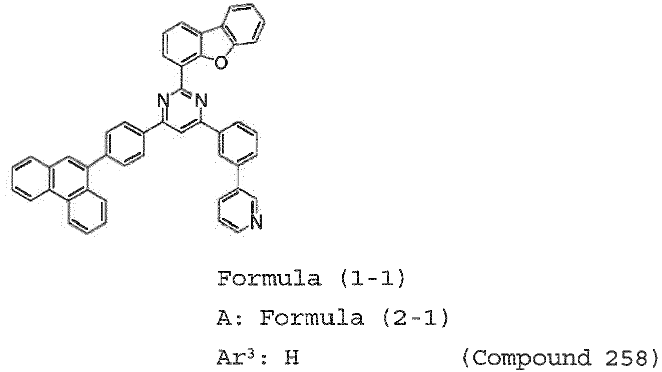

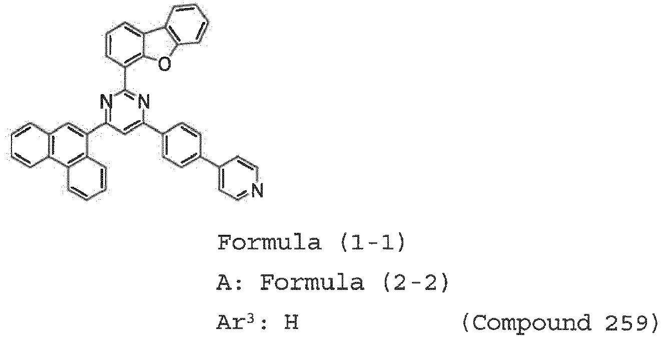

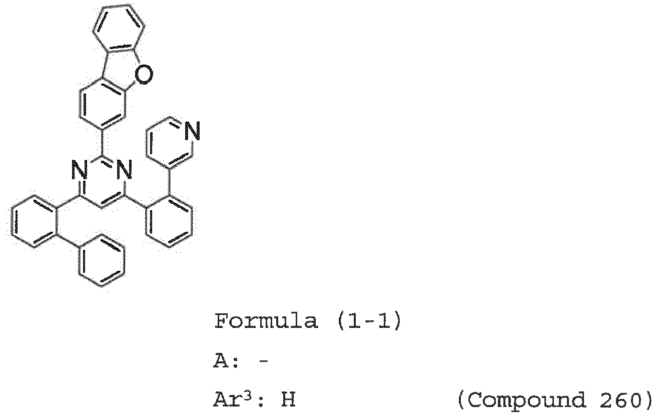

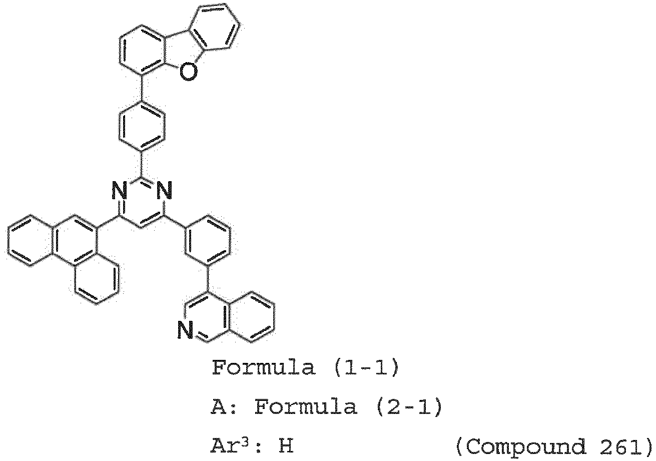

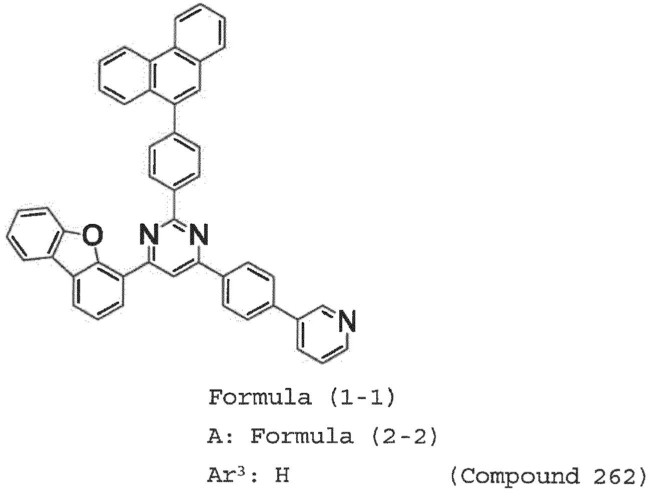

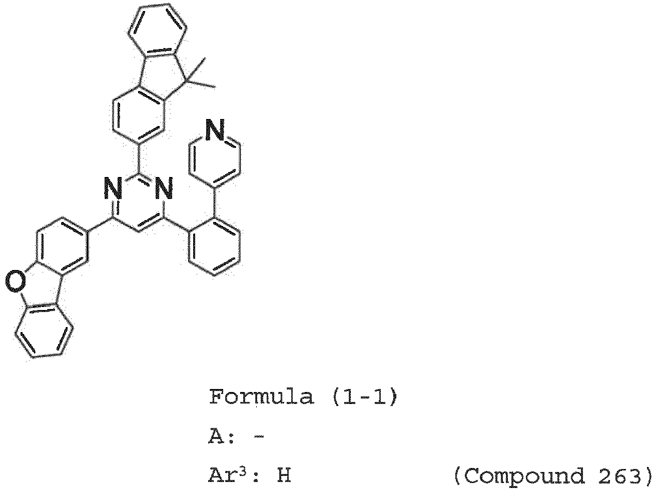

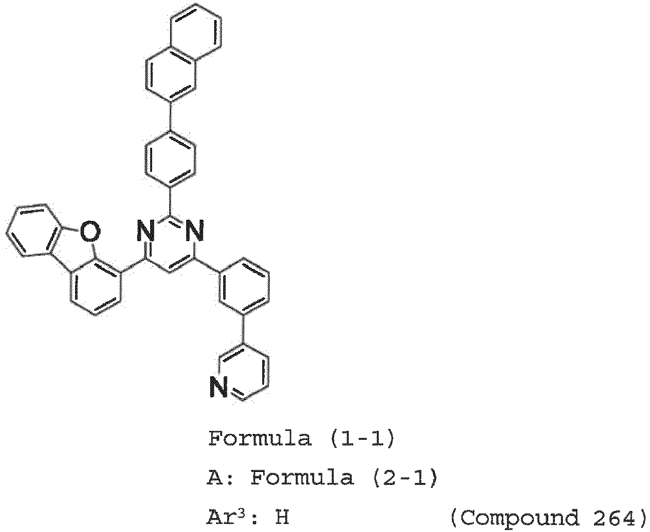

### <Organic EL device>

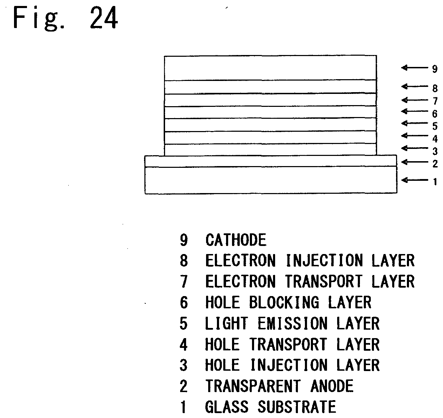

An organic EL device having at least one organic layer provided between a pair of electrodes, the organic layer being formed using the pyrimidine derivative of the present invention described above, (may hereinafter be referred to as the organic EL device of the present invention) has a layered structure, for example, as shown in Fig. 24. That is, in the organic EL device of the present invention, for example, an anode 2, a hole injection layer 3, a hole transport layer 4, a luminous layer 5, a hole blocking layer 6, an electron transport layer 7, an electron injection layer 8, and a cathode 9 are provided in sequence on a substrate.

The organic EL device of the present invention is not limited to such a structure, but for example, may have an electron blocking layer (not shown) provided between the hole transport layer 4 and the luminous layer 5. In this multilayer structure, some of the organic layers may be omitted. For example, there can be a configuration in which the hole injection layer 3 between the anode 2 and the hole transport layer 4, the hole blocking layer 6 between the luminous layer 5 and the electron transport layer 7, and the electron injection layer 8 between the electron transport layer 7 and the cathode 9 are omitted, and the anode 2, the hole transport layer 4, the luminous layer 5, the electron transport layer 7, and the cathode 9 are provided sequentially on the substrate 1.

The anode 2 may be composed of an electrode material publicly known per se and, for example, an electrode material having a great work function, such as ITO or gold, is used.

The hole injection layer 3 may be composed of a material publicly known per se which has hole injection properties, and can be formed, for example, using any of the following materials:
porphyrin compounds typified by copper phthalocyanine;
triphenylamine derivatives of starburst type;
triphenylamine trimers and tetramers, for example, arylamine compounds having a structure in which 3 or more triphenylamine structures are coupled together by a single bond, or a divalent group containing no hetero atom, in the molecule;
acceptor type heterocyclic compounds, for example, hexacyanoazatriphenylene; and
coating type polymeric materials.

The hole injection layer 3 (thin film) can be formed by vapor deposition or any other publicly known method such as a spin coat method or an ink jet method. Various layers to be described below can be similarly formed as films by a publicly known method such as vapor deposition, spin coating, or ink jetting.

The hole transport layer 4 may be composed of a material publicly known per se which has hole transporting properties, and can be formed, for example, using any of the following materials:
benzidine derivatives, for example,
   N,N'-diphenyl-N,N'-di(m-tolyl)benzidine (hereinafter abbreviated as TPD),
   N,N'-diphenyl-N,N'-di(α-naphthyl)benzidine (hereinafter abbreviated as NPD), and
   N,N,N',N'-tetrabiphenylylbenzidine;
1,1-bis[(di-4-tolylamino)phenyl]cyclohexane (hereinafter abbreviated as TAPC); and
various triphenylamine trimers and tetramers.

The above hole transport materials may be used singly for film formation, but may also be mixed with other materials for film formation. It is permissible to form a hole transport layer having a laminated structure in which each layer is formed using any one of the above materials, a laminated structure in which layers are formed using a mixture of the above materials, or a laminated structure in which layers formed by using any one of the above materials and layers formed by using a mixture of the above materials are laminated.

In the present invention, moreover, it is also possible to form a layer concurrently serving as the hole injection layer 3 and the hole transport layer 4. Such a hole injection/transport layer can be formed using a coating type polymeric material such as poly(3,4-ethylenedioxythiophene) (hereinafter abbreviated as PEDOT)/poly(styrenesulfonate) (hereinafter abbreviated as PSS).

In forming the hole injection layer 3 or the hole transport layer 4, the material usually used for this layer is further P-doped with trisbromophenylaminium hexachloroantimonate or radialene derivatives (see Patent Document 5), and can be used for the layer, or a polymeric compound having the structure of a benzidine derivative, such as TPD, in its partial structure can also be used for the layer, in addition to the usual material.

The electron blocking layer (not shown) can be formed using a publicly known compound having an electron blocking action. The publicly known electron blocking compound can be exemplified by the following:
carbazole derivatives, for example,
   4,4',4"-tri(N-carbazolyl)triphenylamine (hereinafter abbreviated as TCTA),
   9,9-bis[4-(carbazol-9-yl)phenyl]fluorene,
   1,3-bis(carbazol-9-yl)benzene (hereinafter abbreviated as mCP), and
   2,2-bis(4-carbazol-9-ylphenyl)adamantane (hereinafter abbreviated as Ad-Cz); and
compounds having a triphenylsilyl group and a triarylamine structure, for example,
   9-[4-(carbazol-9-yl)phenyl]-9-[4-(triphenylsilyl) phenyl]-9H-fluorene.

The above material for the electron blocking layer may be used alone for film formation, but may be mixed with other material for film formation. It is permissible to form an electron blocking layer having a laminated structure in which each layer is formed using any one of the above materials, a laminated structure in which layers are formed using a mixture of the above materials, or a laminated structure in which layers formed by using any one of the above materials and layers formed by using a mixture of the above materials are laminated.

The luminous layer 5 can be formed using a publicly known material, aside from the pyrimidine derivative of the present invention. The publicly known material can be exemplified by the following:
metal complexes of quinolinol derivatives including Alq₃;
various metal complexes;
anthracene derivatives;
bisstyrylbenzene derivatives;
pyrene derivatives;
oxazole derivatives; and
polyparaphenylenevinylene derivatives.

The luminous layer 5 may be composed of a host material and a dopant material. As the host material, thiazole derivatives, benzimidazole derivatives, and polydialkylfluorene derivatives can be used in addition to the pyrimidine derivative of the present invention and the above-mentioned luminescent materials.

Usable as the dopant material are, for example, quinacridone, coumarin, rubrene, perylene and derivatives thereof; benzopyran derivatives; rhodamine derivatives; and aminostyryl derivatives.

The above material for the luminous layer may be used alone for film formation, but may be mixed with other material for film formation. It is permissible to form a luminous layer having a laminated structure in which each layer is formed using any one of the materials, a laminated structure in which layers are formed using a mixture of the materials, or a laminated structure in which layers formed by using any one of the materials and layers formed by using a mixture of the materials are laminated.

Furthermore, a phosphorescent luminous body can be used as the luminescent material. As the phosphorescent luminous body, a phosphorescent luminous body in the form of a metal complex containing iridium, platinum or the like can be used. Concretely,
a green phosphorescent luminous body such as Ir(ppy)₃;
a blue phosphorescent luminous body such as FIrpic or FIr6;
a red phosphorescent luminous body such as Btp₂Ir(acac); and the like
can be used. As a hole injecting/transporting host material of the host materials used in this case,
carbazole derivatives, for example,
4,4'-di(N-carbazolyl)biphenyl (hereinafter abbreviated as CBP),
TCTA, and
mCP
can be used in addition to the pyrimidine derivative of the present invention. Examples of the electron transporting host material are as follows:
p-bis(triphenylsilyl)benzene (hereinafter abbreviated as UGH2); and
2,2',2"-(1,3,5-phenylene)-tris(1-phenyl-1H-benzimidazole) (hereinafter abbreviated as TPBI).
By using any such material, a high performance organic EL device can be prepared.

Doping of the host material with the phosphorescent luminous material is preferably performed by codeposition in a range of 1 to 30% by weight based on the entire luminous layer in order to avoid concentration quenching.

A material which emits delayed fluorescence, such as a CDCB derivative, for example, PIC-TRZ, CC2TA, PXZ-TRZ, 4CzIPN and the like can be used as the luminescent material.

The hole blocking layer 6 can be formed using a publicly known compound having hole blocking properties, aside from the pyrimidine derivative of the present invention. The publicly known compound having the hole blocking properties can be exemplified by the following:
phenanthroline derivatives, for example, bathocuproine (hereinafter abbreviated as BCP);
metal complexes of quinolinol derivatives, for example, BAlq;
various rare earth complexes;
oxazole derivatives;
triazole derivatives; and
triazine derivatives.
The hole blocking material may be used alone for film formation, but may be mixed with other material for film formation. It is permissible to form a hole blocking layer having a laminated structure in which each layer is formed using any one of the above materials, a laminated structure in which layers are formed using a mixture of the above materials, or a laminated structure in which layers formed by using any one of the above materials and layers formed by using a mixture of the above materials are laminated.

The above-mentioned publicly known material having the hole blocking properties can also be used for the formation of the electron transport layer 7 to be described blow. That is, the layer concurrently serving as the hole blocking layer 6 and the electron transport layer 7 can be formed by using the above-mentioned publicly known material having the hole blocking properties.

The electron transport layer 7 is formed using a publicly known compound having electron transporting properties, aside from the pyrimidine derivative of the present invention. The publicly known compound having the electron transporting properties can be exemplified by the following:
metal complexes of quinolinol derivatives including Alq₃ and BAlq;
various metal complexes;
triazole derivatives;
triazine derivatives;
oxadiazole derivatives;
pyridine derivatives;
benzimidazole derivatives;
thiadiazole derivatives;
anthracene derivatives;
carbodiimide derivatives;
quinoxaline derivatives;
pyridoindole derivatives;
phenanthroline derivatives; and
silole derivatives.
The electron transport material may be used alone for film formation, but may be mixed with other material for film formation. It is permissible to form an electron transport layer having a laminated structure in which each layer is formed formed using any one of the above materials, a laminated structure in which layers are formed using a mixture of the above materials, or a laminated structure in which layers formed by using any one of the above materials and layers formed by using a mixture of the above materials are laminated.

The electron injection layer 8 can be formed using a material publicly known per se, aside from the pyrimidine derivative of the present invention. Examples of such a material are as follows:
alkali metal salts such as lithium fluoride and cesium fluoride;
alkaline earth metal salts such as magnesium fluoride;
metal complexes of quinolinol derivatives such as lithium quinolinol; and
metal oxides such as aluminum oxide.
Upon preferred selection of the electron transport layer and the cathode, the electron injection layer 8 can be omitted.

In the electron injection layer 7 or the electron transport layer 8, moreover, the material usually used for this layer is further N-doped with a metal such as cesium, and can be used for the layer, in addition to the usual material.

In connection with the cathode 9, an electrode material with a low work function such as aluminum, or an alloy having a lower work function, such as a magnesium-silver alloy, a magnesium-indium alloy, or an aluminum-magnesium alloy, is used as an electrode material.

### Examples

Embodiments of the present invention will be described more concretely by way of Examples, but the present invention is in no way limited to the following Examples.

### <Example 1: Synthesis of Compound 74> (Reference Example) Synthesis of 4-(biphenyl-4-yl)-2-{3-(naphthalen-1-yl)phenyl}-6-{4-(pyrid in-3-yl)phenyl}pyrimidine:

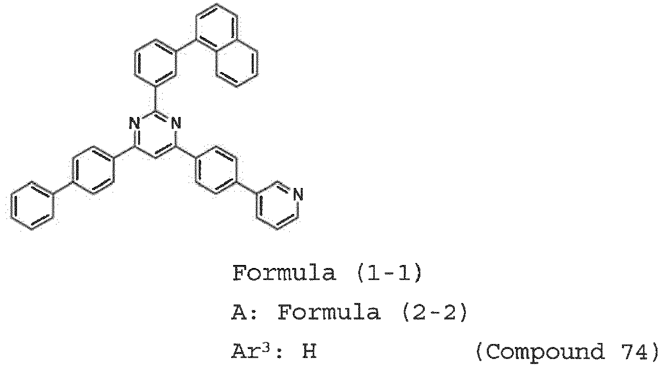

| | |
|---|---|
| A nitrogen-purged reaction vessel was charged with 3-(naphthalen-1-yl)phenylboronic acid | 5.0 g, |
| 2-chloro-4-(biphenyl-4-yl)-6-{4-(pyridin-3-yl)phenyl} pyrimidine | 7.0 g, |
| tetrakistriphenylphosphine | 0.96 g, |
| potassium carbonate | 6.9 g, |
| toluene | 35 ml, |
| 1,4-dioxane | 70 ml and |
| water | 35 ml. |

The mixture was heated, and stirred for 12 hours at 85°C. The mixture was cooled to room temperature, and then an organic layer was collected by liquid separation. Then, the organic layer was concentrated under reduced pressure to obtain a crude product. The crude product was purified by column chromatography (carrier: silica gel, eluent: heptane/dichloromethane/THF), and then subjected to purification by recrystallization using a chlorobenzene/dichloromethane mixed solvent to obtain 3.1 g (yield 32%) of 4-(biphenyl-4-yl)-2-{3-(naphthalen-1-yl)phenyl}-6-{4-(pyrid in-3-yl)phenyl}pyrimidine (Compound 74) as a white powder.

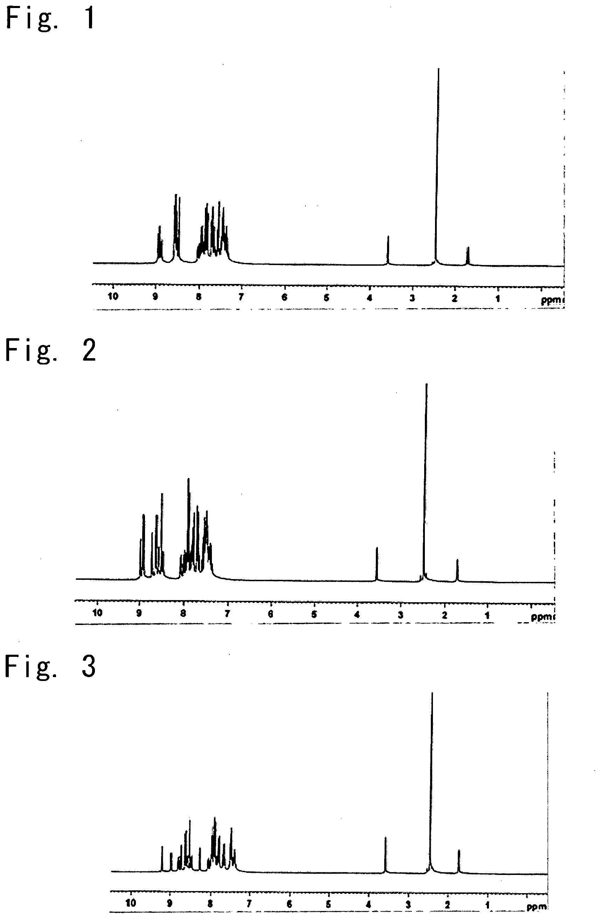

In connection with the resulting white powder, its structure was identified using NMR. The results of its ¹H-NMR measurement are shown in Fig. 1. In ¹H-NMR (THF-d₈), the following signals of 29 hydrogens were detected.

| | | |
|---|---|---|
| δ (ppm) | = | 8.97-8.84 (3H) |
| | | 8.60-8.45 (6H) |
| | | 8.08-7.32 (20H) |

### <Example 2: Synthesis of Compound 84> (Reference Example) 4-(biphenyl-3-yl)-2-{4-(naphthalen-1-yl)phenyl}-6-{4-(pyrid in-3-yl)phenyl}pyrimidine:

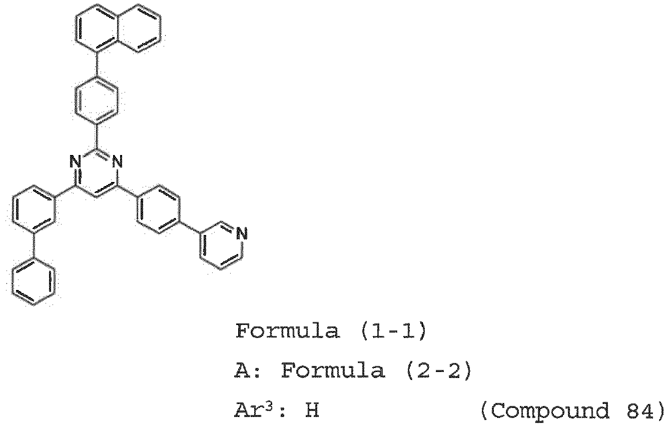

In Example 1,
4-(naphthalen-1-yl)phenylboronic acid was used instead of
3-(naphthalen-1-yl)phenylboronic acid, and
2-chloro-4-(biphenyl-3-yl)-6-{4-(pyridin-3-yl)phenyl} pyrimidine was used instead of
2-chloro-4-(biphenyl-4-yl)-6-{4-(pyridin-3-yl)phenyl} pyrimidine,
and the reaction was performed under the same conditions. As a result, 3.8 g (yield 39%) of 4-(biphenyl-3-yl)-2-{4-(naphthalen-1-yl)phenyl}-6-{4-(pyrid in-3-yl)phenyl}pyrimidine (Compound 84) was obtained as a white powder.

In connection with the resulting white powder, its structure was identified using NMR. The results of its ¹H-NMR measurement are shown in Fig. 2. In ¹H-NMR (THF-d₈), the following signals of 29 hydrogens were detected.

| | | |
|---|---|---|
| δ (ppm) | = | 8.99 (1H) |
| | | 8.93 (2H) |
| | | 8.72 (1H) |
| | | 8.65 (2H) |
| | | 8.59 (1H) |
| | | 8.52 (1H) |
| | | 8.49 (1H) |
| | | 8.09 (1H) |
| | | 8.04-7.34 (19H) |

### <Example 3: Synthesis of Compound 89> (Reference Example) 4-(biphenyl-3-yl)-2-{3-(naphthalen-2-yl)phenyl}-6-{4-(pyrid in-3-yl)phenyl}pyrimidine:

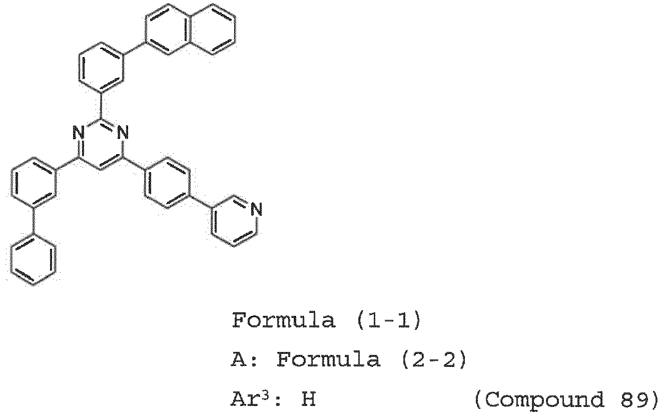

In Example 1,
3-(naphthalen-2-yl)phenylboronic acid was used instead of
3-(naphthalen-1-yl)phenylboronic acid, and
2-chloro-4-(biphenyl-3-yl)-6-{4-(pyridin-3-yl)phenyl} pyrimidine was used instead of
2-chloro-4-(biphenyl-4-yl)-6-{4-(pyridin-3-yl)phenyl} pyrimidine,
and the reaction was performed under the same conditions. As a result, 5.8 g (yield 59%) of 4-(biphenyl-3-yl)-2-{3-(naphthalen-2-yl)phenyl}-6-{4-(pyrid in-3-yl)phenyl}pyrimidine (Compound 89) was obtained as a yellow powder.

In connection with the resulting yellow powder, its structure was identified using NMR. The results of its ¹H-NMR measurement are shown in Fig. 3. In ¹H-NMR (THF-d₈), the following signals of 29 hydrogens were detected.

| | | |
|---|---|---|
| δ (ppm) | = | 9.21 (1H) |
| | | 8.98 (1H) |
| | | 8.80 (1H) |
| | | 8.74 (1H) |
| | | 8.64 (2H) |
| | | 8.59 (1H) |
| | | 8.53 (1H) |
| | | 8.46 (1H) |
| | | 8.29 (1H) |
| | | 8.08 (1H) |
| | | 8.00-7.76 (10H) |
| | | 7.72-7.62 (2H) |
| | | 7.55-6.34 (6H) |

### <Example 4: Synthesis of Compound 130> 2-{3-(naphthalen-1-yl)phenyl}-4-{4-(phenanthren-9-yl)phenyl }-6-{4-(pyridin-3-yl)phenyl}pyrimidine:

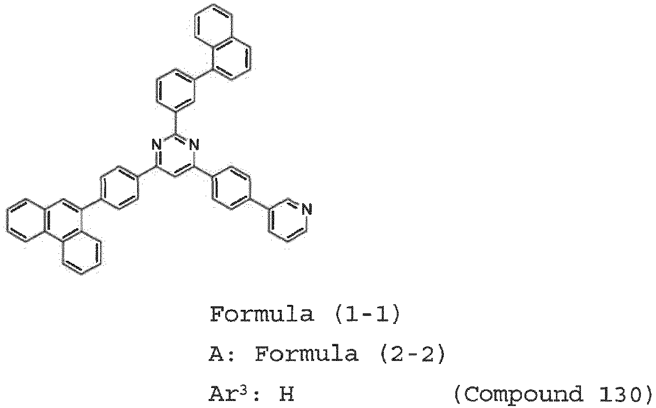

In Example 1,
2-chloro-4-{4-(phenanthren-9-yl)phenyl}-6-{4-(pyridin -3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 9.3 g (yield 35%) of 2-{3-(naphthalen-1-yl)phenyl}-4-{4-(phenanthren-9-yl)phenyl }-6-{4-(pyridin-3-yl)phenyl}pyrimidine (Compound 130) was obtained as a yellow powder.

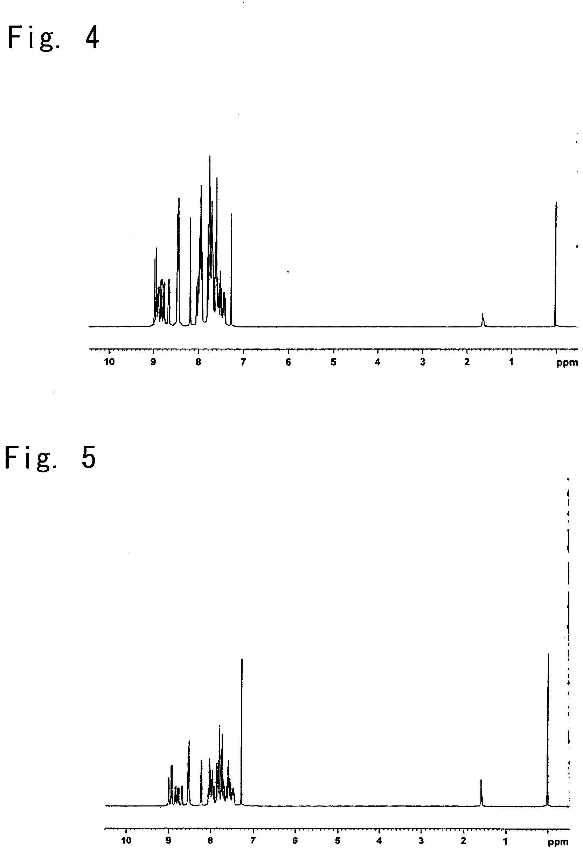

In connection with the resulting yellow powder, its structure was identified using NMR. The results of its ¹H-NMR measurement are shown in Fig. 4. In ¹H-NMR (CDCl₃), the following signals of 33 hydrogens were detected.

| | | |
|---|---|---|
| δ (ppm) | = | 8.98-8.84 (3H) |
| | | 8.84 (1H) |
| | | 8.78 (1H) |
| | | 8.68 (1H) |
| | | 8.47-8.44 (4H) |
| | | 8.19 (1H) |
| | | 8.06-7.93 (6H) |
| | | 7.81-7.41 (16H) |

### <Example 5: Synthesis of Compound 131> 2-{4-(naphthalen-1-yl)phenyl}-4-{4-(phenanthren-9-yl)phenyl }-6-{4-(pyridin-3-yl)phenyl}pyrimidine:

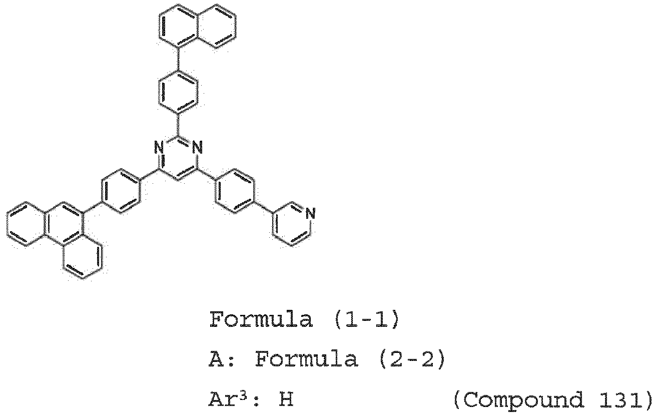

In Example 1,
4-(naphthalen-1-yl)phenylboronic acid was used instead of
3-(naphthalen-1-yl)phenylboronic acid, and
2-chloro-4-{4-(phenanthren-9-yl)phenyl}-6-{4-(pyridin -3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 22.6 g (yield 85%) of 2-{4-(naphthalen-1-yl)phenyl}-4-{4-(phenanthren-9-yl)phenyl }-6-{4-(pyridin-3-yl)phenyl}pyrimidine (Compound 131) was obtained as a yellow powder.

In connection with the resulting yellow powder, its structure was identified using NMR. The results of its ¹H-NMR measurement are shown in Fig. 5. In ¹H-NMR (CDCl₃), the following signals of 33 hydrogens were detected.

| | | |
|---|---|---|
| δ (ppm) | = | 9.00 (1H) |
| | | 8.94 (2H) |
| | | 8.85 (1H) |
| | | 8.78 (1H) |
| | | 8.69 (1H) |
| | | 8.55-8.51 (4H) |
| | | 8.23 (1H) |
| | | 8.23-7.44 (22H) |

### <Example 6: Synthesis of Compound 92> 2-{4-(naphthalen-1-yl)phenyl}-4-{3-(naphthalen-1-yl)phenyl} -6-{4-(pyridin-3-yl)phenyl}pyrimidine:

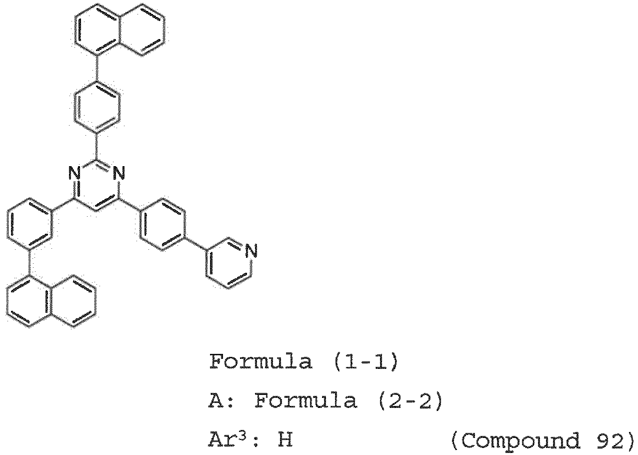

In Example 1,
4-(naphthalen-1-yl)phenylboronic acid was used instead of
3-(naphthalen-1-yl)phenylboronic acid, and
2-chloro-4-{3-(naphthalen-1-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 6 g (yield 74%) of 2-{4-(naphthalen-1-yl)phenyl}-4-{3-(naphthalen-1-yl)phenyl} -6-{4-(pyridin-3-yl)phenyl}pyrimidine (Compound 92) was obtained as a yellow powder.

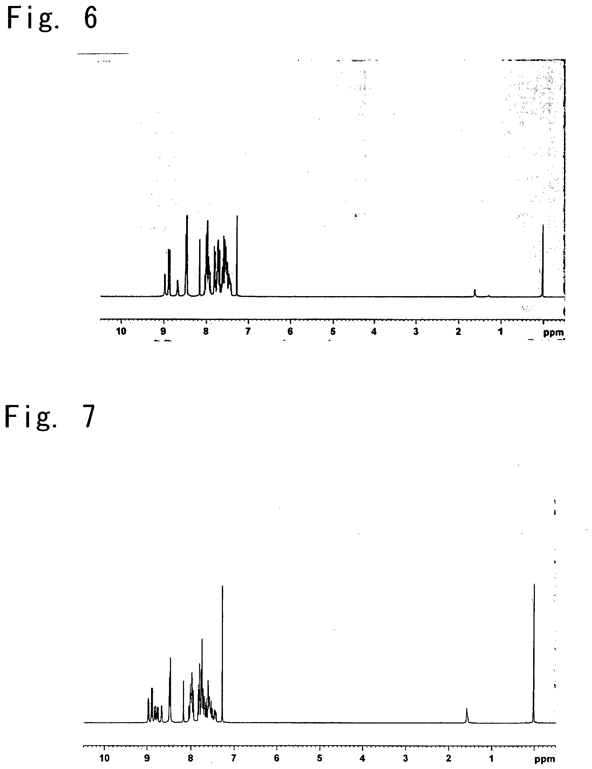

In connection with the resulting yellow powder, its structure was identified using NMR. The results of its ¹H-NMR measurement are shown in Fig. 6. In ¹H-NMR (CDCl₃), the following signals of 31 hydrogens were detected.

| | | |
|---|---|---|
| δ (ppm) | = | 8.98 (1H) |
| | | 8.87 (2H) |
| | | 8.67 (1H) |
| | | 8.48-8.46 (4H) |
| | | 8.16 (1H) |
| | | 8.04-7.82 (7H) |
| | | 7.80 (2H) |
| | | 7.76-7.42 (13H) |

### <Example 7: Synthesis of Compound 136> 2-{4-(phenanthren-9-yl)phenyl}-4-{3-(naphthalen-1-yl)phenyl }-6-{4-(pyridin-3-yl)phenyl}pyrimidine:

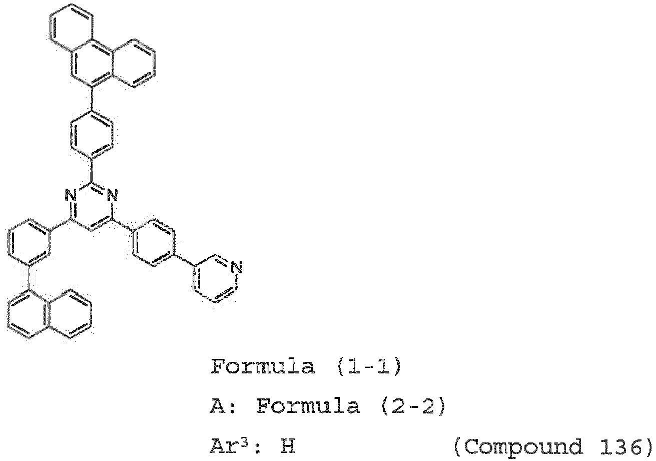

In Example 1,
4-(phenanthren-9-yl)phenylboronic acid was used instead of
3-(naphthalen-1-yl)phenylboronic acid, and
2-chloro-4-{3-(naphthalen-1-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 2 g (yield 26%) of 2-{4-(phenanthren-9-yl)phenyl}-4-{3-(naphthalen-1-yl)phenyl }-6-{4-(pyridin-3-yl)phenyl}pyrimidine (Compound 136) was obtained as a yellow powder.

In connection with the resulting yellow powder, its structure was identified using NMR. The results of its ¹H-NMR measurement are shown in Fig. 7. In ¹H-NMR (CDCl₃), the following signals of 33 hydrogens were detected.

| | | |
|---|---|---|
| δ (ppm) | = | 8.98 (1H) |
| | | 8.90 (2H) |
| | | 8.83 (1H) |
| | | 8.78 (1H) |
| | | 8.68 (1H) |
| | | 8.50-8.45 (4H) |
| | | 8.17 (1H) |
| | | 8.04-7.94 (6H) |
| | | 7.83-7.41 (16H) |

### <Example 8: Synthesis of Compound 125> 2-{4-(naphthalen-1-yl)phenyl}-4-{4-(naphthalen-1-yl)phenyl} -6-{4-(pyridin-3-yl)phenyl}pyrimidine:

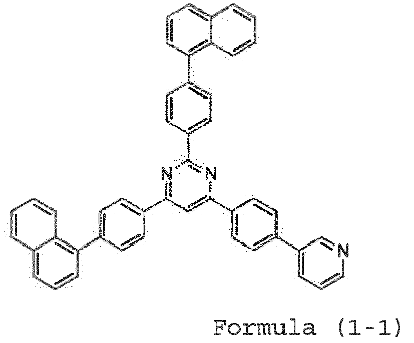

A: Formula (2-2)
Ar³: H (Compound 125)

In Example 1,
4-(naphthalen-1-yl)phenylboronic acid was used instead of
3-(naphthalen-1-yl)phenylboronic acid, and
2-chloro-4-{4-(naphthalen-1-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 21.6 g (yield 80%) of 2-{4-(naphthalen-1-yl)phenyl}-4-{4-(naphthalen-1-yl)phenyl} -6-{4-(pyridin-3-yl)phenyl}pyrimidine (Compound 125) was obtained as a yellow powder.

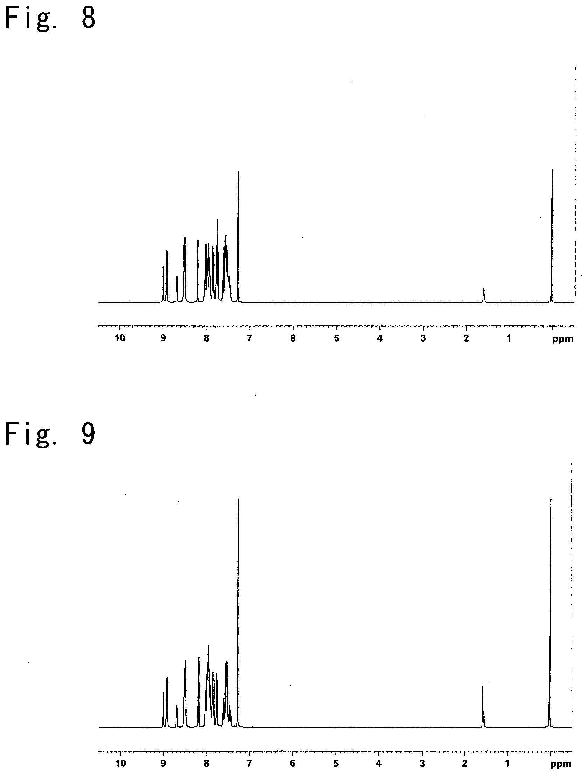

In connection with the resulting yellow powder, its structure was identified using NMR. The results of its ¹H-NMR measurement are shown in Fig. 8. In ¹H-NMR (THF-d₈), the following signals of 31 hydrogens were detected.

| | | |
|---|---|---|
| δ (ppm) | = | 9.00 (1H) |
| | | 8.95 (2H) |
| | | 8.68 (1H) |
| | | 8.54-8.48 (4H) |
| | | 8.22 (1H) |
| | | 8.21-7.91 (7H) |
| | | 7.82 (2H) |
| | | 7.79-7.72 (4H) |
| | | 7.64-7.42 (9H) |

### <Example 9: Synthesis of Compound 138> 2-{4-(naphthalen-2-yl)phenyl}-4-{4-(naphthalen-1-yl)phenyl} -6-{4-(pyridin-3-yl)phenyl}pyrimidine:

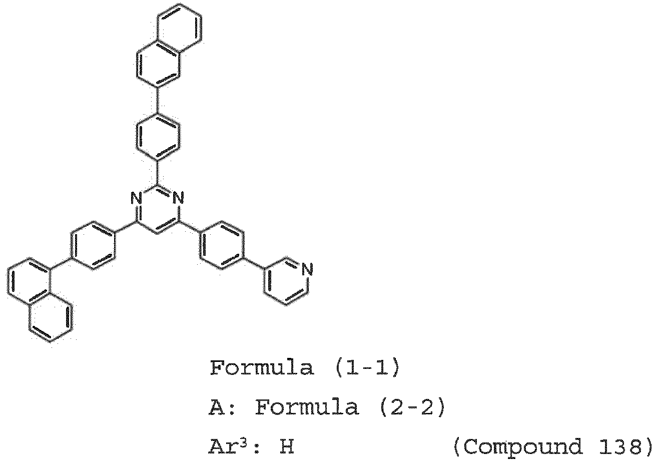

In Example 1,
4-(naphthalen-2-yl)phenylboronic acid was used instead of
3-(naphthalen-1-yl)phenylboronic acid, and
2-chloro-4-{4-(naphthalen-1-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 3.5 g (yield 43%) of 2-{4-(naphthalen-2-yl)phenyl}-4-{4-(naphthalen-1-yl)phenyl} -6-{4-(pyridin-3-yl)phenyl}pyrimidine (Compound 138) was obtained as a yellow powder.

In connection with the resulting yellow powder, its structure was identified using NMR. The results of its ¹H-NMR measurement are shown in Fig. 9. In ¹H-NMR (CDCl₃), the following signals of 31 hydrogens were detected.

| | | |
|---|---|---|
| δ (ppm) | = | 9.00 (1H) |
| | | 8.92 (2H) |
| | | 8.68 (1H) |
| | | 8.53-8.48 (4H) |
| | | 8.20 (2H) |
| | | 8.03-7.81 (12H) |
| | | 7.77 (2H) |
| | | 7.63-7.42 (7H) |

### <Example 10: Synthesis of Compound 78> 2-{4-(phenanthren-9-yl)phenyl}-4-{4-(naphthalen-1-yl)phenyl }-6-{4-(pyridin-3-yl)phenyl}pyrimidine:

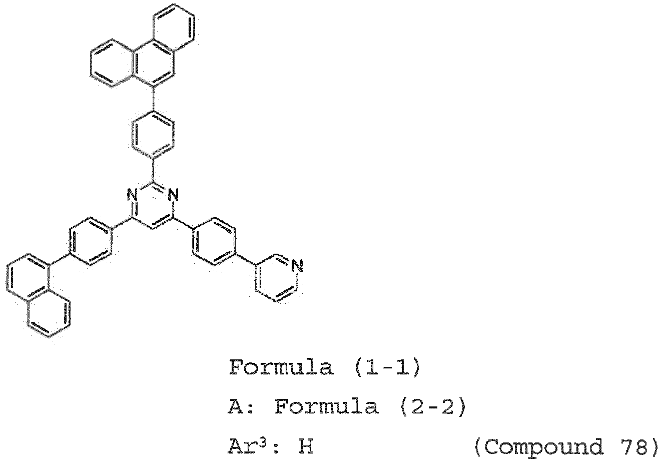

In Example 1,
4-(phenanthren-9-yl)phenylboronic acid was used instead of
3-(naphthalen-1-yl)phenylboronic acid, and
2-chloro-4-{4-(naphthalen-1-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 16.2 g (yield 56%) of 2-{4-(phenanthren-9-yl)phenyl}-4-{4-(naphthalen-1-yl)phenyl }-6-{4-(pyridin-3-yl)phenyl}pyrimidine (Compound 78) was obtained as a yellow powder.

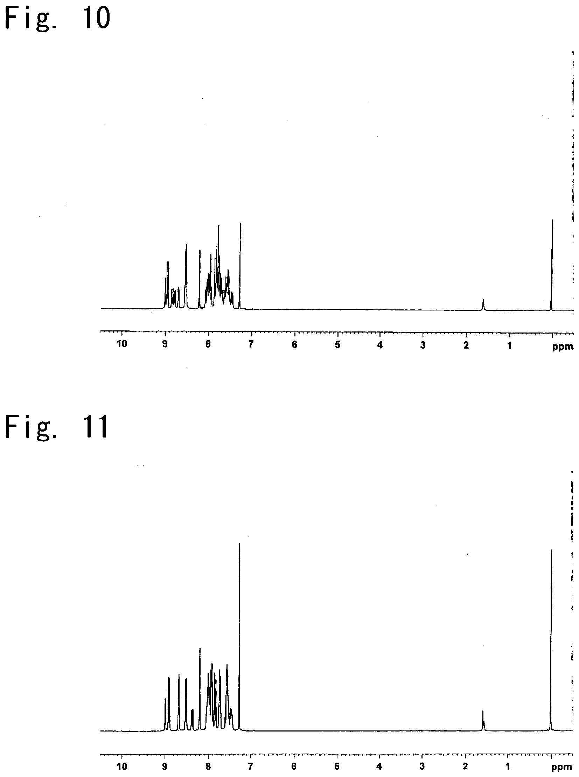

In connection with the resulting yellow powder, its structure was identified using NMR. The results of its ¹H-NMR measurement are shown in Fig. 10. In ¹H-NMR (CDCl₃), the following signals of 33 hydrogens were detected.

| | | |
|---|---|---|
| δ (ppm) | = | 9.00 (1H) |
| | | 8.95 (2H) |
| | | 8.83 (1H) |
| | | 8.76 (1H) |
| | | 8.69 (1H) |
| | | 8.52-8.48 (4H) |
| | | 8.22 (1H) |
| | | 8.08-7.91 (6H) |
| | | 7.86-7.42 (16H) |

### <Example 11: Synthesis of Compound 76> 2-{4-(naphthalen-1-yl)phenyl}-4-{3-(naphthalen-2-yl)phenyl} -6-{4-(pyridin-3-yl)phenyl}pyrimidine:

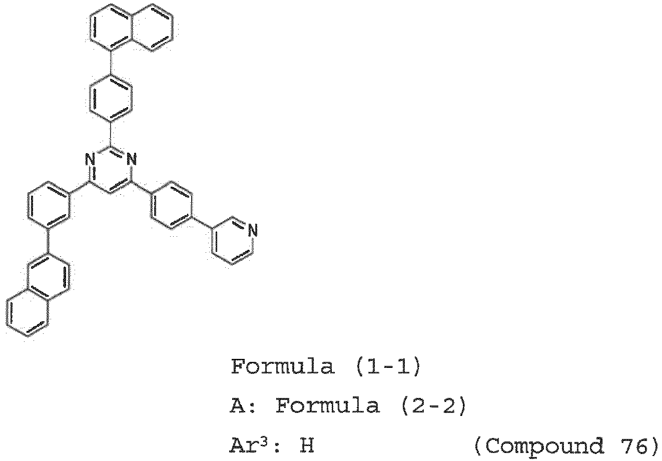

In Example 1,
4-(naphthalen-1-yl)phenylboronic acid was used instead of
3-(naphthalen-1-yl)phenylboronic acid, and
2-chloro-4-{3-(naphthalen-2-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 24.0 g (yield 52%) of 2-{4-(naphthalen-1-yl)phenyl}-4-{3-(naphthalen-2-yl)phenyl} -6-{4-(pyridin-3-yl)phenyl}pyrimidine (Compound 76) was obtained as a yellow powder.

In connection with the resulting yellow powder, its structure was identified using NMR. The results of its ¹H-NMR measurement are shown in Fig. 11. In ¹H-NMR (CDCl₃), the following signals of 31 hydrogens were detected.

| | | |
|---|---|---|
| δ (ppm) | = | 9.00 (1H) |
| | | 8.90 (2H) |
| | | 8.68 (2H) |
| | | 8.53 (2H) |
| | | 8.37 (1H) |
| | | 8.21 (2H) |
| | | 8.08-7.81 (11H) |
| | | 7.78-7.71 (3H) |
| | | 7.64-7.42 (7H) |

### <Example 12: Synthesis of Compound 126> (Reference Example) 2-(biphenyl-4-yl)-4-{4-(phenanthren-9-yl)phenyl}-6-{4-(pyri din-3-yl)phenyl}pyrimidine:

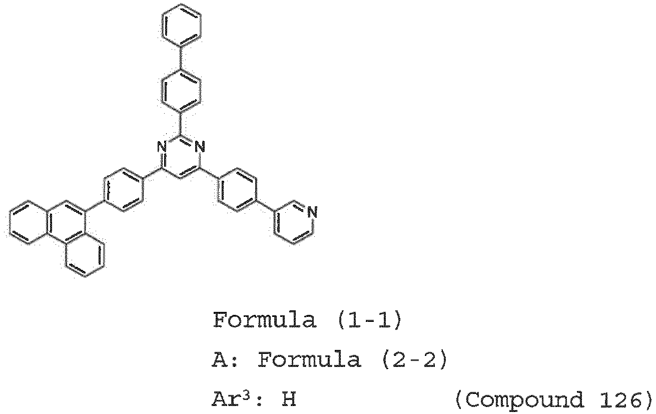

In Example 1,
4-biphenylboronic acid was used instead of
3-(naphthalen-1-yl)phenylboronic acid, and
2-chloro-4-{4-(phenanthren-9-yl)phenyl}-6-{4-(pyridin -3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 6.5 g (yield 85%) of 2-(biphenyl-4-yl)-4-{4-(phenanthren-9-yl)phenyl}-6-{4-(pyri din-3-yl)phenyl}pyrimidine (Compound 126) was obtained as a yellow powder.

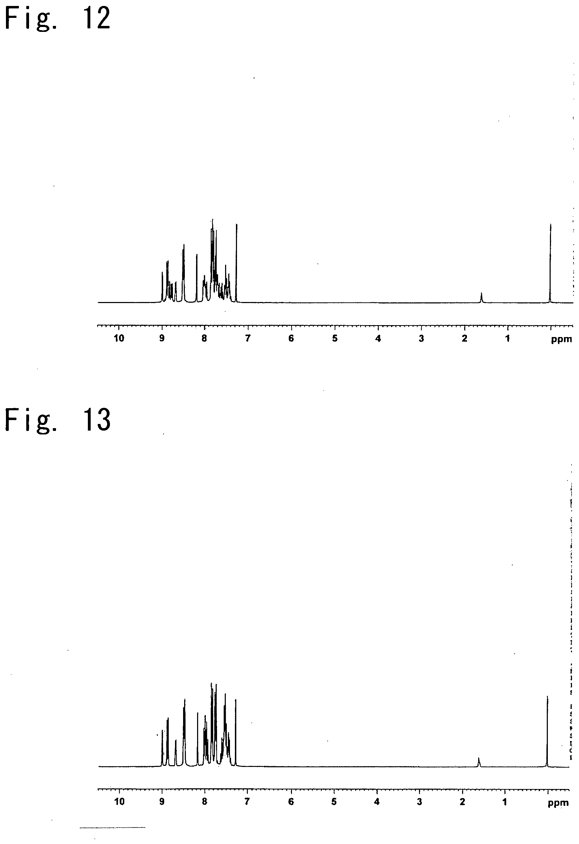

In connection with the resulting yellow powder, its structure was identified using NMR. The results of its ¹H-NMR measurement are shown in Fig. 12. In ¹H-NMR (CDCl₃), the following signals of 31 hydrogens were detected.

| | | |
|---|---|---|
| δ (ppm) | = | 9.00 (1H) |
| | | 8.92-8.76 (4H) |
| | | 8.68 (1H) |
| | | 8.54-8.46 (4H) |
| | | 8.18 (1H) |
| | | 8.05-7.94 (3H) |
| | | 7.88-7.38 (17H) |

### <Example 13: Synthesis of Compound 124> (Reference Example) 2-(biphenyl-4-yl)-4-{4-(naphthalen-1-yl)phenyl}-6-{4-(pyrid in-3-yl)phenyl}pyrimidine:

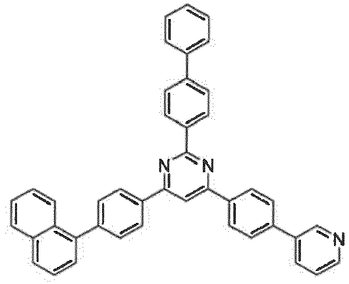

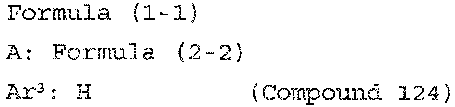

In Example 1,
4-biphenylboronic acid was used instead of
3-(naphthalen-1-yl)phenylboronic acid, and
2-chloro-4-{4-(naphthalen-1-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 17.6 g (yield 64%) of 2-(biphenyl-4-yl)-4-{4-(naphthalen-1-yl)phenyl}-6-{4-(pyrid in-3-yl)phenyl}pyrimidine (Compound 124) was obtained as a yellow powder.

In connection with the resulting yellow powder, its structure was identified using NMR. The results of its ¹H-NMR measurement are shown in Fig. 13. In ¹H-NMR (THF-d₈), the following signals of 29 hydrogens were detected.

| | | |
|---|---|---|
| δ (ppm) | = | 9.00 (1H) |
| | | 8.89 (2H) |
| | | 8.67 (1H) |
| | | 8.51-8.48 (4H) |
| | | 8.17 (1H) |
| | | 8.03-7.93 (4H) |
| | | 7.86-7.81 (4H) |
| | | 7.78-7.72 (4H) |
| | | 7.63-7.38 (8H) |

### <Example 14: Synthesis of Compound 123> (Reference Example) 2-(biphenyl-3-yl)-4-{4-(phenanthren-9-yl)phenyl}-6-{4-(pyri din-3-yl)phenyl}pyrimidine:

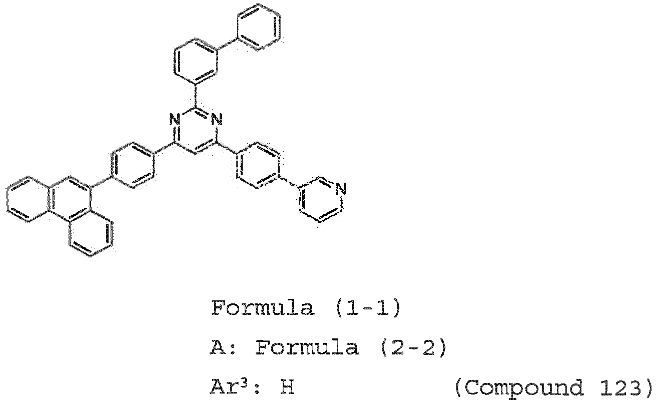

In Example 1,
3-biphenylboronic acid was used instead of
3-(naphthalen-1-yl)phenylboronic acid, and
2-chloro-4-{4-(phenanthren-9-yl)phenyl}-6-{4-(pyridin -3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 3.0 g (yield 38%) of 2-(biphenyl-3-yl)-4-{4-(phenanthren-9-yl)phenyl}-6-{4-(pyri din-3-yl)phenyl}pyrimidine (Compound 123) was obtained as a yellow powder.

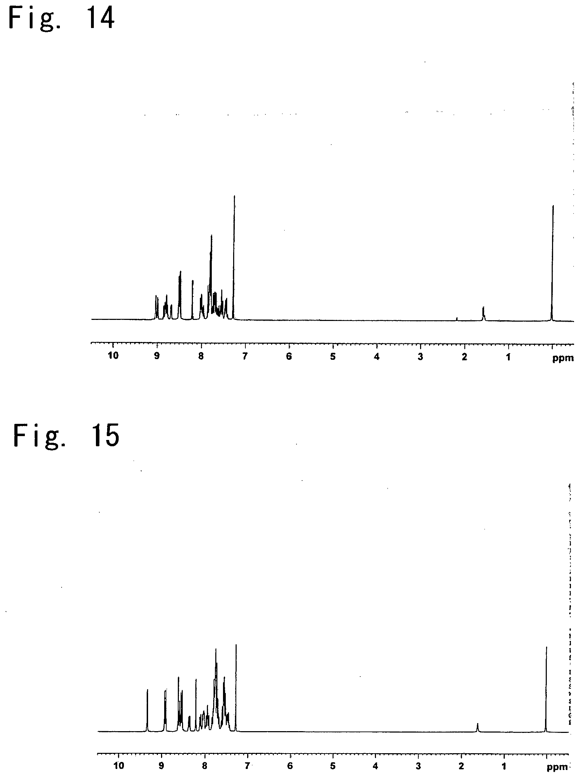

In connection with the resulting yellow powder, its structure was identified using NMR. The results of its ¹H-NMR measurement are shown in Fig. 14. In ¹H-NMR (CDCl₃), the following signals of 31 hydrogens were detected.

| | | |
|---|---|---|
| δ (ppm) | = | 9.02 (2H) |
| | | 8.86-8.76 (3H) |
| | | 8.69 (1H) |
| | | 8.52-8.48 (4H) |
| | | 8.21 (1H) |
| | | 8.05-7.93 (3H) |
| | | 7.89-7.40 (17H) |

### <Example 15: Synthesis of Compound 146> (Reference Example) 2-{4-(naphthalen-1-yl)phenyl}-4-(biphenyl-4-yl)-6-{4-(quino lin-3-yl)phenyl}pyrimidine:

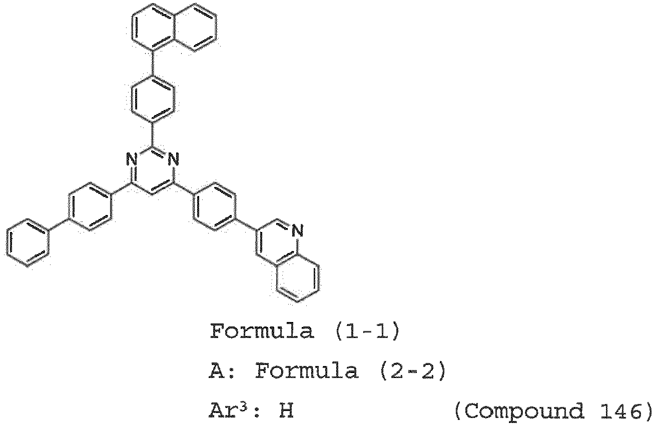

In Example 1,
4-(naphthalen-1-yl)phenylboronic acid was used instead of
3-(naphthalen-1-yl)phenylboronic acid, and
2-chloro-4-(biphenyl-4-yl)-6-{4-(quinolin-3-yl)phenyl }pyrimidine was used instead of
2-chloro-4-(biphenyl-4-yl)-6-{4-(pyridin-3-yl)phenyl} pyrimidine,
and the reaction was performed under the same conditions. As a result, 1.2 g (yield 15%) of 2-{4-(naphthalen-1-yl)phenyl}-4-(biphenyl-4-yl)-6-{4-(quino lin-3-yl)phenyl}pyrimidine (Compound 146) was obtained as a yellow powder.

In connection with the resulting yellow powder, its structure was identified using NMR. The results of its ¹H-NMR measurement are shown in Fig. 15. In ¹H-NMR (CDCl₃), the following signals of 31 hydrogens were detected.

| | | |
|---|---|---|
| δ (ppm) | = | 9.34 (1H) |
| | | 8.91 (2H) |
| | | 8.62-8.52 (4H) |
| | | 8.37 (1H) |
| | | 8.22 (1H) |
| | | 8.14-7.90 (5H) |
| | | 7.86-7.40 (17H) |

### <Example 16: Synthesis of Compound 98> 2-{3-(phenanthren-9-yl)phenyl}-4-{3-(naphthalen-2-yl)phenyl }-6-{4-(pyridin-3-yl)phenyl}pyrimidine:

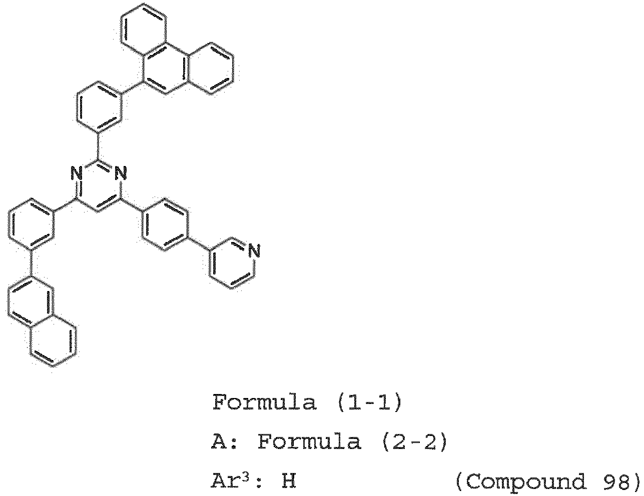

In Example 1,
3-(phenanthren-9-yl)phenylboronic acid was used instead of
3-(naphthalen-1-yl)phenylboronic acid, and
2-chloro-4-{3-(naphthalen-2-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 5.0 g (yield 57%) of 2-{3-(phenanthren-9-yl)phenyl}-4-{3-(naphthalen-2-yl)phenyl }-6-{4-(pyridin-3-yl)phenyl}pyrimidine (Compound 98) was obtained as a yellow powder.

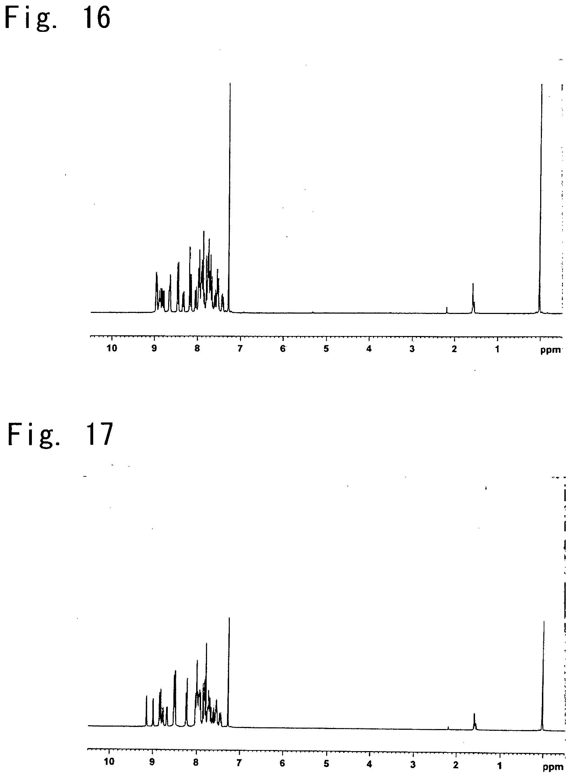

In connection with the resulting yellow powder, its structure was identified using NMR. The results of its ¹H-NMR measurement are shown in Fig. 16. In ¹H-NMR (CDCl₃), the following signals of 33 hydrogens were detected.

| | | |
|---|---|---|
| δ (ppm) | = | 8.98-8.78 (5H) |
| | | 8.68-8.62 (2H) |
| | | 8.45 (2H) |
| | | 8.32 (1H) |
| | | 8.17 (2H) |
| | | 8.03 (1H) |
| | | 8.00-7.39 (20H) |

### <Example 17: Synthesis of Compound 153> 2-{3-(naphthalen-2-yl)phenyl}-4-{4-(phenanthren-9-yl)phenyl }-6-{4-(pyridin-3-yl)phenyl}pyrimidine:

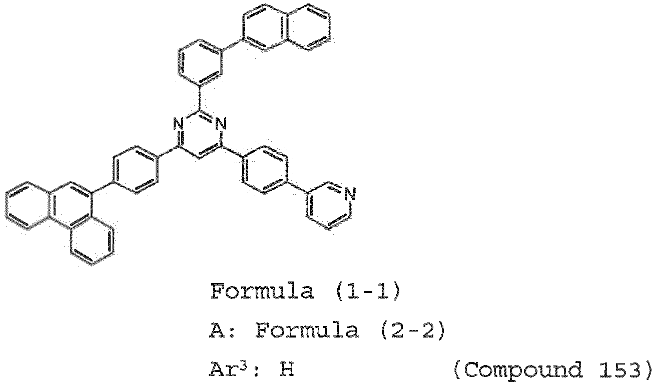

In Example 1,
3-(naphthalen-2-yl)phenylboronic acid was used instead of
3-(naphthalen-1-yl)phenylboronic acid, and
2-chloro-4-{4-(phenanthren-9-yl)phenyl}-6-{4-(pyridin -3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 15.4 g (yield 73%) of 2-{3-(naphthalen-2-yl)phenyl}-4-{4-(phenanthren-9-yl)phenyl }-6-{4-(pyridin-3-yl)phenyl}pyrimidine (Compound 153) was obtained as a yellow powder.

In connection with the resulting yellow powder, its structure was identified using NMR. The results of its ¹H-NMR measurement are shown in Fig. 17. In ¹H-NMR (CDCl₃), the following signals of 33 hydrogens were detected.

| | | |
|---|---|---|
| δ (ppm) | = | 9.15 (1H) |
| | | 9.00 (1H) |
| | | 8.83 (2H) |
| | | 8.78 (1H) |
| | | 8.68 (1H) |
| | | 8.53-8.47 (4H) |
| | | 8.22 (2H) |
| | | 8.04-7.42 (21H) |

### <Example 18: Synthesis of Compound 155> 2-{4-(naphthalen-1-yl)phenyl}-4-{3-(phenanthren-9-yl)phenyl }-6-{4-(pyridin-3-yl)phenyl}pyrimidine:

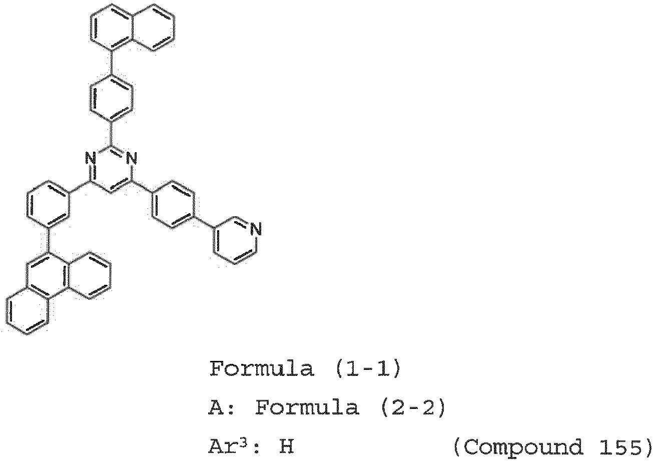

In Example 1,
4-(naphthalen-1-yl)phenylboronic acid was used instead of
3-(naphthalen-1-yl)phenylboronic acid, and
2-chloro-4-{3-(phenanthren-9-yl)phenyl}-6-{4-(pyridin -3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 2.5 g (yield 42%) of 2-{4-(naphthalen-1-yl)phenyl}-4-{3-(phenanthren-9-yl)phenyl }-6-{4-(pyridin-3-yl)phenyl}pyrimidine (Compound 155) was obtained as a yellow powder.

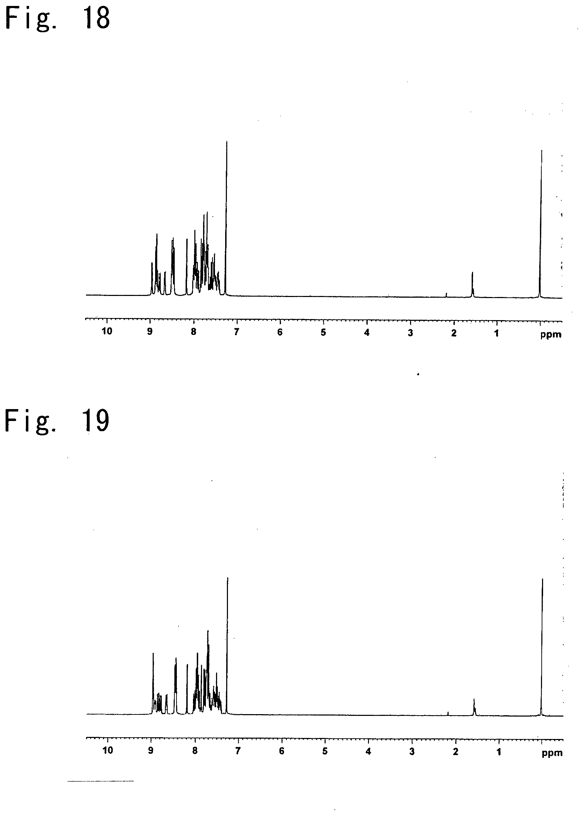

In connection with the resulting yellow powder, its structure was identified using NMR. The results of its ¹H-NMR measurement are shown in Fig. 18. In ¹H-NMR (CDCl₃), the following signals of 33 hydrogens were detected.

| | | |
|---|---|---|
| δ (ppm) | = | 8.97 (1H) |
| | | 8.97-8.76 (4H) |
| | | 8.67 (1H) |
| | | 8.52-8.46 (4H) |
| | | 8.17 (1H) |
| | | 8.01-7.43 (22H) |

### <Example 19: Synthesis of Compound 82> 2-{3-(phenanthren-9-yl)phenyl}-4-{4-(naphthalen-1-yl)phenyl }-6-{4-(pyridin-3-yl)phenyl}pyrimidine:

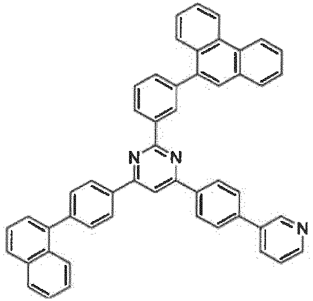

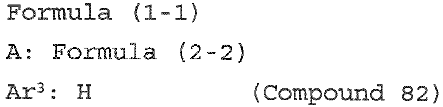

In Example 1,
3-(phenanthren-9-yl)phenylboronic acid was used instead of
3-(naphthalen-1-yl)phenylboronic acid, and
2-chloro-4-{4-(naphthalen-1-yl)phenyl}-6-{4-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 6.3 g (yield 72%) of 2-{3-(phenanthren-9-yl)phenyl}-4-{4-(naphthalen-1-yl)phenyl }-6-{4-(pyridin-3-yl)phenyl}pyrimidine (Compound 82) was obtained as a yellow powder.

In connection with the resulting yellow powder, its structure was identified using NMR. The results of its ¹H-NMR measurement are shown in Fig. 19. In ¹H-NMR (CDCl₃), the following signals of 33 hydrogens were detected.

| | | |
|---|---|---|
| δ (ppm) | = | 8.97-8.88 (3H) |
| | | 8.87-8.76 (2H) |
| | | 8.66 (1H) |
| | | 8.49-8.42 (4H) |
| | | 8.20 (1H) |
| | | 8.08-7.84 (7H) |
| | | 7.83-7.40 (15H) |

### <Example 20: Synthesis of Compound 182> (Reference Example) 2-{3-(naphthalen-1-yl)phenyl}-4-{4-(naphthalen-1-yl)phenyl} -6-{4-(quinolin-3-yl)phenyl}pyrimidine:

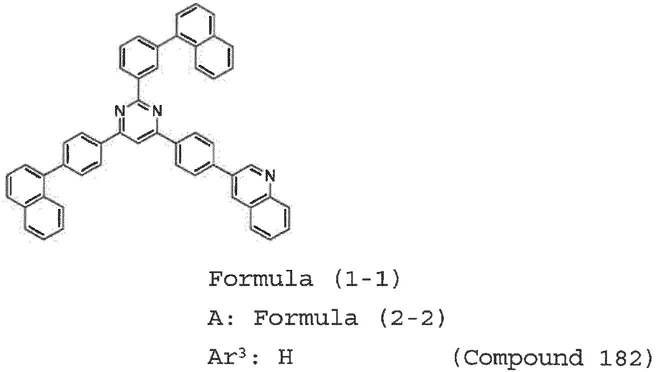

In Example 1,
2-chloro-4-{4-(naphthalen-1-yl)phenyl}-6-{4-(quinolin -3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 1.5 g (yield 23%) of 2-{3-(naphthalen-1-yl)phenyl}-4-{4-(naphthalen-1-yl)phenyl} -6-{4-(quinolin-3-yl)phenyl}pyrimidine (Compound 182) was obtained as a yellow powder.

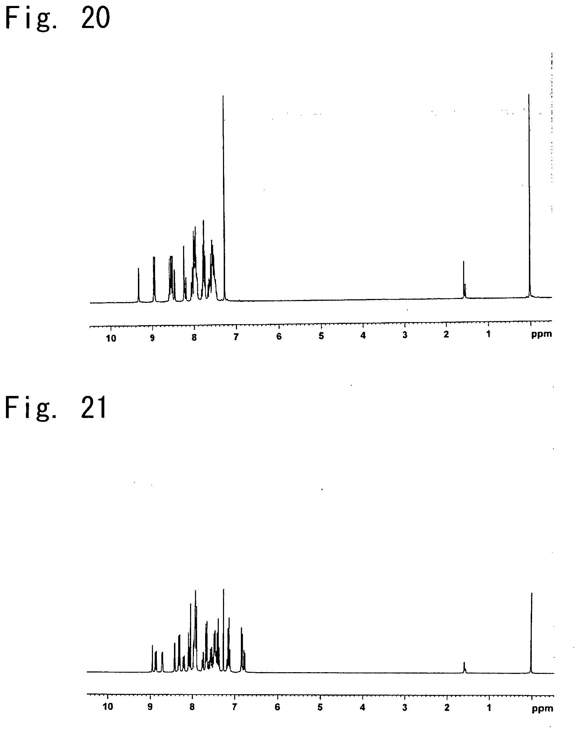

In connection with the resulting yellow powder, its structure was identified using NMR. The results of its ¹H-NMR measurement are shown in Fig. 20. In ¹H-NMR (THF-d₈), the following signals of 33 hydrogens were detected.

| | | |
|---|---|---|
| δ (ppm) | = | 9.33 (1H) |
| | | 8.94 (2H) |
| | | 8.59-8.42 (5H) |
| | | 8.23-8.17 (2H) |
| | | 8.04-7.90 (9H) |
| | | 7.82-7.72 (5H) |
| | | 7.64-7.45 (9H) |

### <Example 21: Synthesis of Compound 227> (Reference Example) 2-(9,9'-spirobi[9H-fluoren]-2-yl)-4-{4-(naphthalen-1-yl)phe nyl}-6-{3-(pyridin-3-yl)phenyl}pyrimidine:

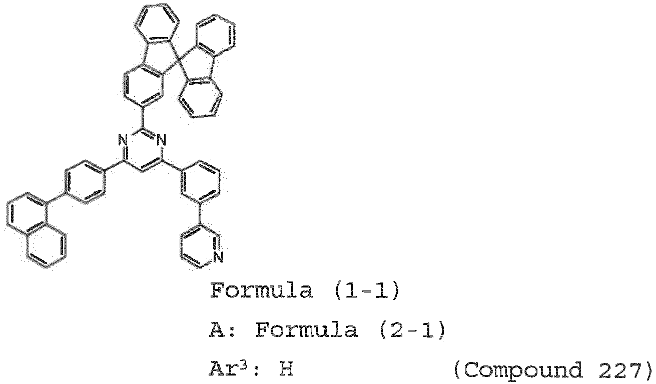

In Example 1,
9,9'-spirobi[9H-fluoren]-2-boronic acid was used instead of
3-(naphthalen-1-yl)phenylboronic acid, and
2-chloro-4-{4-(naphthalen-1-yl)phenyl}-6-{3-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 1.3 g (yield 20%) of 2-(9,9'-spirobi[9H-fluoren]-2-yl)-4-{4-(naphthalen-1-yl)phe nyl}-6-{3-(pyridin-3-yl)phenyl}pyrimidine (Compound 227) was obtained as a yellow powder.

In connection with the resulting yellow powder, its structure was identified using NMR. The results of its ¹H-NMR measurement are shown in Fig. 21. In ¹H-NMR (CDCl₃), the following signals of 35 hydrogens were detected.

| | | |
|---|---|---|
| δ (ppm) | = | 8.95 (1H) |
| | | 8.86 (1H) |
| | | 8.70 (1H) |
| | | 8.42 (1H) |
| | | 8.30 (2H) |
| | | 8.22 (1H) |
| | | 8.12-8.03 (3H) |
| | | 8.01-7.89 (7H) |
| | | 7.74 (1H) |
| | | 7.67-7.37 (11H) |
| | | 7.20-7.10 (3H) |
| | | 6.83 (2H) |
| | | 6.78 (1H) |

### <Example 22: Synthesis of Compound 234> (Reference Example) 2-(9,9'-spirobi[9H-fluoren]-2-yl)-4-(biphenyl-4-yl)-6-{3-(p yridin-3-yl)phenyl}pyrimidine:

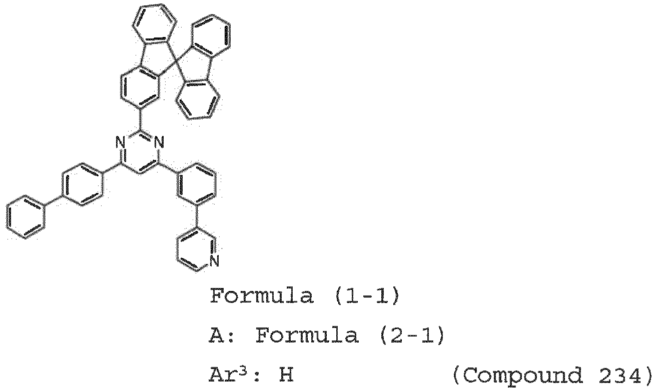

In Example 1,
9,9'-spirobi[9H-fluoren]-2-boronic acid was used instead of
3-(naphthalen-1-yl)phenylboronic acid, and
2-chloro-4-(biphenyl-4-yl)-6-{3-(pyridin-3-yl)phenyl} pyrimidine was used instead of
2-chloro-4-(biphenyl-4-yl)-6-{4-(pyridin-3-yl)phenyl} pyrimidine,
and the reaction was performed under the same conditions. As a result, 1.5 g (yield 25%) of 2-(9,9'-spirobi[9H-fluoren]-2-yl)-4-(biphenyl-4-yl)-6-{3-(p yridin-3-yl)phenyl}pyrimidine (Compound 234) was obtained as a white powder.

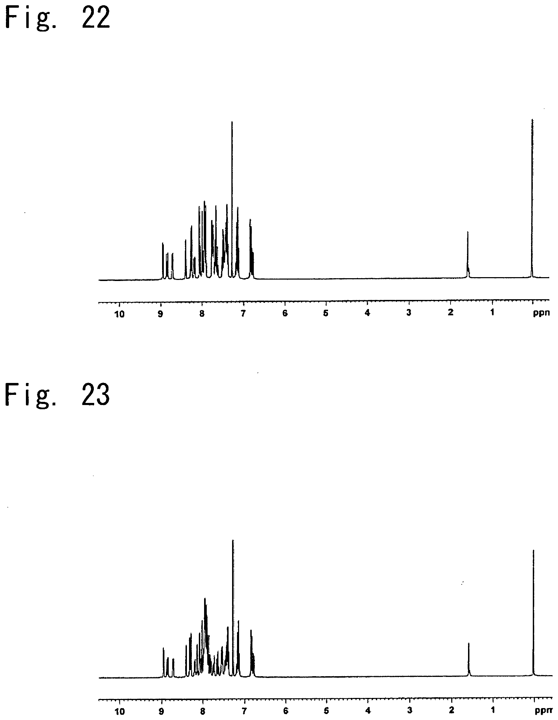

In connection with the resulting white powder, its structure was identified using NMR. The results of its ¹H-NMR measurement are shown in Fig. 22. In ¹H-NMR (CDCl₃), the following signals of 33 hydrogens were detected.

| | | |
|---|---|---|
| δ (ppm) | = | 8.96 (1H) |
| | | 8.86 (1H) |
| | | 8.72 (1H) |
| | | 8.39 (1H) |
| | | 8.26 (2H) |
| | | 8.17 (1H) |
| | | 8.12-7.89 (7H) |
| | | 7.78-7.60 (6H) |
| | | 7.53-7.25 (7H) |
| | | 7.21-7.10 (3H) |
| | | 6.83-6.75 (3H) |

### <Example 23: Synthesis of Compound 235> (Reference Example) 2-(9,9'-spirobi[9H-fluoren]-2-yl)-4-{4-(naphthalen-2-yl)phe nyl}-6-{3-(pyridin-3-yl)phenyl}pyrimidine:

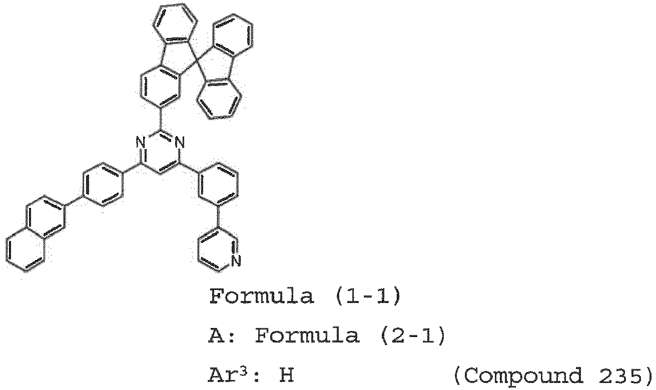

In Example 1,
9,9'-spirobi[9H-fluoren]-2-boronic acid was used instead of
3-(naphthalen-1-yl)phenylboronic acid, and
2-chloro-4-{4-(naphthalen-2-yl)phenyl}-6-{3-(pyridin-3-yl)phenyl}pyrimidine was used instead of
2-chloro-4-(biphenyl-4-yl)-6-{4-(pyridin-3-yl)phenyl} pyrimidine,
and the reaction was performed under the same conditions. As a result, 2.0 g (yield 32%) of 2-(9,9'-spirobi[9H-fluoren]-2-yl)-4-{4-(naphthalen-2-yl)phe nyl}-6-{3-(pyridin-3-yl)phenyl}pyrimidine (Compound 235) was obtained as a white powder.

In connection with the resulting white powder, its structure was identified using NMR. The results of its ¹H-NMR measurement are shown in Fig. 23. In ¹H-NMR (CDCl₃), the following signals of 35 hydrogens were detected.

| | | |
|---|---|---|
| δ (ppm) | = | 8.96 (1H) |
| | | 8.85 (1H) |
| | | 8.71 (1H) |
| | | 8.41 (1H) |
| | | 8.30 (2H) |
| | | 8.19 (1H) |
| | | 8.12 (1H) |
| | | 8.79-7.39 (21H) |
| | | 7.20-7.11 (3H) |
| | | 6.83 (2H) |
| | | 6.78 (1H) |

### <Measurement of work function>

Using each of the compounds of the present invention, a vapor deposited film with a film thickness of 100 nm was prepared on an ITO substrate, and its work function was measured using an ionization potential measuring device (PYS-202, produced by Sumitomo Heavy Industries, Ltd.). The compounds of Examples 1 to 3, 12 to 15 and 20 to 23 are Reference Examples.

**Work function**

| | |
|---|---|
| Compound of Example 1 (Compound 74) | 6.63 eV |
| Compound of Example 2 (Compound 84) | 6.60 eV |
| Compound of Example 3 (Compound 89) | 6.39 eV |
| Compound of Example 4 (Compound 130) | 6.50 eV |
| Compound of Example 5 (Compound 131) | 6.50 eV |
| Compound of Example 6 (Compound 92) | 6.51 eV |
| Compound of Example 7 (Compound 136) | 6.47 eV |
| Compound of Example 8 (Compound 125) | 6.50 eV |
| Compound of Example 9 (Compound 138) | 6.47 eV |
| Compound of Example 10 (Compound 78) | 6.48 eV |
| Compound of Example 11 (Compound 76) | 6.55 eV |
| Compound of Example 12 (Compound 126) | 6.56 eV |
| Compound of Example 13 (Compound 124) | 6.50 eV |
| Compound of Example 14 (Compound 123) | 6.53 eV |
| Compound of Example 15 (Compound 146) | 6.53 eV |
| Compound of Example 16 (Compound 98) | 6.48 eV |
| Compound of Example 17 (Compound 153) | 6.46 eV |
| Compound of Example 18 (Compound 155) | 6.51 eV |
| Compound of Example 19 (Compound 82) | 6.46 eV |
| Compound of Example 20 (Compound 182) | 6.54 eV |
| Compound of Example 21 (Compound 227) | 6.55 eV |
| Compound of Example 22 (Compound 234) | 6.55 eV |
| Compound of Example 23 (Compound 235) | 6.54 eV |

As described above, the compounds of the present invention showed greater values than a work function of 5.5 eV shown by a general hole transport material such as NPD or TPD, and are thus found to have high hole blocking capability.

### <Measurement of glass transition point>

The compounds obtained in Examples 4 to 23 were measured for the glass transition point by a high sensitivity differential scanning calorimeter (DSC3100S, produced by Bruker AXS K.K.). The compounds of Examples 12 to 15 and 20 to 23 are Reference Examples.

**Glass transition point**

| | |
|---|---|
| Compound of Example 4 (Compound 130) | 129°C |
| Compound of Example 5 (Compound 131) | 138°C |
| Compound of Example 6 (Compound 92) | 108°C |
| Compound of Example 7 (Compound 136) | 128°C |
| Compound of Example 8 (Compound 125) | 117°C |
| Compound of Example 9 (Compound 138) | 111°C |
| Compound of Example 10 (Compound 78) | 138°C |
| Compound of Example 11 (Compound 76) | 103°C |
| Compound of Example 12 (Compound 126) | 129°C |
| Compound of Example 13 (Compound 124) | 107°C |
| Compound of Example 14 (Compound 123) | 116°C |
| Compound of Example 15 (Compound 146) | 114°C |
| Compound of Example 16 (Compound 98) | 116°C |
| Compound of Example 17 (Compound 153) | 116°C |
| Compound of Example 18 (Compound 155) | 132°C |
| Compound of Example 19 (Compound 82) | 131°C |
| Compound of Example 20 (Compound 182) | 114°C |
| Compound of Example 21 (Compound 227) | 150°C |
| Compound of Example 22 (Compound 234) | 143°C |
| Compound of Example 23 (Compound 235) | 146°C |

The compounds of the present invention have a glass transition point of 100°C or higher, particularly, 140°C or higher, demonstrating that the compounds of the present invention are stable in a thin film state.

### <Organic EL Device Example 1> (Reference Example)

A hole injection layer 3, a hole transport layer 4, a luminous layer 5, a hole blocking layer 6, an electron transport layer 7, an electron injection layer 8, and a cathode (aluminum electrode) 9 were vapor deposited in this order on an ITO electrode formed beforehand as a transparent anode 2 on a glass substrate 1 to prepare an organic EL device as shown in Fig. 24. Although depicted in Fig. 24 as two distinct layers, the hole blocking layer 6 and the electron transport layer 7 were formed as a single film serving concurrently as the hole blocking layer 6 and the electron transport layer 7, as described below in detail.

Concretely, the glass substrate 1 having a 150 nm thick ITO film formed thereon was ultrasonically cleaned for 20 minutes in isopropyl alcohol, and then dried for 10 minutes on a hot plate heated to 200°C. Then, the glass substrate with ITO was subjected to UV/ozone treatment for 15 minutes. Then, the ITO-equipped glass substrate was mounted within a vacuum deposition machine, and the pressure was reduced to 0.001 Pa or lower. Then, a film of HIM-1, a compound represented by a structural formula indicated below, was formed in a film thickness of 5 nm as the hole injection layer 3 so as to cover the transparent anode 2. On the hole injection layer 3, a film of HTM-1, a compound represented by a structural formula indicated below, was formed in a film thickness of 65 nm as the hole transport layer 4. On the hole transport layer 4, EMD-1 represented by a structural formula indicated below, and EMH-1 represented by a structural formula indicated below were binary vapor deposited at such vapor deposition rates that the vapor deposition rate ratio was EMD-1:EMH-1=5:95, whereby the luminous layer 5 was formed in a film thickness of 20 nm. On this luminous layer 5, the compound of Example 1 (Compound 74) (Reference Example) and ETM-1, a compound represented by a structural formula indicated below, were binary vapor deposited at such vapor deposition rates that the vapor deposition rate ratio was the compound of Example 1 (Compound 74) (Reference Example):ETM-1=50:50, whereby a film concurrently serving as the hole blocking layer 6 and the electron transport layer 7 was formed in a film thickness of 30 nm. On the hole blocking layer 6/electron transport layer 7, a film of lithium fluoride was formed in a film thickness of 1 nm as the electron injection layer 8. Finally, aluminum was vapor deposited to a film thickness of 100 nm to form the cathode 9.

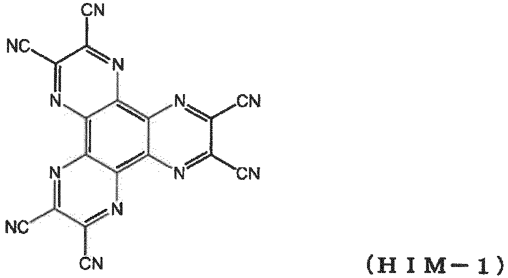

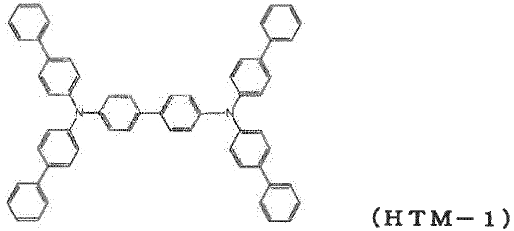

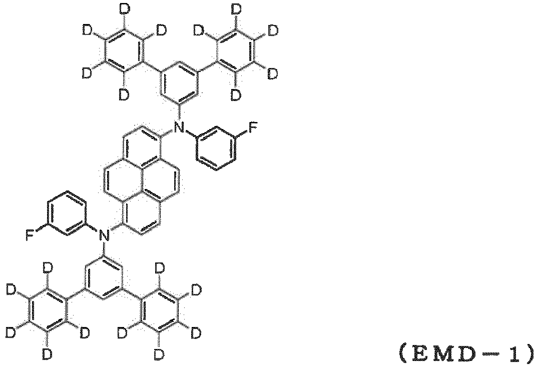

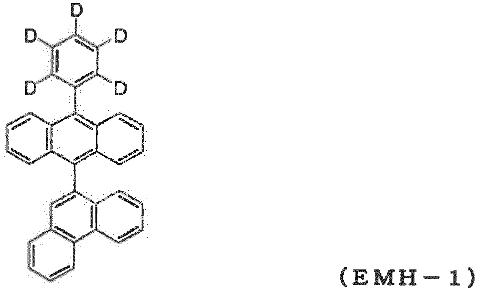

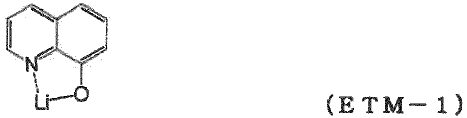

### <Organic EL Device Examples 2 to 20> (Examples 2, 3, 12 to 15 and 20 to 23 are Reference Examples)

Organic EL devices were prepared under the same conditions as in Organic EL Device Example 1, except that the compound of each of Examples 2 to 20 (the compounds of Examples 2, 3, 12 to 15 and 20 to 23 are Reference Examples) was used, instead of the compound of Example 1 (Compound 74), as the material for the hole blocking layer 6/electron transport layer 7, as shown in Table 1.

### <Organic EL Device Comparative Example 1>

For comparison, an organic EL device was prepared under the same conditions as in Organic EL Device Example 1, except that ETM-2 (see Patent Document 4), a compound represented by a structural formula indicated below, was used, instead of the compound of Example 1 (Compound 74), as the material for the hole blocking layer 6/electron transport layer 7.

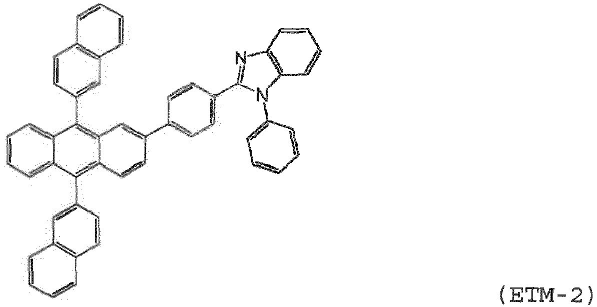

The organic EL devices prepared in Organic EL Device Examples 1 to 20, and Organic EL Device Comparative Example 1 were measured for the light emission characteristics when a direct current voltage was applied at normal temperature in the atmosphere. The results of the measurements are shown in Table 1.

The organic EL devices prepared in Organic EL Device Examples 1 to 20, and Organic EL Device Comparative Example 1 were measured for the device lifetime. Concretely, the device lifetime was measured as the period of time until the emitting brightness attenuated to 1900 cd/m² (corresponding to 95%, with the initial luminance taken as 100%: 95% attenuation) when constant current driving was performed, with the emitting brightness at the start of light emission (initial luminance) being set at 2000 cd/m². The results are shown in Table 1. Examples 1 to 3, 12 to 15 and 20 to 23 are Reference Examples.

### [Table 1]

**Table 1**

| *1 | *2 | *3 | *4 | *5 | *6 | *7 |
|---|---|---|---|---|---|---|
| Ex. 1 | Compound 74/ETM-1 | 3,89 | 719 | 7,22 | 5,84 | 181 |
| Ex. 2 | Compound 84/ETM-1 | 3,75 | 733 | 7,36 | 6,18 | 179 |
| Ex. 3 | Compound 89/ETM-1 | 3,97 | 725 | 7,27 | 5,76 | 208 |
| Ex. 4 | Compound 130/ETM-1 | 3,66 | 795 | 7,98 | 6,85 | 138 |
| Ex. 5 | Compound 131/ETM-1 | 3,79 | 766 | 7,67 | 6,35 | 128 |
| Ex. 6 | Compound 92/ETM-1 | 3,75 | 795 | 7,98 | 6,69 | 156 |
| Ex. 7 | Compound 136/ETM-1 | 3,94 | 734 | 7,37 | 5,88 | 208 |
| Ex. 8 | Compound 125/ETM-1 | 3,70 | 751 | 7,54 | 6,41 | 216 |
| Ex. 9 | Compound 138/ETM-1 | 3,77 | 740 | 7,42 | 6,18 | 226 |
| Ex. 10 | Compound 78/ETM-1 | 3,84 | 710 | 7,11 | 5,82 | 187 |
| Ex. 11 | Compound 76/ETM-1 | 3,83 | 769 | 7,70 | 6,33 | 208 |
| Ex. 12 | Compound 126/ETM-1 | 3,66 | 789 | 7,92 | 6,80 | 152 |
| Ex. 13 | Compound 124/ETM-1 | 3,66 | 761 | 7,64 | 6,56 | 204 |
| Ex. 14 | Compound 123/ETM-1 | 3,67 | 812 | 8,14 | 6,98 | 142 |
| Ex. 15 | Compound 146/ETM-1 | 3,71 | 753 | 7,57 | 6,42 | 201 |
| Ex. 16 | Compound 98/ETM-1 | 3,93 | 734 | 7,36 | 5,90 | 181 |
| Ex. 17 | Compound 153/ETM-1 | 3,87 | 820 | 8,23 | 6,70 | 180 |
| Ex. 18 | Compound 155/ETM-1 | 3,77 | 804 | 8,06 | 6,71 | 166 |
| Ex. 19 | Compound 82/ETM-1 | 3,82 | 780 | 7,81 | 6,43 | 190 |
| Ex. 20 | Compound 182/ETM-1 | 3,73 | 684 | 6,86 | 5,79 | 276 |
| Comp. Ex. 1 | ETM-2/ETM-1 | 3,84 | 635 | 6,35 | 5,20 | 55 |

| | | | | | | |
|---|---|---|---|---|---|---|
| *1: Organic El Device Example/Comparative Example *2: Hole blocking layer/electron transport layer *3: Voltage [V] (@10mA/cm²) *4: Luminance [cd/m²] (@10mA/cm²) *5: Luminous efficiency [cd/A] (@10mA/cm²) *6: Power efficiency [lm/W] (@10mA/cm²) *7: Device lifetime 95% attenuation (hrs) | | | | | | |

As shown in Table 1, the luminous efficiency when an electric current at a current density of 10 mA/cm² was flowed showed a value of 6.35 cd/A in Organic EL Device Comparative Example 1, but showed greatly increased values of 6.86 to 8.23 cd/A in Organic EL Device Examples 1 to 20 (Examples 1 to 3, 12 to 15 and 20 to 23 are Reference Examples).

The power efficiency was 5.20 lm/W in Organic EL Device Comparative Example 1, while those in Organic EL Device Examples 1 to 20 were 5.76 to 6.98 lm/W, showing great increases.

The device lifetime (95% attenuation), in particular, was 55 hours in Organic EL Device Comparative Example 1, but those were 128 to 276 hours in Organic EL Device Examples 1 to 20 (Examples 1 to 3, 12 to 15 and 20 to 23 are Reference Examples), showing marked extensions. Such markedly extended lifetimes could be achieved, probably because the pyrimidine derivatives of the present invention are stable in a thin film state, and excellent in heat resistance, as compared with conventional materials for organic El devices. Moreover, such effects are presumed to result from the excellent carrier balance of the pyrimidine derivatives of the present invention.

Furthermore, the values of the driving voltage in Organic EL Device Examples 1 to 20 (Examples 1 to 3, 12 to 15 and 20 to 23 are Reference Examples) were as low as, or lower than, the value in Organic EL Device Comparative Example 1.

As noted above, the organic EL devices of the present invention were found to be excellent in luminous efficiency and power efficiency and were found to be capable of realizing an organic EL device having a long lifetime as compared with the device using the compound ETM-2, a general electron transport material.

The pyrimidine derivative of the present invention is satisfactory in electron injection characteristics, excellent in hole blocking capability, and stable in a thin film state, so that it excels as a compound for an organic EL device. An organic EL device prepared using this compound can provide a high efficiency, can lower driving voltage, and can improve durability. Thus, the organic EL device of the present invention can be put to uses such as domestic electrical appliances and illumination.

### Explanations of Letters or Numerals

- 1: Glass substrate
- 2: Transparent anode
- 3: Hole injection layer
- 4: Hole transport layer
- 5: Light emission layer
- 6: Hole blocking layer
- 7: Electron Transport layer
- 8: Electron injection layer
- 9: Cathode

## Claims

1. A pyrimidine derivative represented by the following general formula (1-1): 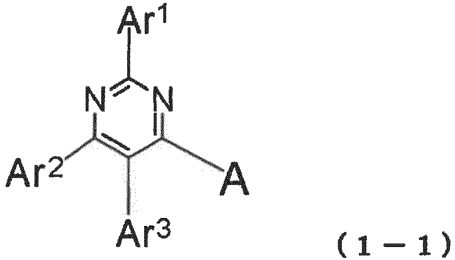 wherein,
Ar¹ is a phenyl group having, as a substituent, an unsubstituted condensed polycyclic aromatic group selected from the group consisting of a naphthyl group, a phenanthrenyl group, a pyrenyl group, a fluoranthenyl group, a triphenylenyl group, and a spirobifluorenyl group,
Ar² is a phenyl group having, as a substituent, an unsubstituted condensed polycyclic aromatic group selected from the group consisting of a naphthyl group, a phenanthrenyl group, a pyrenyl group, a fluoranthenyl group, a triphenylenyl group, and a spirobifluorenyl group,
Ar³ is a hydrogen atom, and
A is a monovalent group represented by the following structural formula (2-2): 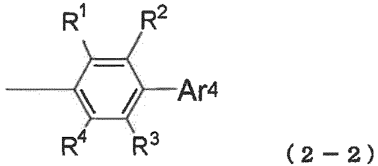 wherein,
Ar⁴ is an unsubstituted pyridyl group, and
R¹ to R⁴ are hydrogen atoms or deuterium atoms.

2. An organic electroluminescent device, comprising:
a pair of electrodes, and
at least one organic layer sandwiched therebetween,
wherein the pyrimidine derivative according to claim 1 is used as a constituent material for the at least one organic layer.

3. The organic electroluminescent device according to claim 2, wherein
the organic layer for which the pyrimidine derivative is used is an electron transport layer.

4. The organic electroluminescent device according to claim 2, wherein
the organic layer for which the pyrimidine derivative is used is a hole blocking layer.

5. The organic electroluminescent device according to claim 2, wherein
the organic layer for which the pyrimidine derivative is used is a luminous layer.

6. The organic electroluminescent device according to claim 2, wherein
the organic layer for which the pyrimidine derivative is used is an electron injection layer.

## Patentansprüche

1. Pyrimidinderivat, dargestellt durch die nachstehende allgemeine Formel (1-1): 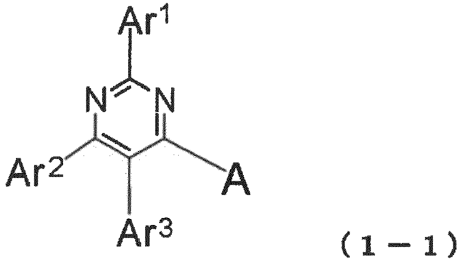 wobei,
Ar¹ eine Phenylgruppe ist, die als einen Substituenten eine unsubstituierte kondensierte polycyclische aromatische Gruppe aufweist, ausgewählt aus der Gruppe, bestehend aus einer Naphthylgruppe, einer Phenanthrenylgruppe, einer Pyrenylgruppe, einer Fluoranthenylgruppe, einer Triphenylenylgruppe und einer Spirobifluorenylgruppe,
Ar² eine Phenylgruppe ist, die als einen Substituenten eine unsubstituierte kondensierte polycyclische aromatische Gruppe aufweist, ausgewählt aus der Gruppe, bestehend aus einer Naphthylgruppe, einer Phenanthrenylgruppe, einer Pyrenylgruppe, einer Fluoranthenylgruppe, einer Triphenylenylgruppe und einer Spirobifluorenylgruppe,
Ar³ ein Wasserstoffatom ist, und
A eine einwertige Gruppe ist, die durch die nachstehende Strukturformel (2-2) dargestellt ist: 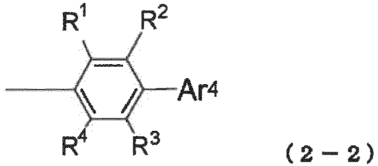 wobei,
Ar⁴ eine unsubstituierte Pyridylgruppe ist, und
R¹ bis R⁴ Wasserstoffatome oder Deuteriumatome sind.

2. Organische Elektrolumineszenzvorrichtung, umfassend: ein Paar von Elektroden, und
mindestens eine organische Schicht, die dazwischen angeordnet ist,
wobei das Pyrimidinderivat nach Anspruch 1 als ein konstituierendes Material für die mindestens eine organische Schicht verwendet wird.

3. Organische Elektrolumineszenzvorrichtung nach Anspruch 2, wobei
die organische Schicht, für die das Pyrimidinderivat verwendet wird, eine Elektronentransportschicht ist.

4. Organische Elektrolumineszenzvorrichtung nach Anspruch 2, wobei
die organische Schicht, für die das Pyrimidinderivat verwendet wird, eine Lochsperrschicht ist.

5. Organische Elektrolumineszenzvorrichtung nach Anspruch 2, wobei
die organische Schicht, für die das Pyrimidinderivat verwendet wird, eine Leuchtschicht ist.

6. Organische Elektrolumineszenzvorrichtung nach Anspruch 2, wobei
die organische Schicht, für die das Pyrimidinderivat verwendet wird, eine Elektroneninjektionsschicht ist.

## Revendications

1. Dérivé de pyrimidine représenté par la formule générale (1-1) suivante : 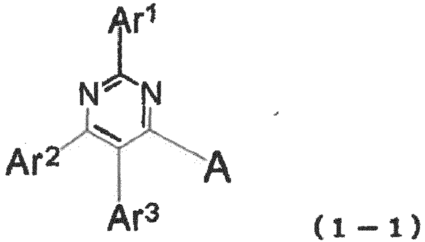 dans laquelle
Ar¹ est un groupe phényle ayant comme substituant, un groupe aromatique polycyclique condensé non substitué choisi parmi le groupe consistant en un groupe naphtyle, un groupe phénanthrényle, un groupe pyrényle, un groupe fluoranthényle, un groupe triphénylényle et un groupe spirobifluorényle,
Ar² est un groupe phényle ayant comme substituant, un groupe aromatique polycyclique condensé non substitué choisi parmi le groupe consistant en un groupe naphtyle, un groupe phénanthrényle, un groupe pyrényle, un groupe fluoranthényle, un groupe triphénylényle et un groupe spirobifluorényle,
Ar³ est un atome d'hydrogène, et
A est un groupe monovalent représenté par la formule structurale (2-2) suivante : 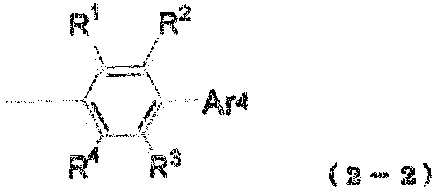 dans laquelle
Ar⁴ est un groupe pyridyle non substitué, et
R¹ à R⁴ sont des atomes d'hydrogène ou des atomes de deutérium.

2. Dispositif électroluminescent organique, comprenant :
une paire d'électrodes, et
au moins une couche organique intercalée entre elles,
dans lequel le dérivé de pyrimidine selon la revendication 1, est utilisé comme matière constitutive de la au moins une couche organique.

3. Dispositif électroluminescent organique selon la revendication 2, dans lequel la couche organique pour laquelle le dérivé de pyrimidine est utilisé est une couche de transport d'électrons.

4. Dispositif électroluminescent organique selon la revendication 2, dans lequel la couche organique pour laquelle le dérivé de pyrimidine est utilisé est une couche de blocage de trous.

5. Dispositif électroluminescent organique selon la revendication 2, dans lequel la couche organique pour laquelle le dérivé de pyrimidine est utilisé est une couche lumineuse.

6. Dispositif électroluminescent organique selon la revendication 2, dans lequel la couche organique pour laquelle le dérivé de pyrimidine est utilisé est une couche d'injection d'électrons.
